# EUROPEAN PATENT APPLICATION

(11) **EP 0 969 089 A1**
(43) Date of publication of application: **05.01.2000**
(21) Application number: 99111949.6
(22) Date of filing: 23.06.1999
(51) Int. Cl.: C12N 9/96, C12N 9/16, A23K 1/165

(54) **Phytase formulation**

(30) Priority: 29.06.1998 EP 98111960
(71) Applicant: F. HOFFMANN-LA ROCHE AG, 4070 Basel (CH)
(72) Inventor: Brugger, Roland, 79539 Lörrach (DE); Lehmann, Martin, Dr., Princeton, NJ 08540 (US); Wyss, Markus, 4410 Liestal (CH)
(74) Representative: Braun, Axel

(57) **Abstract**

A stabilized enzyme formulation is disclosed which comprises phytase and at least one stabilizing agent selected from the group consisting of:
a) C₅ sugars such as xylitol and ribitol,
b) polyethylene glycol having a molecular weight of 600 to 4000 Da,
c) the disodium salts of malonic, succinic and glutaric acid, and
d) carboxymethylcellulose, and
e) sodium alginate.

Alternatively, phytase may be stabilized by chemical crosslinking with either
a) glutaraldehyde, or
b) oxidation of phytase carbohydrate residues with sodium periodate and subsequent addition of adipic acid dihydrazide.

## Description

The present invention relates to liquid and dry phytase formulations having an increased stability, preferably thermostability, which is obtained by the addition of stabilizing agents, or by crosslinking.

Although a large amount of phosphate is present in feed in form of phytate phosphorus, monogastric animals, like pigs and poultry, lack the ability to use this form of phosphate. The alkali or earth alkali salts of phytic acid occur naturally mainly in cereals. Since monogastric animals are not able to use this form of phosphate it is common practice to add phosphate to animal feed.

On the other hand an enzyme called phytase (*myo*-inositol hexakisphosphate phosphohydrolase) is known to occur in plants and in some microorganisms. Since phytase can be produced by fermentation it is known in the art to use phytase as an animal feed additive in order to enhance the nutritive value of plant material by liberation of inorganic phosphate from phytic acid (*myo*-inositol hexakisphosphate). By adding phytase to the animal feed the level of phosphorus pollution of the environment can be reduced since the animal is able to make use of the phosphate liberated from phytate by the use of phytase.

For feed application a stable preferably thermostable phytase is of general interest in order to avoid problems that may occur during the formulation (e.g. spray drying, granulation) and feed treatment processes (e.g. pelleting, extrusion, expansion) where temporarily high temperatures (up to 80-120 °C) and shear stress may affect the protein structure and lead to an undesired loss of activity.

The international patent application WO 93/16175 of Gist-Brocades describes stabilized liquid formulations of phytase. It is suggested to use as stabilizing agent urea and a water-soluble polyol whereby sorbitol, glycerol and polyethylene glycol having a molecular weight of 6000 are mentioned.

It is an object of the present invention to improve the stability, preferably rhermostability of phytase whereby stability is defined as the ability to retain activity under various conditions. This stability aspect relates to the entire life cycle of the enzyme which comprises production (fermentation, downstream processing, formulation and heat treatment of feed), distribution (transport and storage) and final application. For a commercially interesting enzyme like phytase it is important to withstand the high temperatures reached during various feed treatment processes like pelleting, extrusion and expansion (up to 80-120 °C) and to be stable during long-term storage.

The term "stability" as used in the present invention relates to all the specifications of an industrial enzyme which comprise aspects such as activity, specificity, shelf stability, mechanical stability, microbial stability, toxicity, chemical composition and physical parameters such as density, viscosity, hygroscopy, but also colour, odour and dust. A preferred aspect of the present invention relates to the stability of phytase against thermal inactivation during formulation and feed treatment processes such as pelleting, extrusion and expansion.

A major barrier to the wide use of phytases is the constraint of thermal stability (80-120 °C) required for these enzymes to withstand inactivation during feed treatment processes. The currently available industrial phrases all originate from *A*. *niger* and have a low intrinsic resistance to heat inactivation. As an alternative or in addition to molecular biological approaches the present invention enhances the stability, preferably thermostability of a protein by the addition of different additives and in another aspect by the chemical crosslinking of enzyme monomers to oligomers.

The experiments leading to the present invention were also performed with the so-called consensus phytase, a phrase developed according to a theoretical molecular biological approach which has a higher intrinsic stability compared with *Aspergillus* phytases, see European Patent Application Publication No. 897 985. In the practice of the present invention the consensus phytases specifically described in examples 3 - 13 can also be used.

The present invention discloses the use of different additives which act as stabilizing agent on the stability, preferably thermostability of the enzyme.

Regarding the temperature dependence of the specific activity of the non-formulated phytases which can preferably be used in the present invention three different groups can be formed according to their activity maximum. The activity maximum is reached at the following temperatures: for *A. fumigatus and A. niger* phytase at 55 °C, for *A. terreus* CBS and *A. nidulans* phytase at 45 °C and for consensus phytase at 65 °C. A temperature of 10-15 °C above the determined temperature maximum - where the non-formulated phytases were completely inactive - was chosen as screening point for studying the effect of the stabilizing agents on the thermostability of phytases, i.e. 60 °C for *A. nidulans* and *A. terreus* CBS phytase, 65 °C for *A. niger* and *A. fumigatus* phytase, and 75 °C for consensus phytase.

The present invention provides a stabilized, preferably thermostabilized enzyme formulation comprising phytase and at least one stabilizing agent selected from the group consisting of:
a) polyols containing five carbon atoms, preferably C₅ sugars, more preferably xylitol or ribitol,
b) polyethylene glycol having a molecular weight of 600 to 4000 Da,
c) the disodium salts of malonic, glutaric and succinic acid,
d) carboxymethylcellulose, and
e) sodium alginate

The present invention also provides a stabilized, preferably thermostabilized enzyme formulation comprising phytases which have been crosslinked:
a) by chemical reactions with glutaraldehyde; or by
b) oxidation with sodium periodate and subsequent addition of adipic acid dihydrazide

Although it would be possible to use other phytases obtained from other sources than microorganisms it is preferred to use a phytase which has been produced by microorganisms. In the present invention preferably such phytases are used which are produced by a fungus, and more preferably from the group consisting of *Aspergillus fumigatus*, *Aspergillus nidulans*, *Aspergillus terreus*, and *Aspergillus niger*. Another phytase preferably used in this invention is the so called consensus phytase. It is, however, also possible to produce such phytases by genetic engineering whereby the gene obtained from a fungus is transferred to a host organism like a bacterium (e.g. *E.coli*), a yeast or another fungus, for further details, see e.g. European Patent Application Publication No. 684313 and European Patent Application Publication No. 897 010.

The term enzyme formulation comprises all liquid and dry formulations in which the enzyme phytase may be commerciallized. Preferably, the source of phrase for such a formulation is a rather raw, liquid preparation obtained from the fermentation broth. For the preparation of a liquid phytase formulation the selected stabilizing agents are added or the phytase is crosslinked. To obtain a stabilized, preferably thermostabilized dry formulation the phrase is a) spray dried or granulated in the presence of the selected stabilizing agents, or b) chemically crosslinking.

In one preferred embodiment the liquid enzyme formulation comprises as stabilizing agent polyethylene glycol whereby the polyethylene glycol is present in a concentration of 10-50% (w/w) in the final formulation.

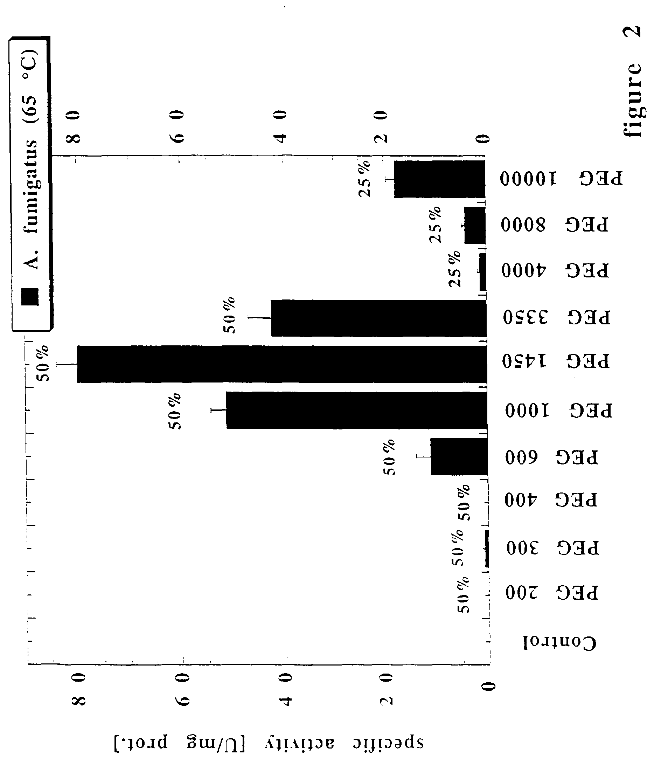

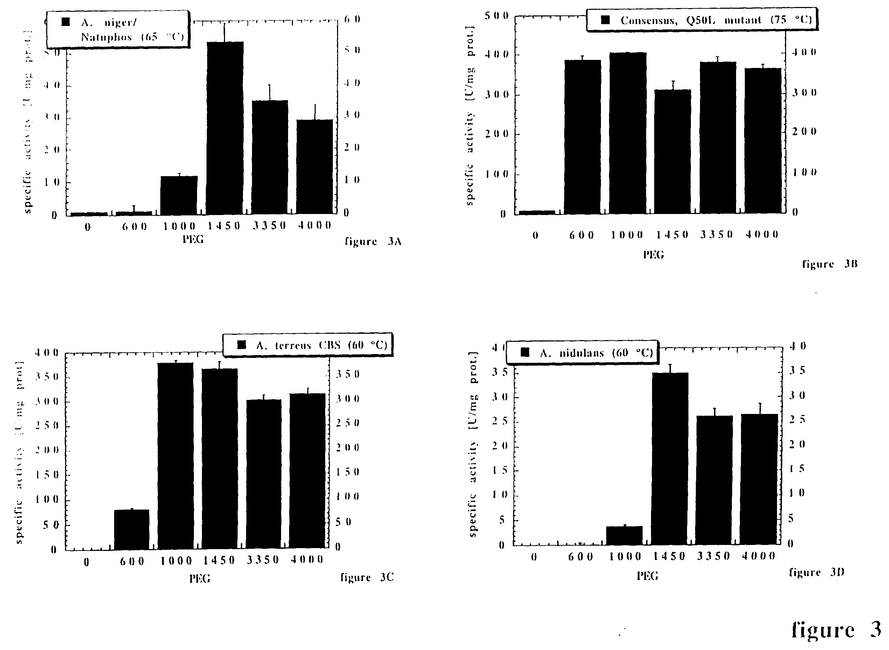

Preferably the enzyme formulation comprises polyethylene glycol having a molecular weight of 1000-3350 Da. It is especially preferred to use a polyethylene glycol having a molecular weight of about 1450. Polyethylene glycols with molecular weights slightly outside of the preferred range (600 Da and 4000 Da, respectively) showed still reasonable effect but are less preferred. The stabilizing effect of polyethylene glycol was shown to be molecular weight-dependent (see Figures 2 and 3).

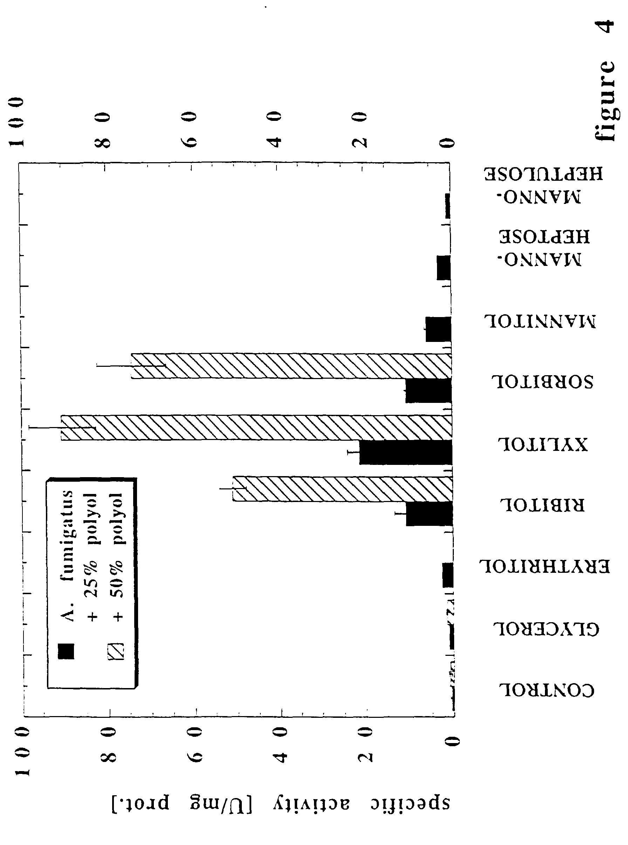

In another preferred embodiment of the present invention the stabilizing agent is xylitol or ribitol. Both are sugar alcohols having a five carbon atom structure. Xylitol and ribitol are preferably used in a concentration of 20 to 60% (w/w) in the final liquid formulation. Surprisingly xylitol and ribitol as stabilizing agents of, e. g. , *A. fumigatus* phytase increased the specific activity measured at 65 °C to 11-12 U/mg at a concentration of 12.5%, and to 51-90 U/mg at a concentration of 25% of the polyol (see Figure 4).

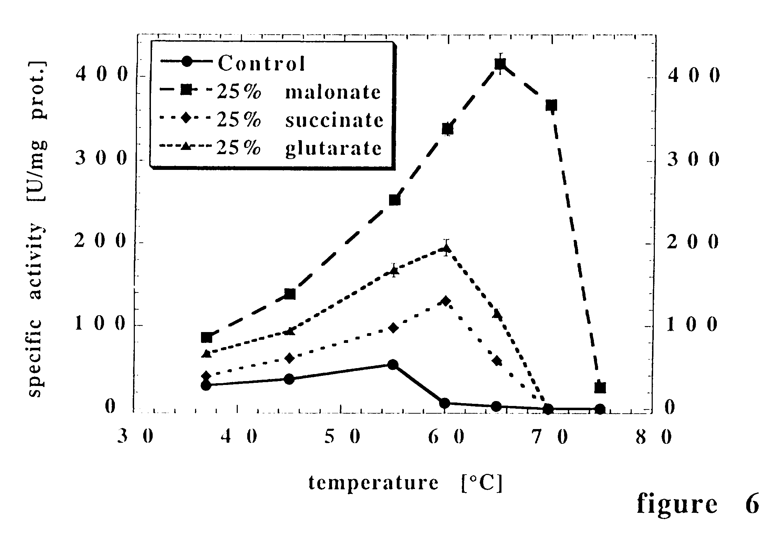

In another embodiment of the present invention the liquid enzyme formulation comprises as stabilizing agent the disodium salts of glutaric, succinic or malonic acid whereby the concentration of the salt in the final formulation ranges between 10 and 30% (w/w). The addition of malonate, succinate and glutarate at a concentration of 25% resulted in a significant increase in *A. fumigatus* phytase thermostability with considerable activity still being detected at 70 °C for malonate and 65 °C for succinate and glutarate as can be seen in Figure 6.

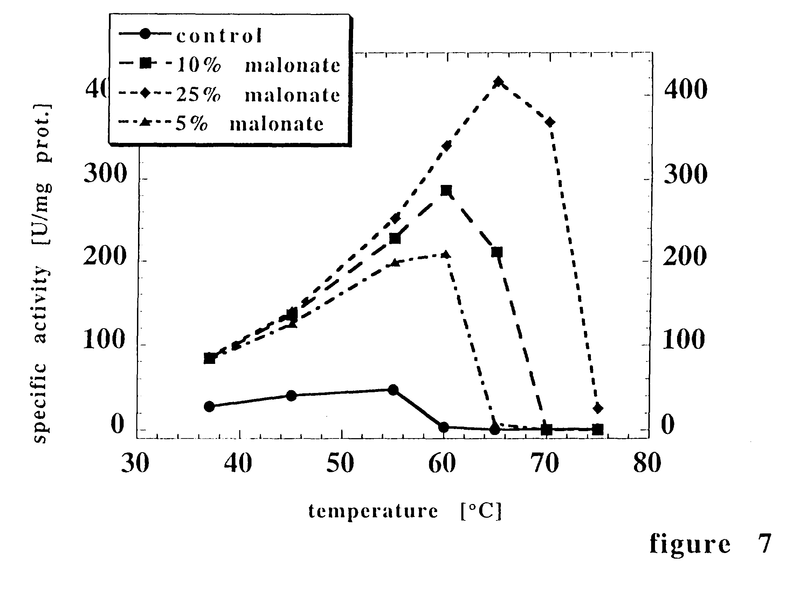

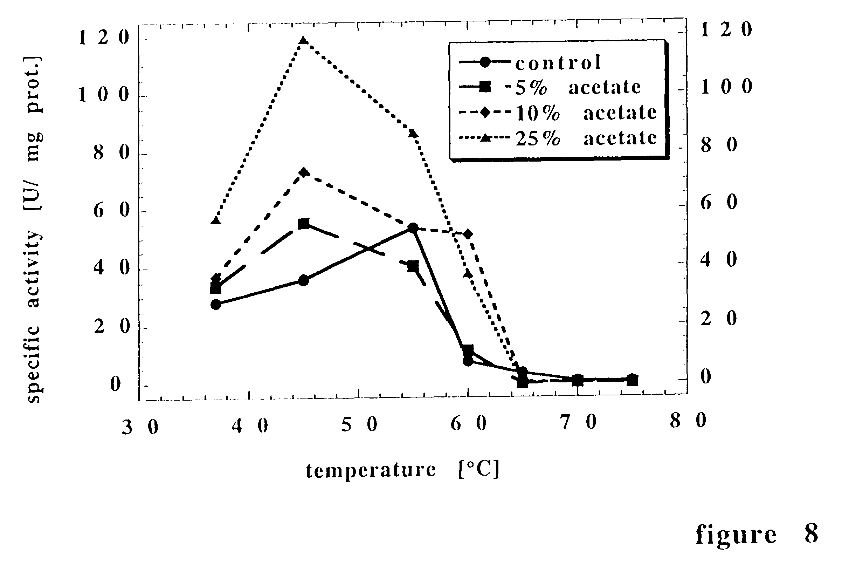

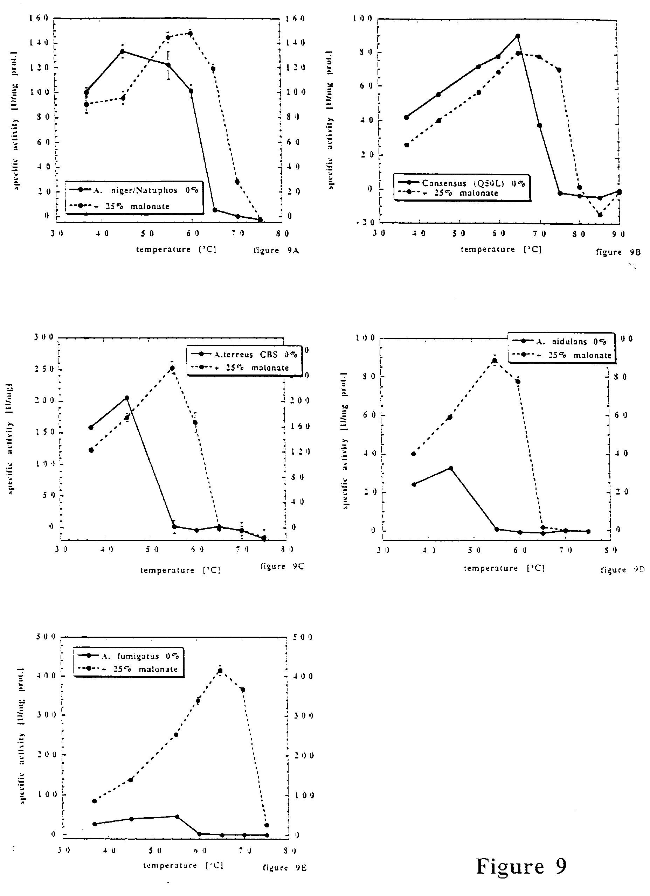

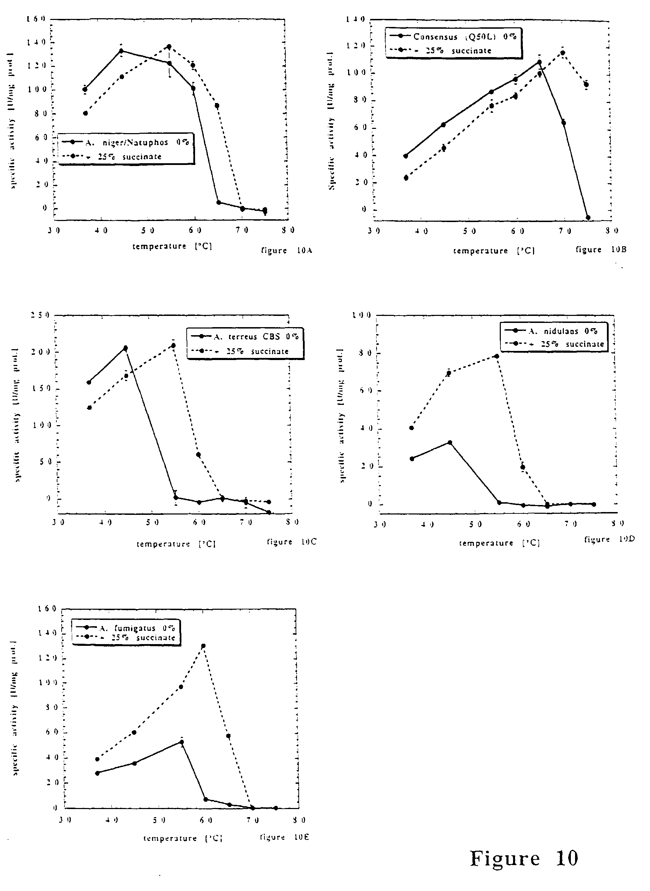

In addition thereto the carboxylates stimulated *A. fumigatus* phytase activity measured at 37 °C with an approximately 4-fold increase in phytase activity beeing observed in the case of malonate, a 2-fold increase for succinate and minor effects for glutarate. Investigation of different concentrations (5, 10 and 25%) of malonate showed that thermostabilization of *A. fumigatus* phytase is concentration-dependent whereas stimulation of enzymatic activity, at least in this concentration range, is not (see Figure 7). In contrast to these findings different concentrations (5, 10 and 25%) of sodium acetate which is a monocarboxylic acid, caused an up to 2-fold increase in specific activity of *A. fumigatus* phytase at 37 °C, but had only minor effects on the thermostability of the protein (see Figure 8). Therefore, it may be concluded that carboxylate groups are responsible for activity modulation whereas bifunctional dicarboxylates stabilize phytases possibly by ionic interactions. The sodium malonate and succinate generally increased the thermostability of *A. nidulans*, *A. terreus* CBS, *A. niger* and consensus phytase by 5-15 °C. On the other hand stimulation of phytase activity was only observed for *A. nidulans* and *A. fumigatus* phytase both having rather low specific activity but not for *A. terreus* CBS, *A. niger* and consensus phytase (see Figures 9 and 10).

In another embodiment of the present invention the enzyme formulation comprises as stabilizing agent the polymers carboxymethylcellulose and/or sodium alginate whereby the concentration in the final liquid formulation is between 1 and 20% preferably 1 and 10% (w/w). The addition of these polymeres to *A. fumigatu*s phytase preparations resulted in a significant 5 to 10% increase of phytase thermostability.

In another embodiment of the present invention the enzyme formulation comprises as stabilizing agent alginate, preferably sodium alginate and most preferably in a concentration of 1 to 10% (w/w) in the final liquid formulation.

In a further embodiment of the present invention the enzyme formulation comprises crosslinked phytase. For the preparation of such a stabilized phytase form, glutaraldehyde is added to the phytase at a concentration resulting in an oligomerization of the protein.

In another embodiment the enzyme formulation comprises phytase which has been crosslinked via its carbohydrate chains. Crosslinking involves as a first step periodate oxidation of the carbohydrate residues followed by reaction of the generated aldehyde groups with adipic acid dihydrazide.

Depending on the conditions employed, the crosslinking reaction can lead to various derivatives of an enzyme, namely
a) modified enzyme molecules that have reacted with only one hydrazide group of adipic acid dihydrazide,
b) intramolecularly crosslinked enzymes, with or without intermolecular crosslinking, and
c) intermolecularly crosslinked, soluble oligomers or insoluble polymers.

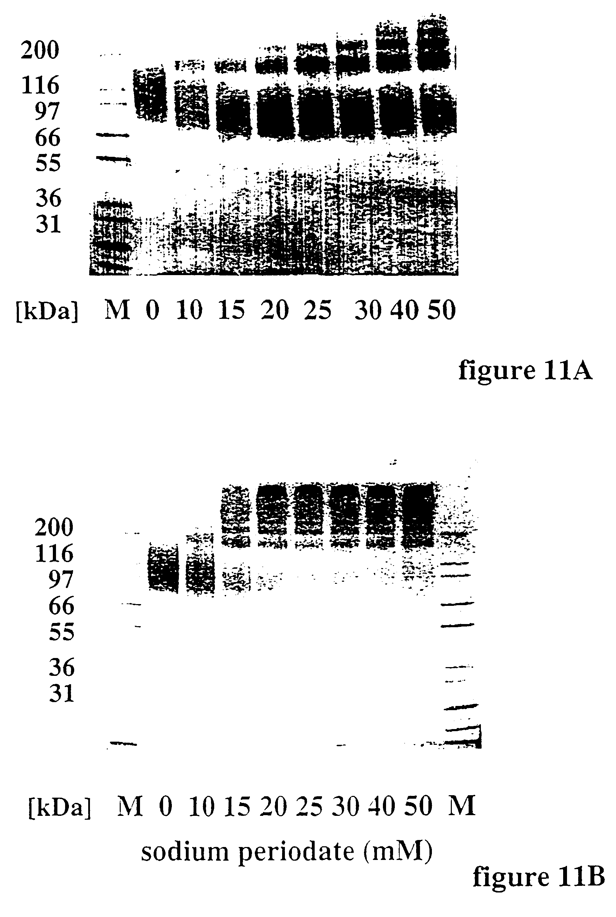

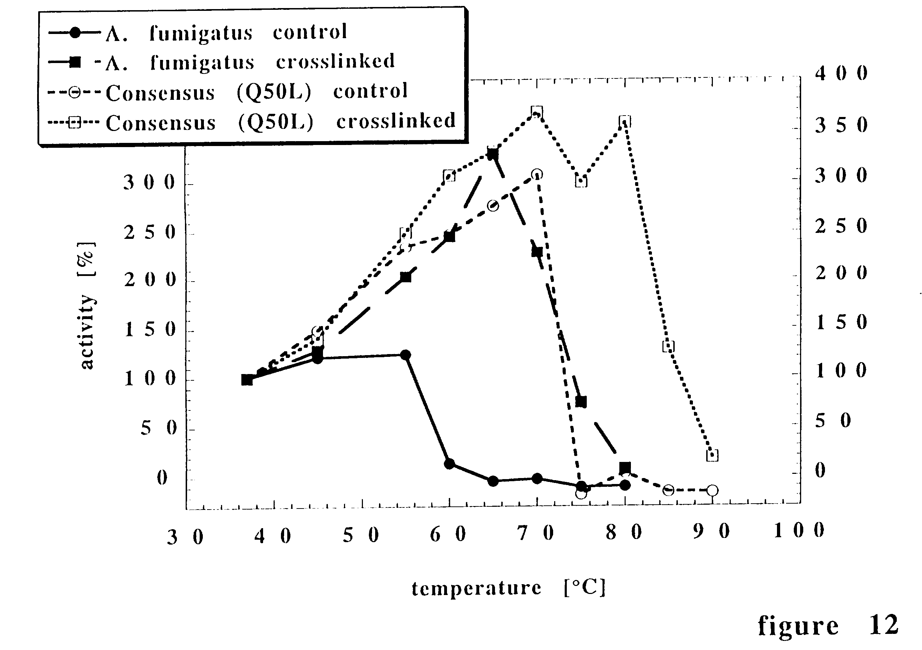

In most cases the reaction results in a mixture of several forms. Crosslinking of *A. fumigatus* and consensus phytase both expressed in *Hansenula polymorpha* resulted in the formation of oligomeric forms. The degree of crosslinking could be controlled effectively by changing the degree of enzyme oxidation. An optimal thermostabilization of phytase has been observed at a concentration of 50 mM sodium periodate applied to a 5 mg/ml phytase solution. For both phytases an increase in thermostability between 10 and 15 °C has been observed (see Figure 12). It should be noted that the oxidized phytases formed a significant amount of dimers, trimers and tetramers even without addition of adipic acid dihydrazide (see Figure 11A).

Another aspect of the present invention concerns the use of the listed stabilizers as additives for the production of dry/solid phytase formulations. In this embodiment of the present invention the addition of stabilizers (1 to 20% (w/w) of xylitol/ribitol, 1 to 20% (w/w) of polyethylene glycols with a molecular weight preferably between 1000 and 3350 Da and/or 1 to 20% (w/w) of dicarboxylates like malonate, succinate and glutarate, and/or 1 to 10% (w/w) of the polymers carboxymethlycellulose and/or alignate, preferably sodium alginate disolved in 100-200 ml phytase liquid (crosslinked or non-crosslinked) or added as solid compounds to the standard granulation mixture (containing ligninsulfonat as binder, silica and gipsum as carrier) Such formulation can result in an increased recovery (up to 20%) of phytase activity determined after a high shear granulation process which included a drying step of the granulates on a fluid bed dryer at 45°C for 15 mm. In addition such granulates which contain stabilizers can show, when mixed with feed, an increased recovery of enzymatic activity after the feed treatment (e.g. a pelleting process at 85°C) compared to granulates without such additives.

Another aspect of the present invention concerns methods of preparing feed compositions for monogastric animals, whereby the feed is supplemented with a thermostabilized dry or liquid enzyme formulation according to any of claims (1-13). The phytase supplemented feed can be subjected on several methods of feed processing like extrusion, expansion and pelleting, where temporarily high temperatures may occure and thermostabilization is an advantage.

The stabilized enzyme formulation of the present invention can be appllied for example on feed pellets. The thermostabilized liquid enzyme formulation may be diluted with tap water to yield a solution having the desired activity of phytase (100 - 200 phytase units/g solution). The feed pellets can be transferred to a mechanical mixer and the diluted enzyme formulation is sprayed onto the feed pellets while being agitated in order to yield a homogeneous product with an added phytase activity of for example 500 units phytase/kg feed pellets. Alternatively the dry or liquid enzyme formulation can be directly mixed with the mash feed before this mixture is then subjected to a process such as pelleting, expansion or extrusion.

In a further aspect the present invention concerns a method of providing a monogastric animal with its dietary requirement of phosphorus wherein the animal is fed with a feed according to the present invention and whereby no additional phosphorus is added to the feed.

The results of the experiments of the present invention are shown in the following Figures.

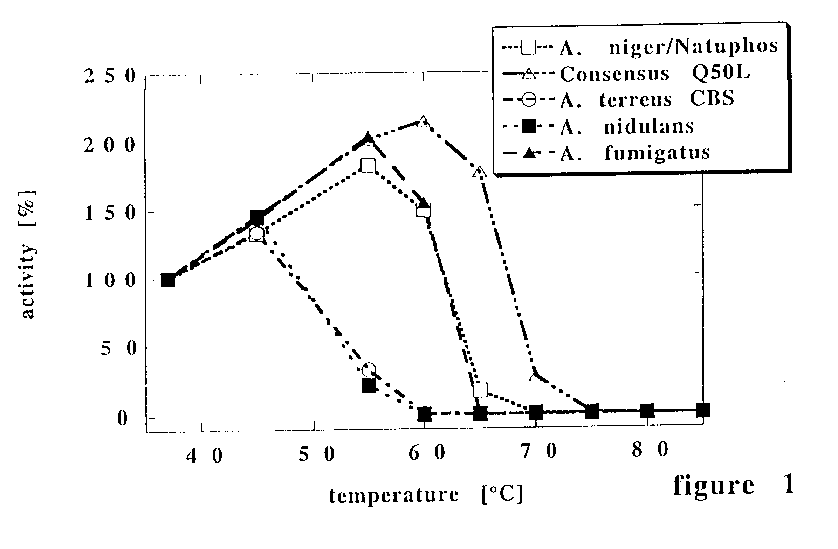

**Figure 1.** Comparison of the temperature dependence of activity of *A. fumigatus, A. nidulans, A. terreus* CBS, *A. niger* and consensus phytase measured under standard assay conditions as described in Example 1.
**Figure 2.** Effect of different polyethylene glycols on the specific activity of *A. fumigatus* phytase at 65 °C.
**Figure 3.** Effect of 50% solutions of polyethylene glycols with different molecular weights on the thermostability of *A. niger,* consensus, *A. terreus* CBS and *A. nidulans* phytase. The specific activities were measured at 60 °C for *A. terreus* CBS and *A. nidulans* phytase, at 65 °C for *A. niger* phytase and at 75 °C for consensus phytase.
**Figure 4.** Effect of 25 and 50% solutions of different polyols on the specific activity of *A. fumigatus* phytase at 65 °C.
**Figure 5.** Temperature dependence of activity of *A. niger* (A), consensus (B), *A. nidulans* (C) and *A. terreus* CBS (D) phytase in the presence of 50% xylitol as additive.
**Figure 6.** Temperature dependence of activity of *A. fumigatus* phytase in the presence of 25% concentrations of disodium malonate, succinate and glutarate.
**Figure 7.** Temperature dependence of activity of *A. fumigatus* phytase in the presence of 5, 10 and 25% disodium malonate.
**Figure 8.** Temperature dependence of activity of *A. fumigatus* phytase in the presence of 5, 10 and 25% sodium acetate.
**Figure 9.** Temperature dependence of activity of *A. niger* (A), consensus (B), *A. terreus* CBS (C) and *A. nidulans* (D) phytase in the presence of 25% disodium malonate.
**Figure 10.** Temperature dependence of activity of *A. niger* (A), consensus (B), *A. terreus* CBS (C) and *A. nidulans* (D) phytase in the presence of 25% disodium succinate.
**Figure 11.**
   **A)** SDS-PAGE of *A. fumigatus* phytase samples after incubation with different concentrations of sodium periodate.
   **B)** SDS-PAGE of the different oxidized *A. fumigatus* phytase samples from (A) after subsequent crosslinking with adipic acid dihydrazide.
**Figure 12** Temperature dependence of activity of *A. fumigatus* phytase and consensus phytase before and after crosslinking with periodate/adipic acid dihydrazide.

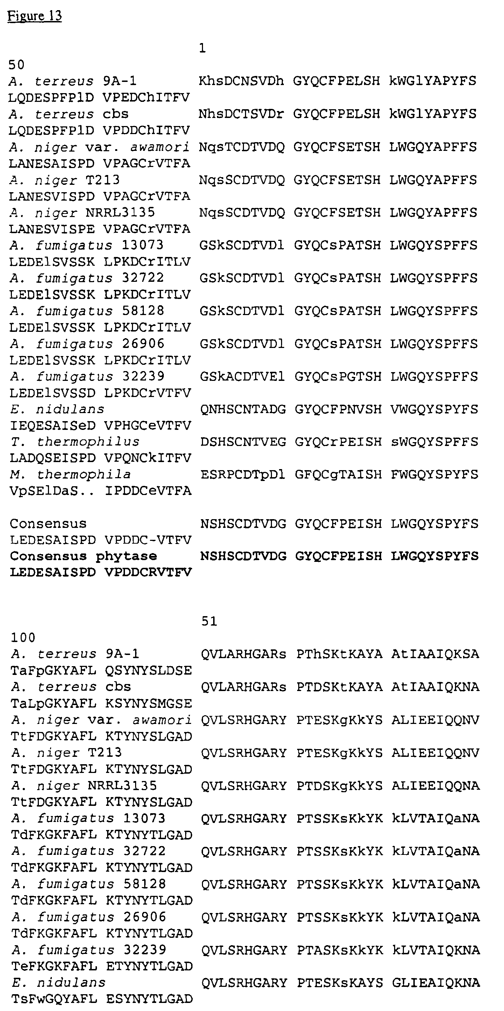

**Figure 13** Design of the consensus phytase sequence. The letters represent the amino acid residues in the one-letter code. The following sequences were used for the alignment: *phyA* from *Aspergillus terreus* 9A-1 (Mitchell et al, 1997; from amino acid (aa) 27), *phyA* from *A. terreus* cbs116.46; (van Loon et al., 1998; from aa 27), *phyA* from *Aspergillus niger* var. *awamori* (Piddington et al, 1993; from aa 27), *phyA* from *A. niger* T213; from aa 27), *phyA* from *A*. *niger* stain NRRL3135 (van Hartingsveldt et al, 1993; from aa 27), *phyA* from *Aspergillus fumigatus* ATCC 13073 (Pasamontes et al, 1993; from aa 25), *phyA* from *A. fumigatus* ATCC 32722 (van Loon et al, 1998; from aa 27), *phyA* from *A. fumigatus* ATCC 58128 (van Loon *et al.,* 1998; from aa 27), *phyA* from *A. fumigatus* ATCC 26906 (van Loon et al, 1998; from aa 27), *phyA* from *A. fumigatus* ATCC 32239 (van Loon et al, 1998; from aa 30), *phyA* from *Emericella nidulans* (Pasamontes et al, 1997a; from aa 25), *phyA* from *Talaromyces rhermophilus* (Pasamontes et al, 1997a; from aa 24), and *phyA* from *Myceliophthora thermophila (*Mitchell et al, 1997; from aa 19). The alignment was calculated using the program PILEUP. The location of the gaps was refined by hand. Capitalized amino acid residues in the alignment at a given position belong to the amino acid coalition that establish the consensus residue. In bold, beneath the calculated consensus sequence, the amino acid sequence of the finally constructed consensus phytase (Fcp) is shown. The gaps in the calculated consensus sequence were filled by hand according to principals stated in Example 3.

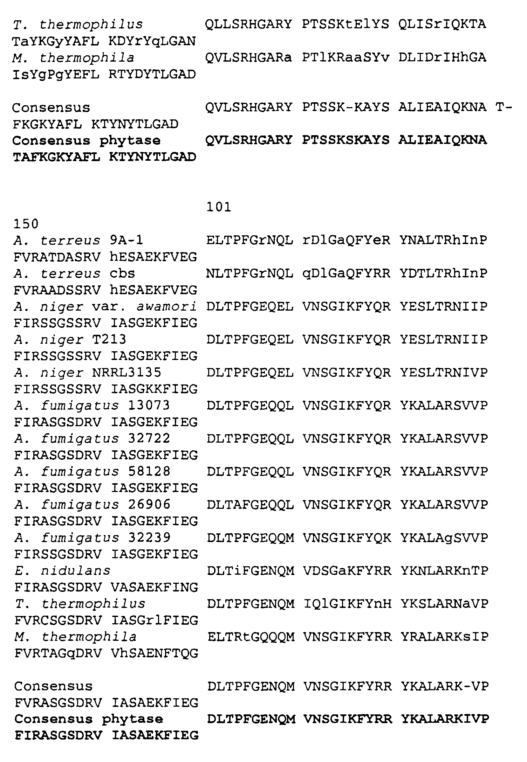

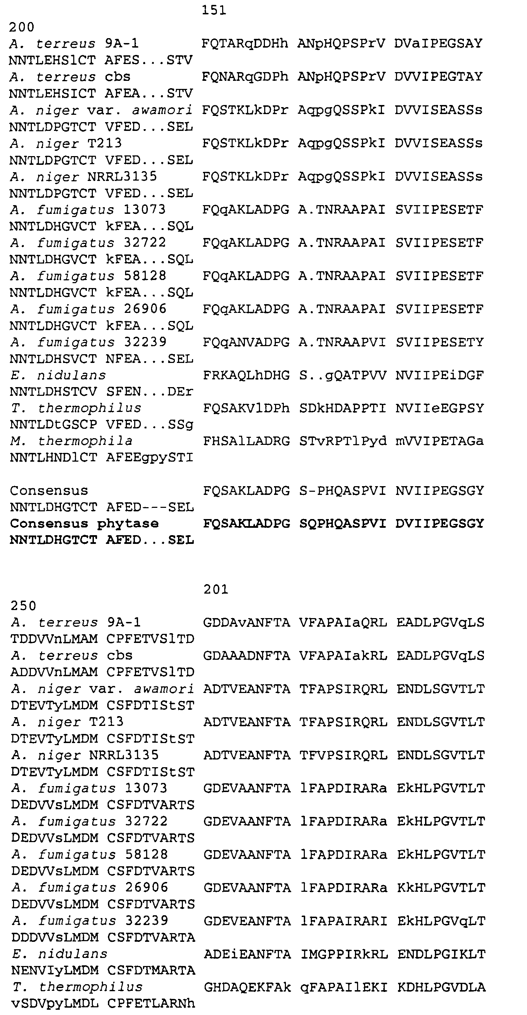

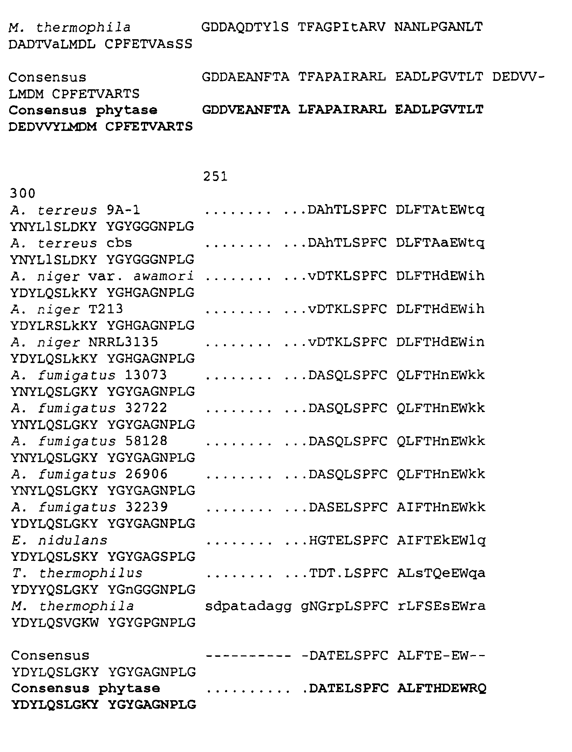

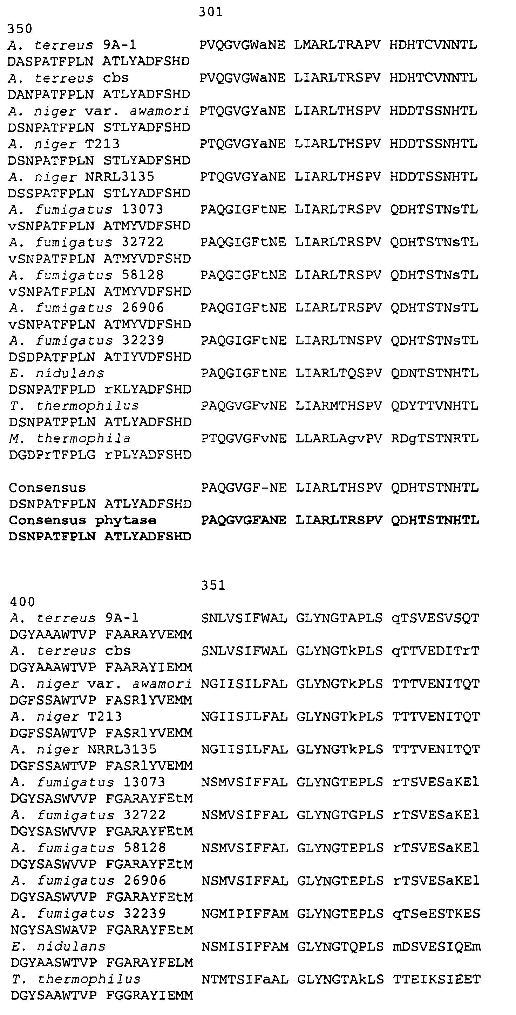

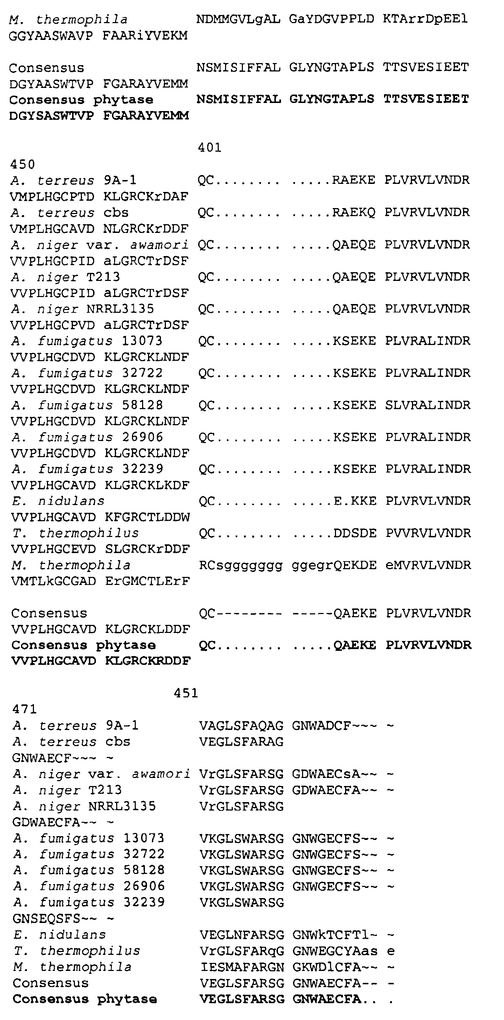

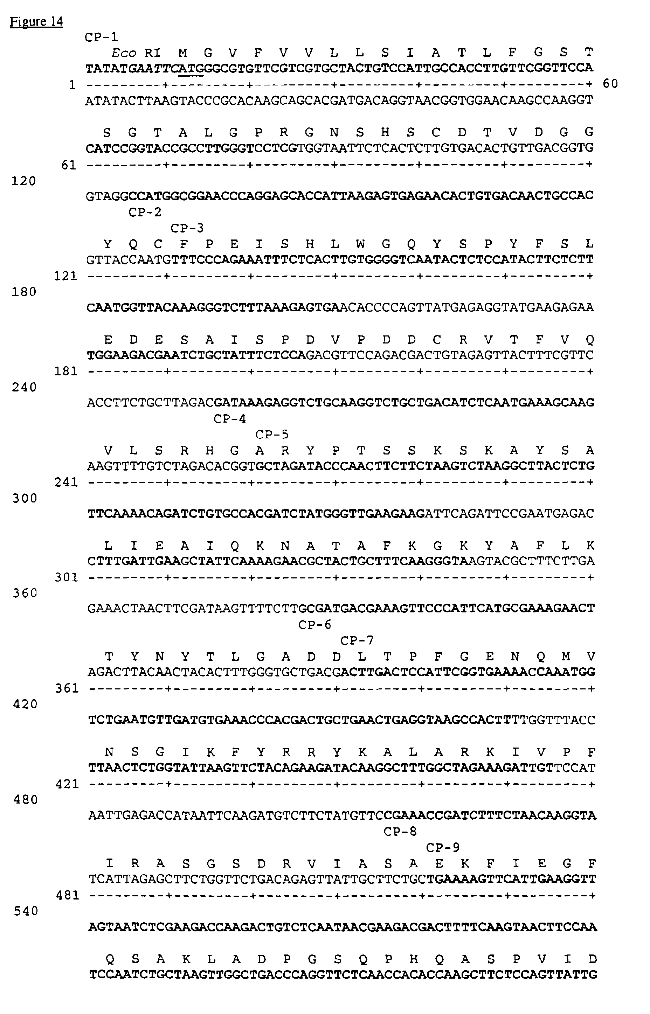

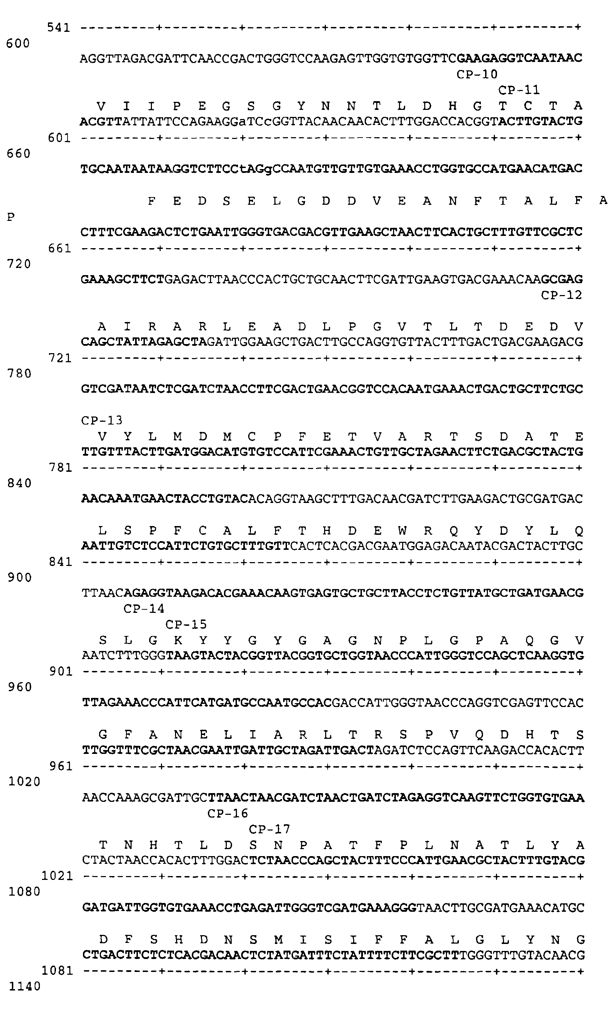

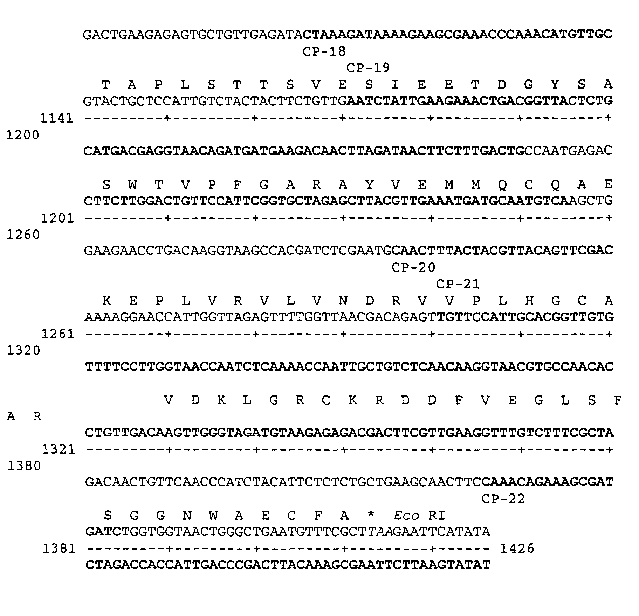

**Figure 14** DNA sequence of the consensus phytase-1 gene (*fcp*) and of the primers used for the gene construction. The calculated amino acid sequence (Figure 13) was convened into a DNA sequence using the program BACKTRANSLATE (Devereux *et al.,* 1984) and the codon frequency table of highly expressed yeast genes (GCG program package, 9.0). The signal peptide of the phytase from *A. terreus* cbs.116.46 was fused to the *N-*terminus. The bold bases represent the sequences of the oligonucleotides used to generate the gene. The names of the respective oligonucleotides are alternately noted above or below the sequence. The underlined bases represent the start and stop codon of the gene. The bases written in italics show the two introduced *Eco* RI sites.

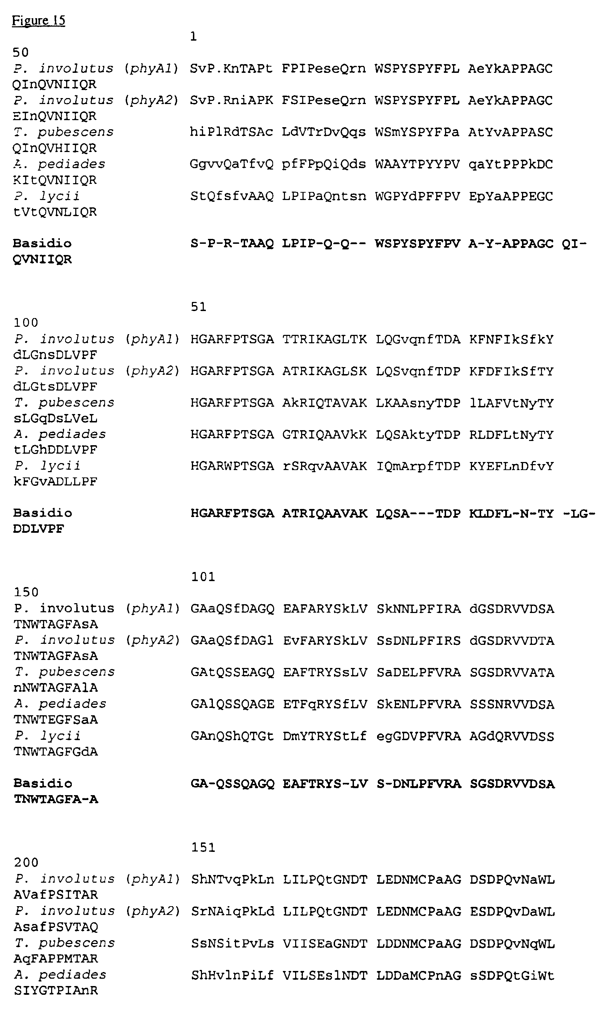

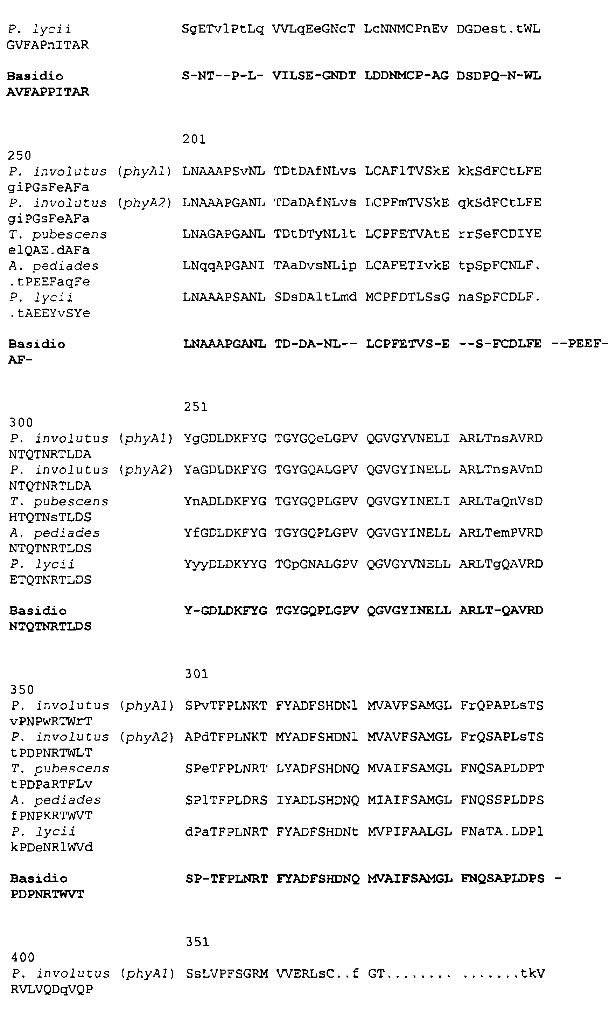

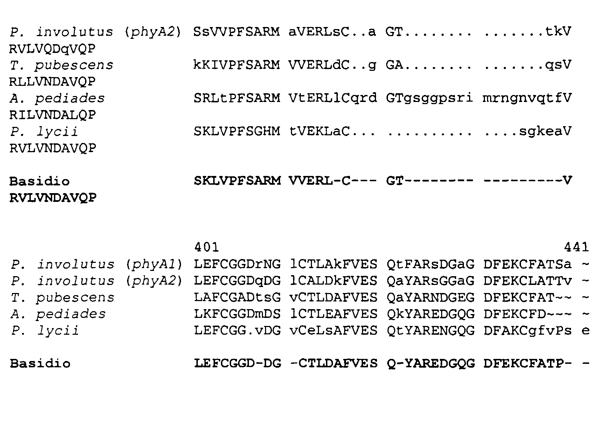

**Figure 15** Alignment and consensus sequence of five *Basidiomycetes* phytases. The letters represent the amino acid residues in the one-letter code. The amino acid sequences of the phytases from *Paxillus involutus*, phyA1 (aa 21) and phyA2 (aa 21, WO 98/28409), *Trametes pubescens* (aa 24, WO 98/28409), *Agrocybe pediades* (aa 19, WO 98/28409), and *Peniophora lycii* (aa 21, WO 98/28409) starting with the amino acid residues mentioned in parentheses, were used for the alignment and the calculation of the corresponding consensus sequence called "Basidio" (Example 4). The alignment was performed by the program PILEPUP. The location of the gaps was refined by hand. The consensus sequence was calculated by the program PRETTY. While a vote weight of 0.5 was assigned to the two *P. involutus* phytases, all other genes were used with a vote weight of 1.0 for the consensus sequence calculation. At positions, where the program was not able to determine a consensus residues, the Basidio sequence contains a dash. Capitalized amino acid residues in the alignment at a given position belong to the amino acid coalition that establish the consensus residue.

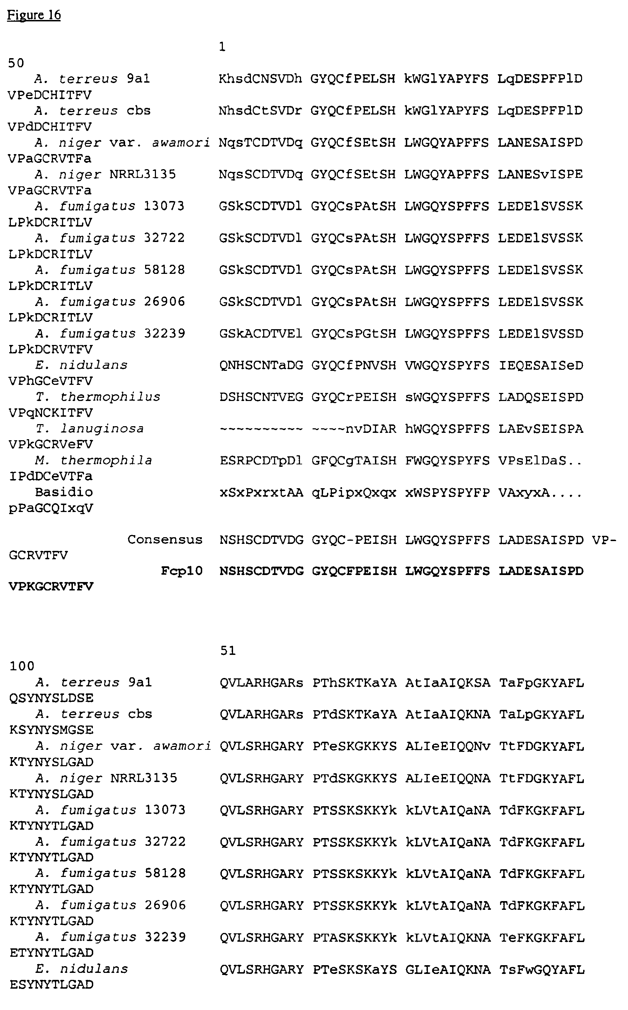

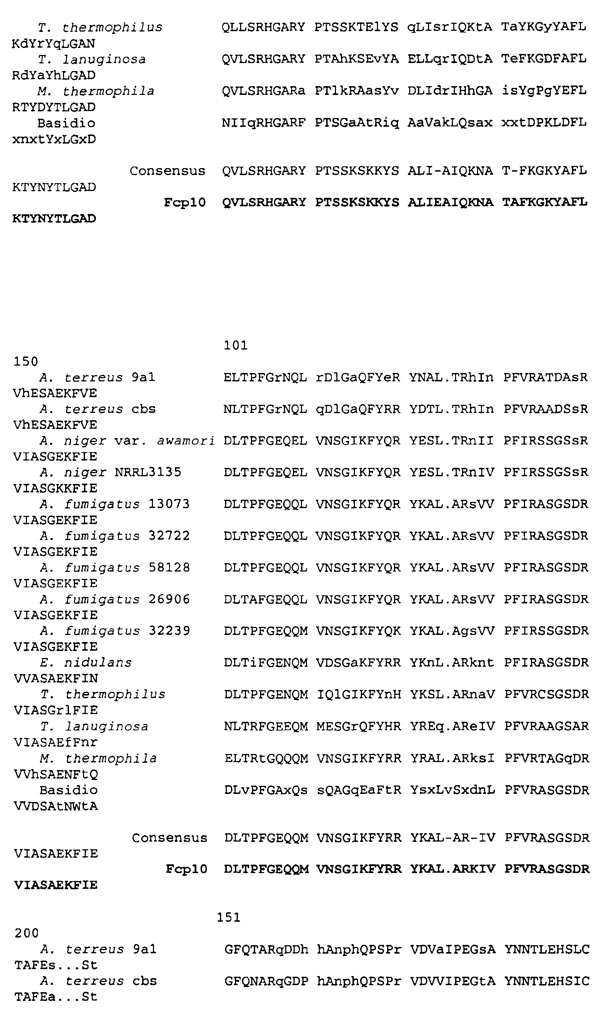

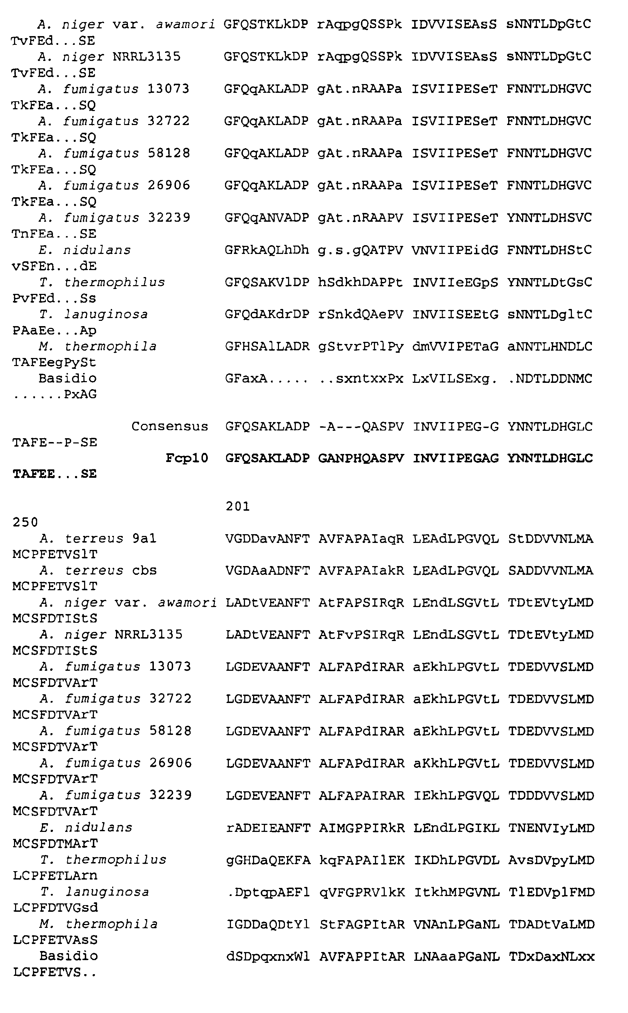

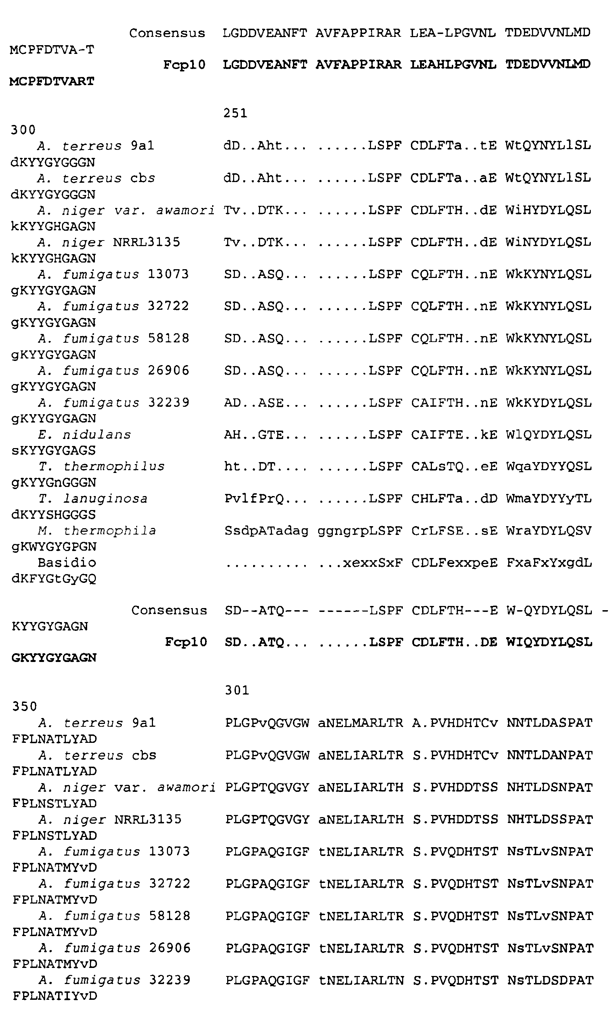

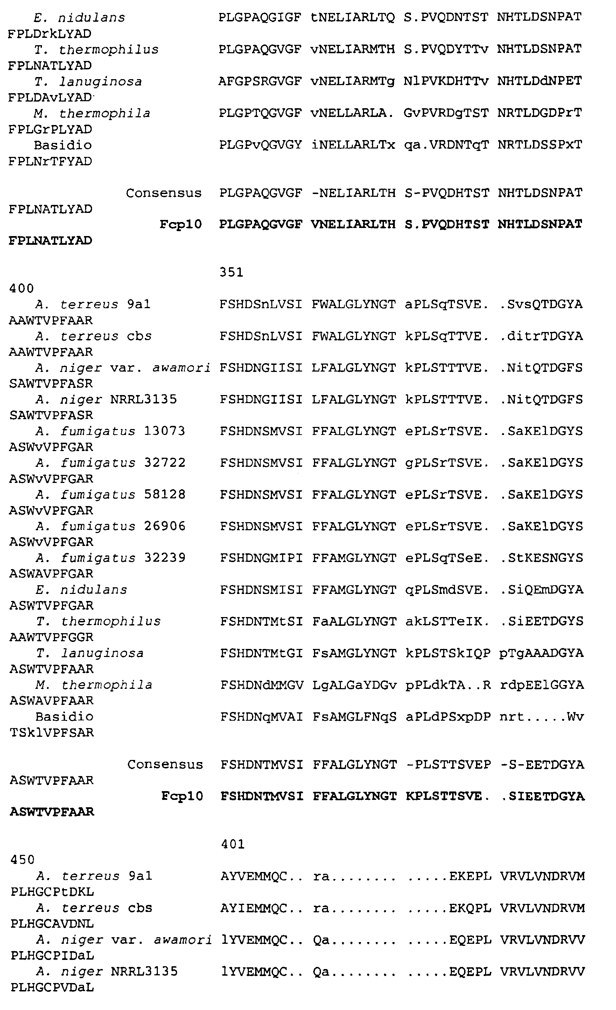

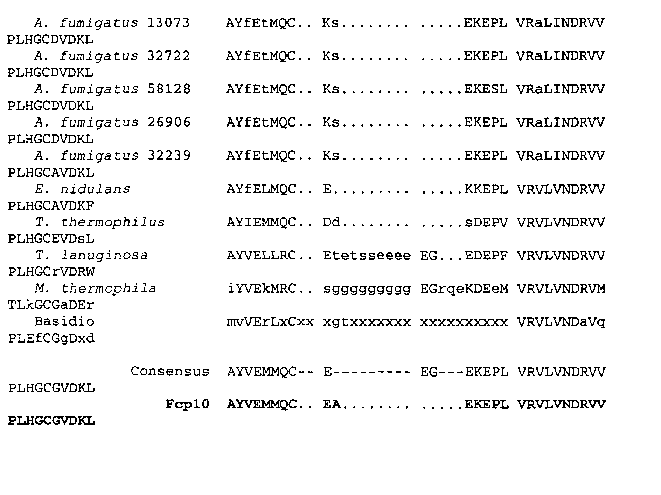

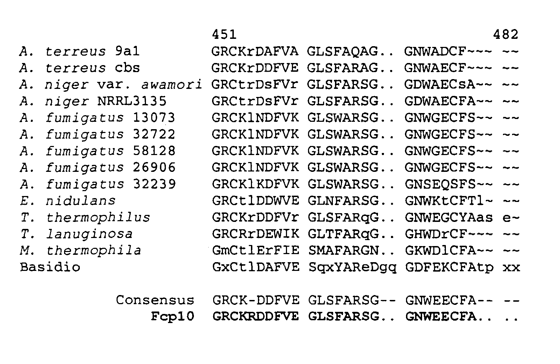

**Figure 16** Design of consensus phytase-10 amino acid sequence. Adding the phytase sequence of *Thermomyces lanuginosa* (Berka *et al.,* 1998) and the consensus sequence of the phytases from five *Basidiomycetes* to the alignment of Figure 13, an improved consensus sequence was calculated by the program PRETTY. Additionally, the amino acid sequence of *A. niger* T213 was omitted, therefore, using a vote weight of 0.5 for the remaining *A. niger* phytase sequences. For further information see Example 14.

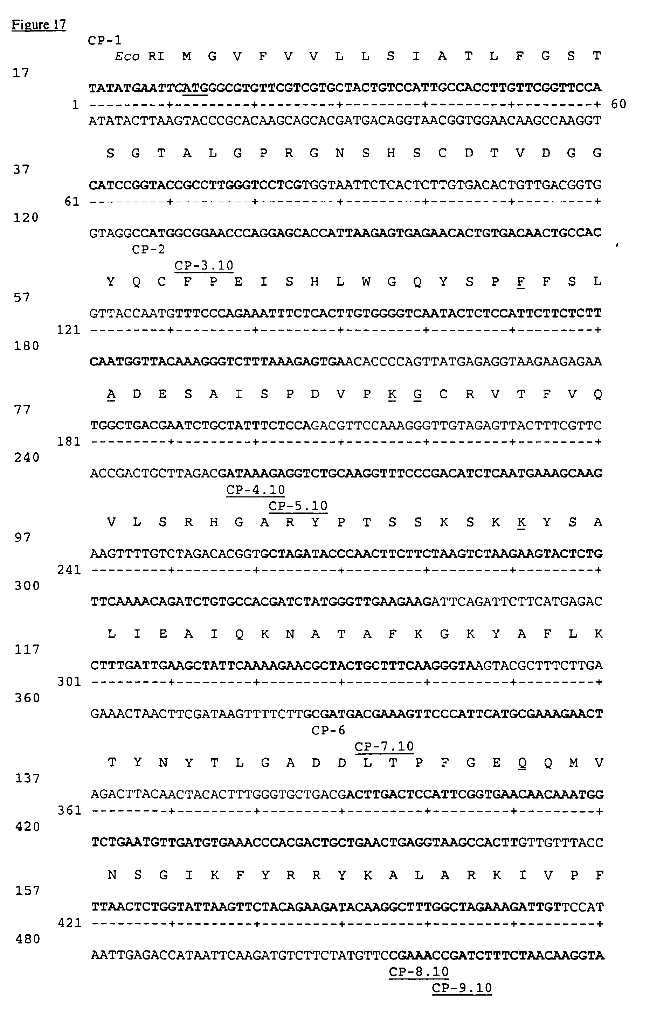

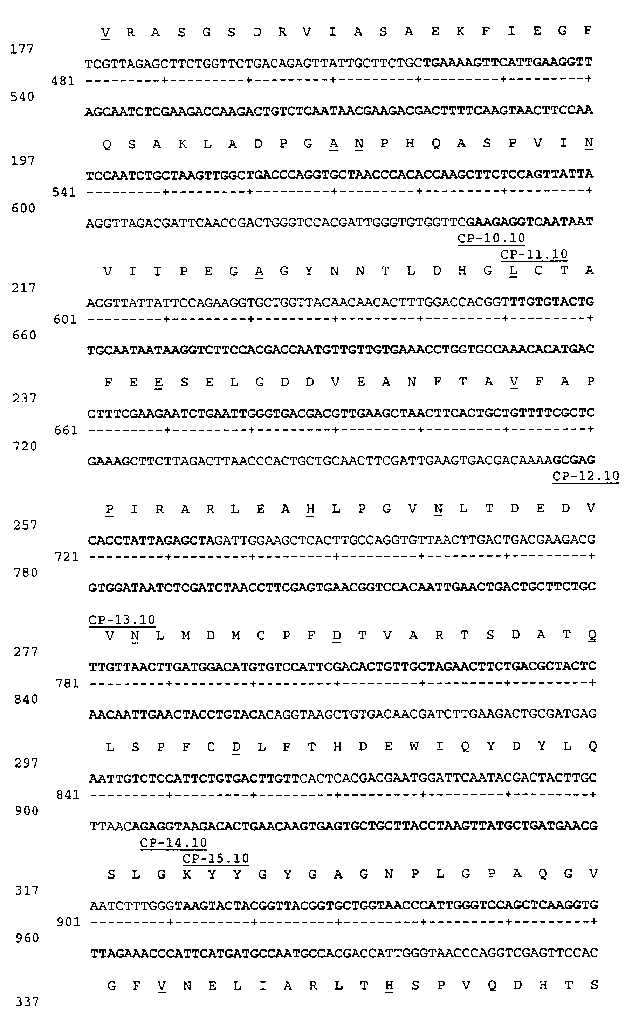

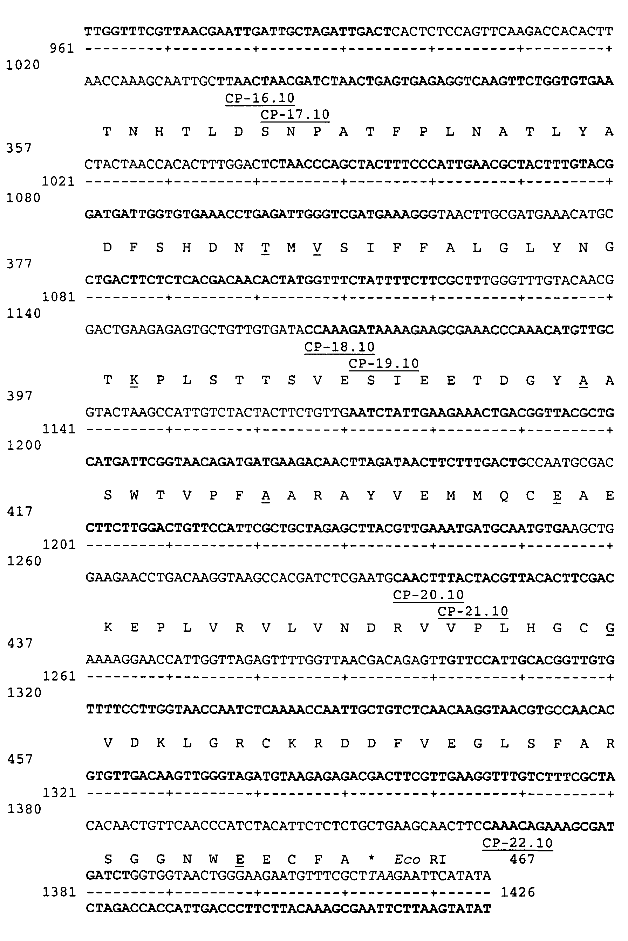

**Figure 17** DNA and amino acid sequence of consensus phytase-10. The amino acid sequence is written above the corresponding DNA sequence using the one-letter code. The sequence of the oligonucleotides which were used to assemble the gene are in bold letters. The label of oligonucleotides and the amino acids, which were changed compared to those for consensus phytase -1, are underlined and their corresponding triplets are highlighted in small cases. The *fcp*10 gene was assembled from the following oligonucleotides: CP-1, CP-2, CP-3.10, CP-4.10, CP-5.10, CP-6, CP-7.10, CP-8.10, CP-9.10, CP-10.10, CP-11.10, CP-12.10, CP-13.10, CP-14.10, CP-15.10, CP-16.10, CP-17.10, CP18.10, CR-19.10, CP-20.10, CP-21.10, CP-22.10. The newly synthesized oligonucleotides are additionally marked by number 10. The phytase contains the following 32 exchanges: Y54F, **E58A**, D69K, D70G, A94K, N134Q, I158V, S187A, Q188N, **D197N**, S204A, T214L, D220E, L234V, A238P, D246H, T251N, Y259N, **E267D**, E277Q, A283D, **R291I**, A320V, **R329H**, **S364T**, I366V, **A379K**, S396A, **G404A**, Q415E, A437G, A463E. The mutations accentuated in bold letters revealed a stabilizing effect on consensus phytase-1 as tested as single mutation in consensus phytase-1.

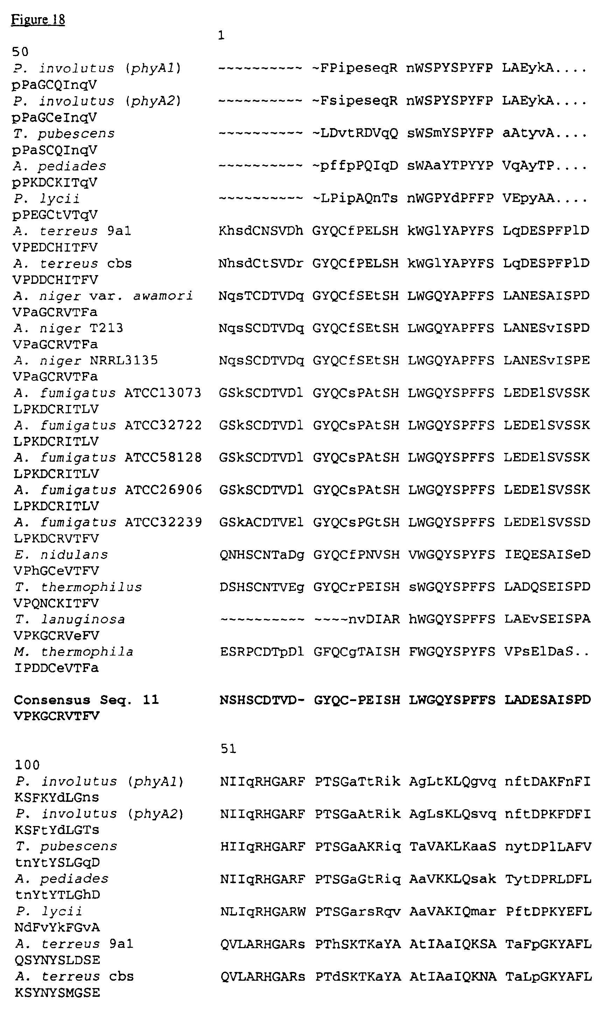

**Figure 18** Alignment for the design of consensus phytase-11. In contrast to the design of consensus phytase-10, for the design of the amino acid sequence of consensus phytase-11, all *Basidiomycetes* phytases were used as independent sequences using an assigned vote weight of 0.2 for each *Basidiomycetes* sequence. Additionally, the amino acid sequence of *A. niger* T213 was used in that alignment, again.

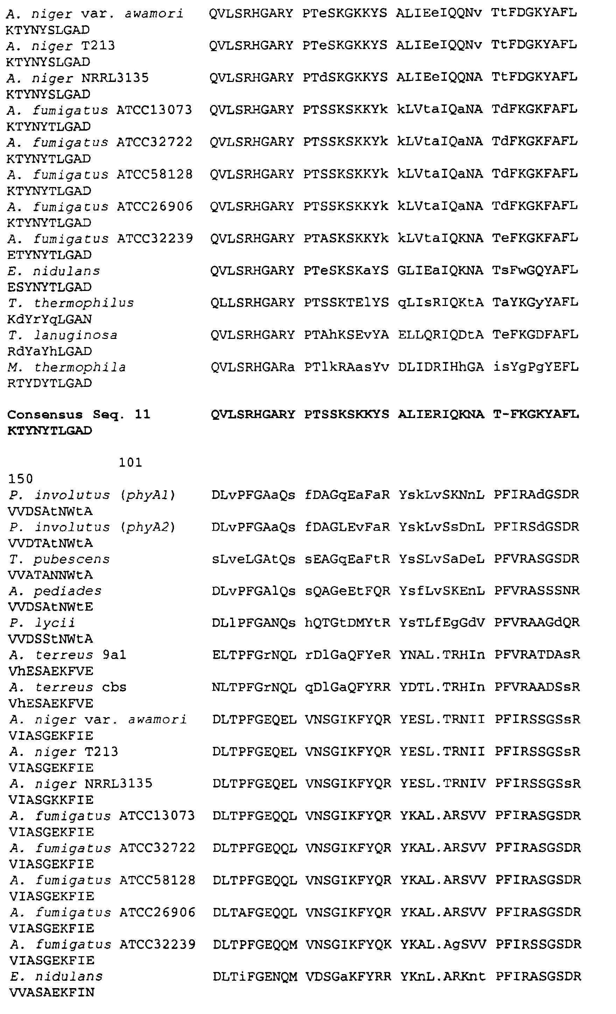

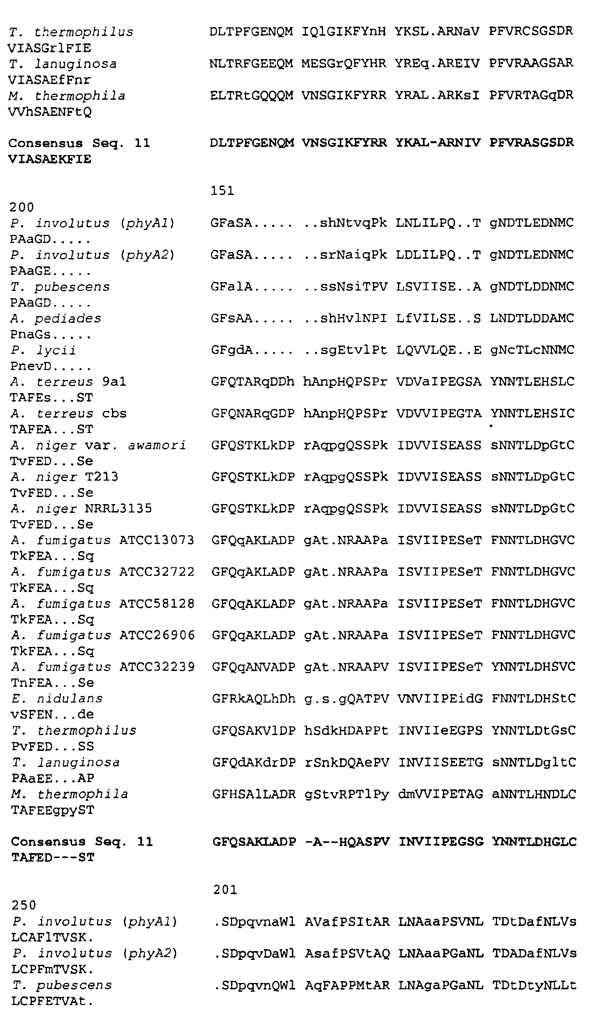

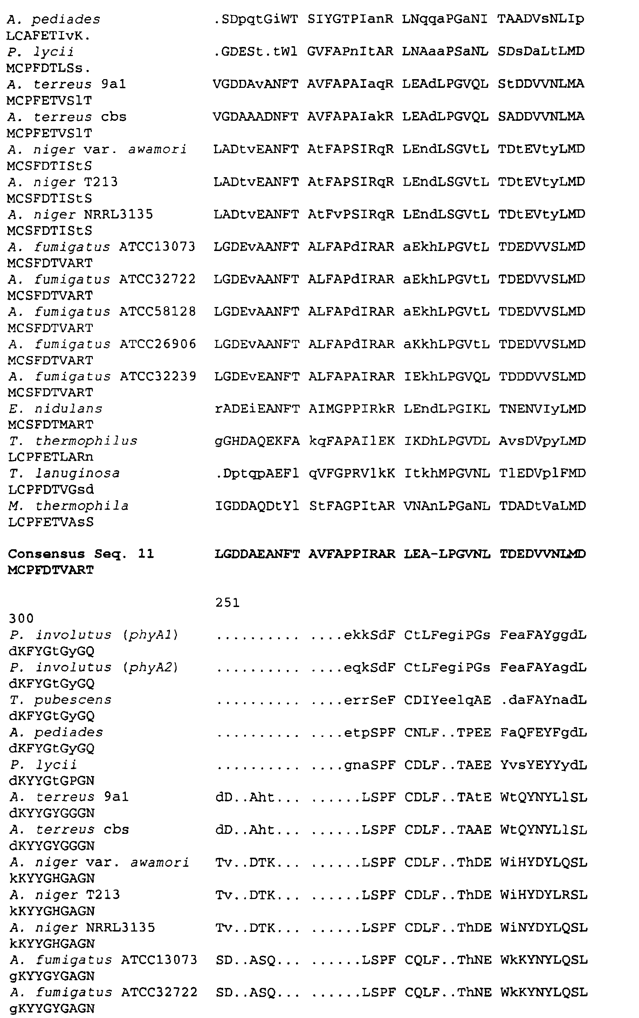

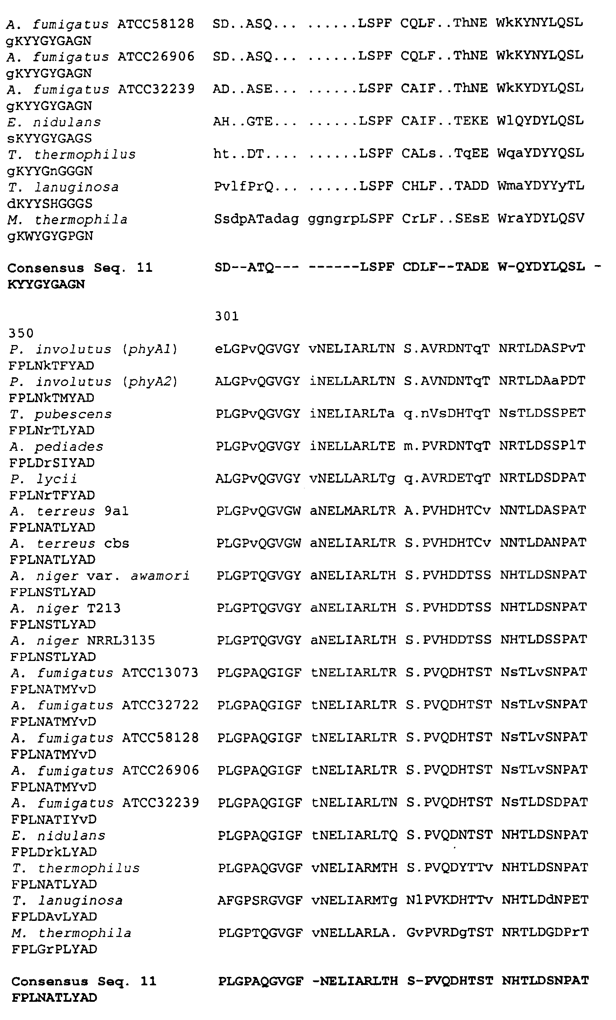

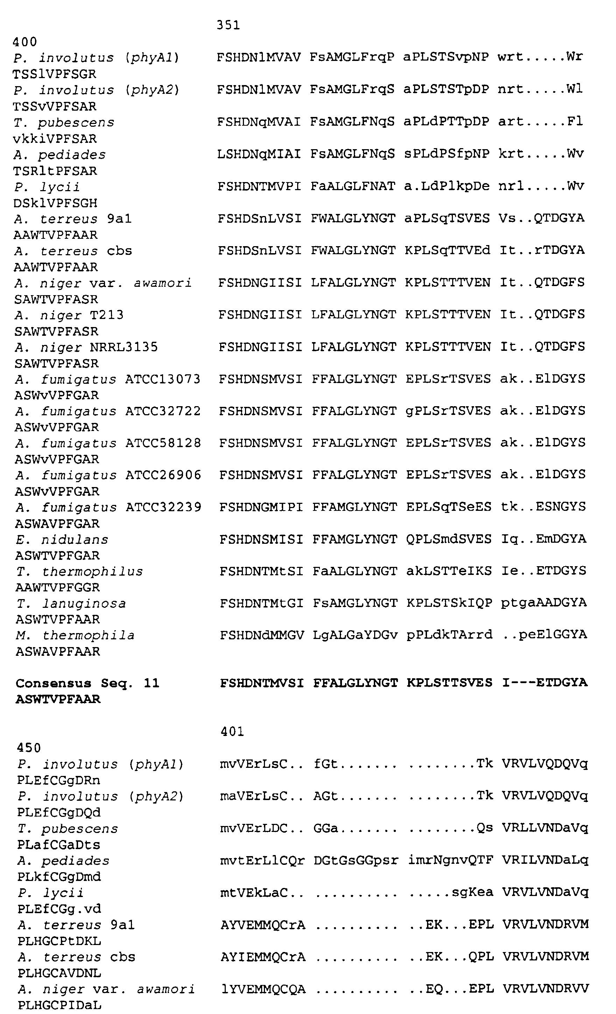

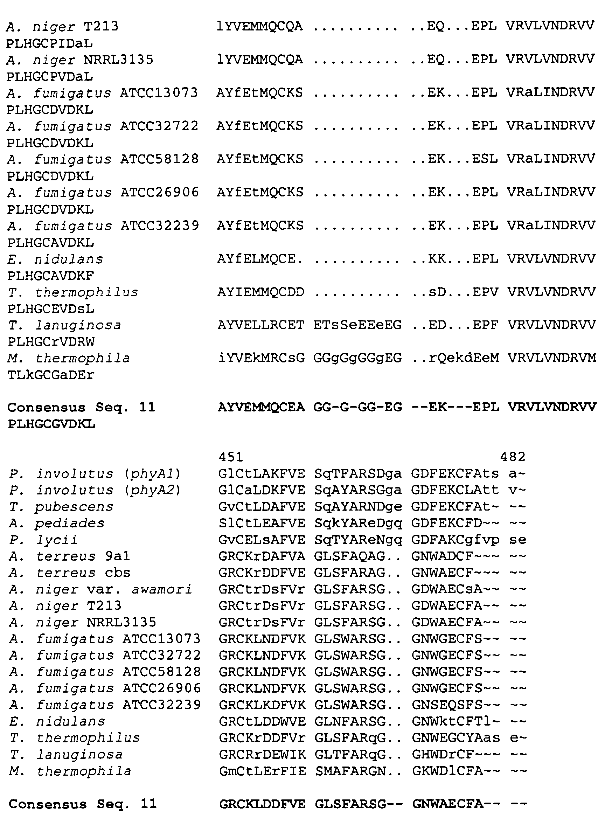

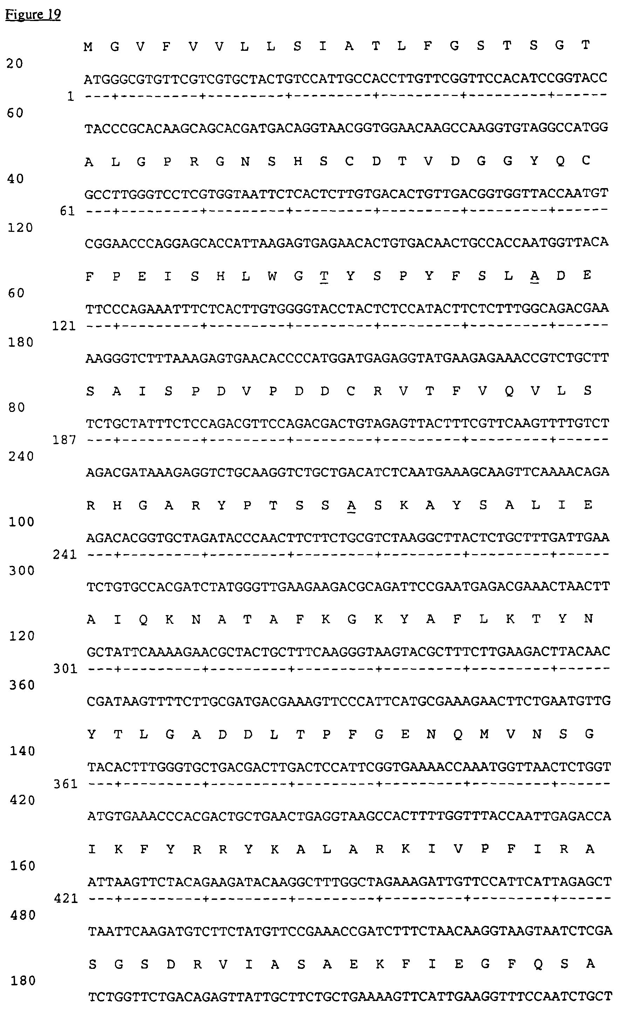

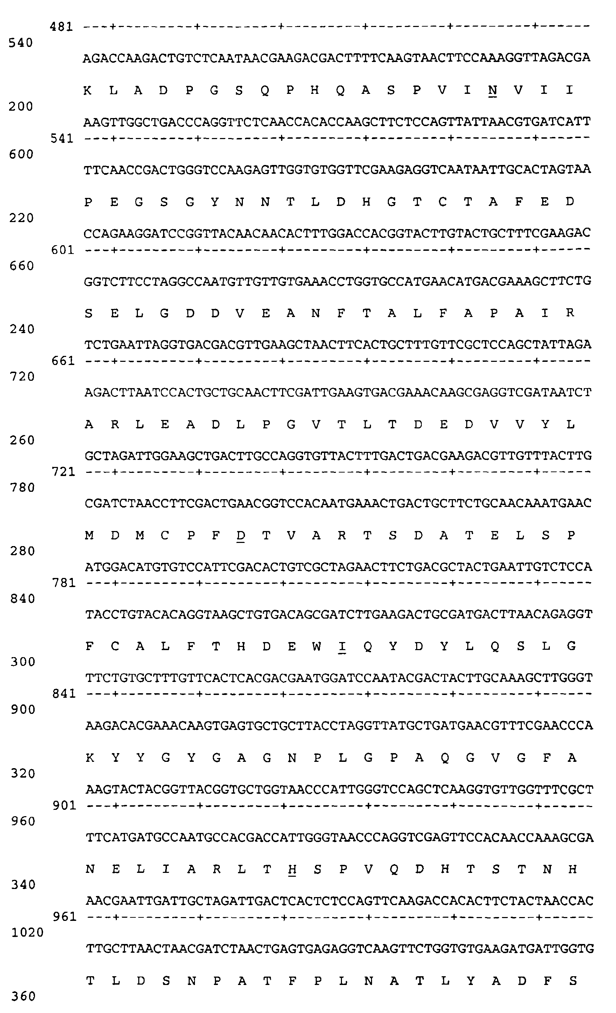

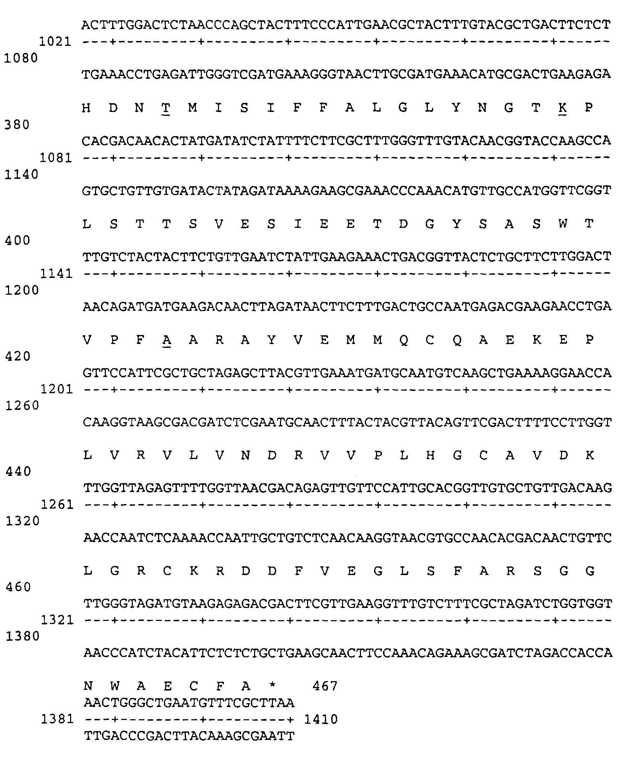

**Figure 19** DNA and amino acid sequence of consensus phytase-1-thermo[8]-Q50T-K91A. The amino acid sequence is written above the corresponding DNA sequence using the one-letter code. The replaced amino acid residues are underlined. The stop codon of the gene is marked by a star (*).

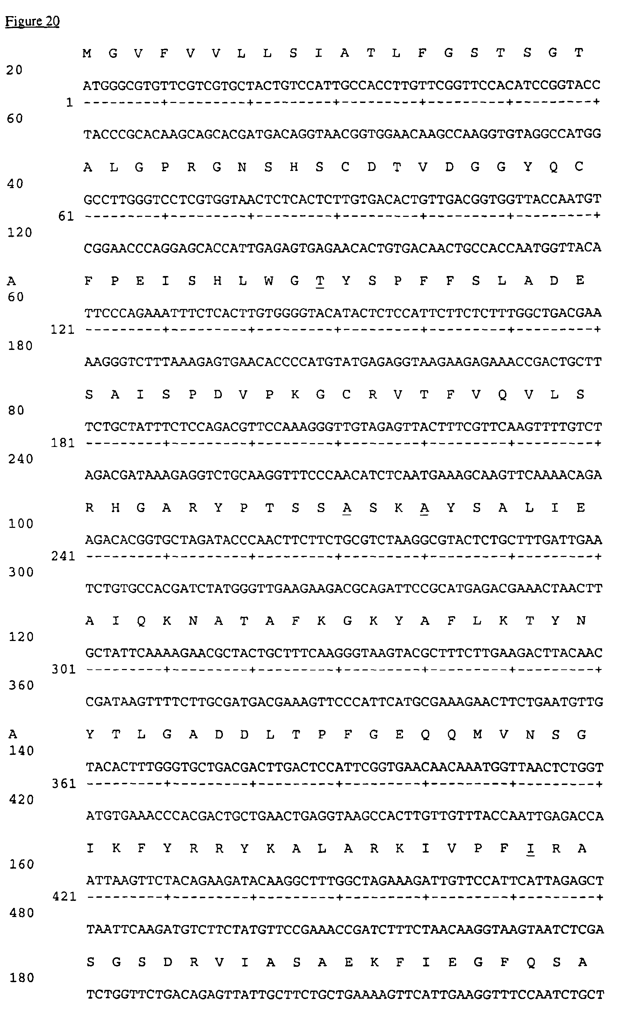

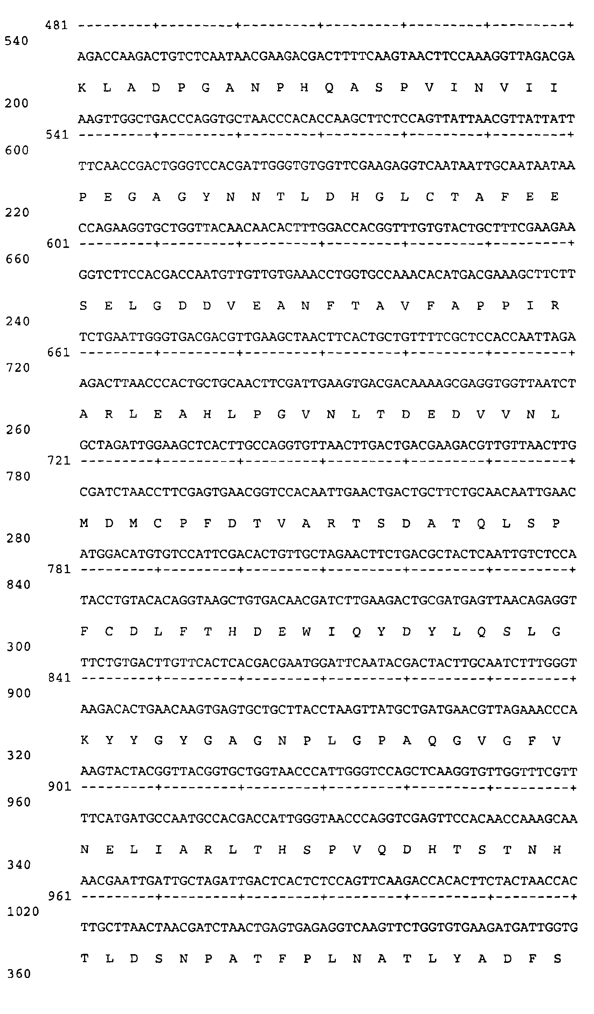

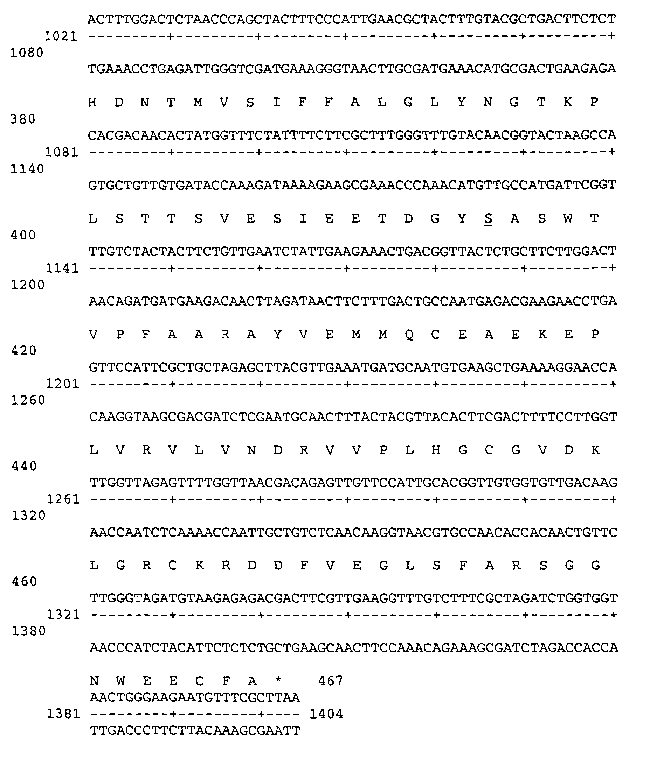

**Figure 20** DNA and amino acid sequence of consensus phytase-10-thermo[3]-Q50T-K91A. The amino acid sequence is written above the corresponding DNA sequence using the one-letter code. The replaced amino acid residues are underlined. The stop codon of the gene is marked by a star (*).

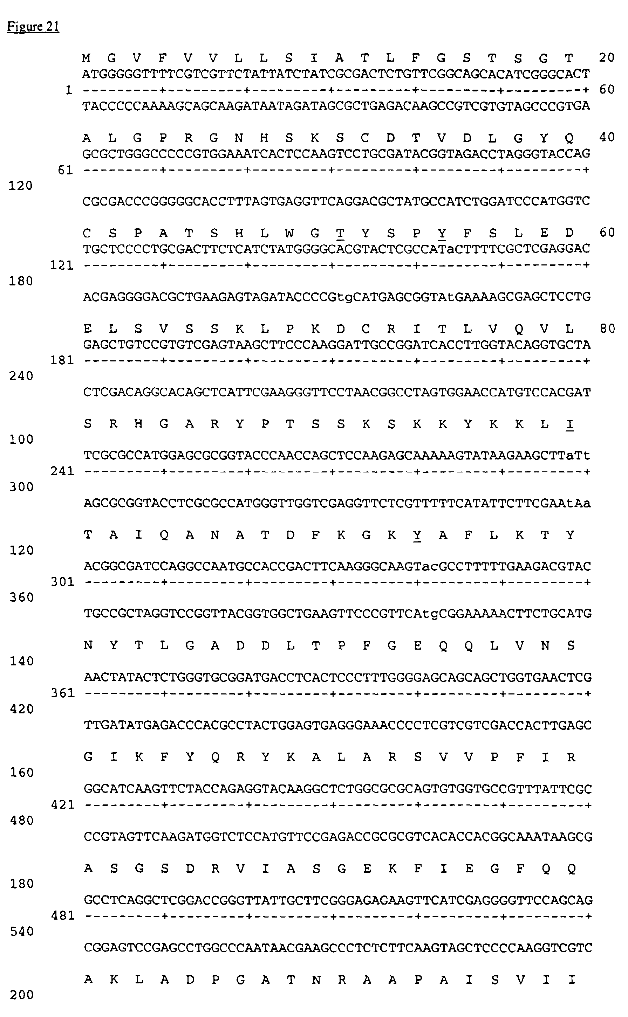

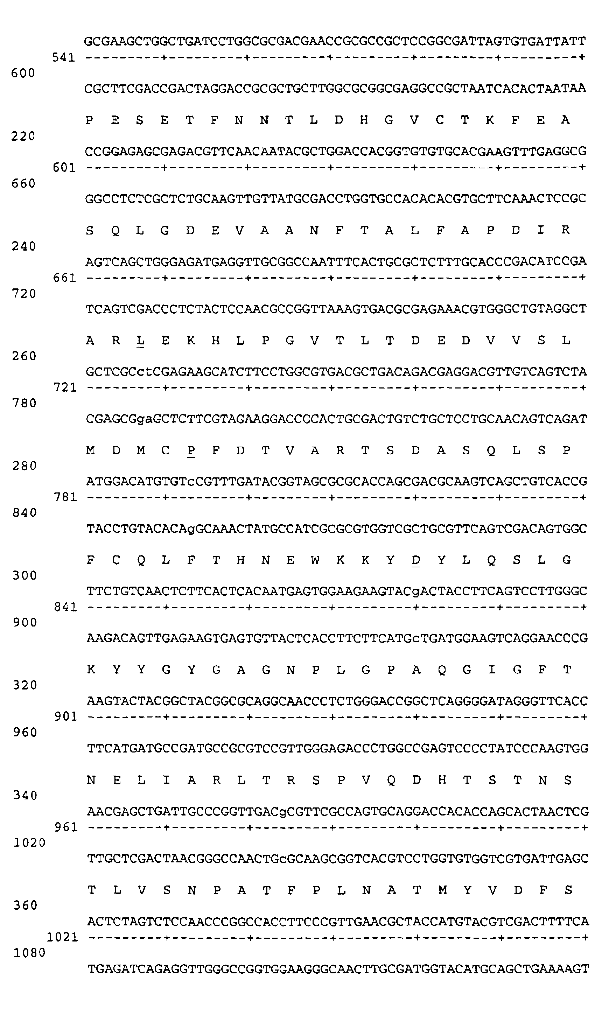

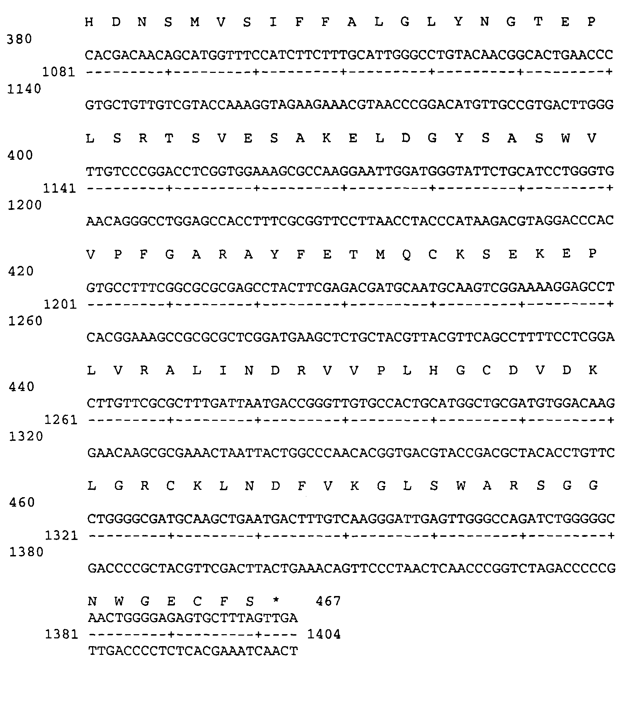

**Figure 21** DNA and amino acid sequence of *A. fumigatus* ATCC 13073 phytase a-mutant. The amino acid sequence is written above the corresponding DNA sequence using the one-letter code. The replaced amino acid residues are underlined. The stop codon of the gene is marked by a star (*).

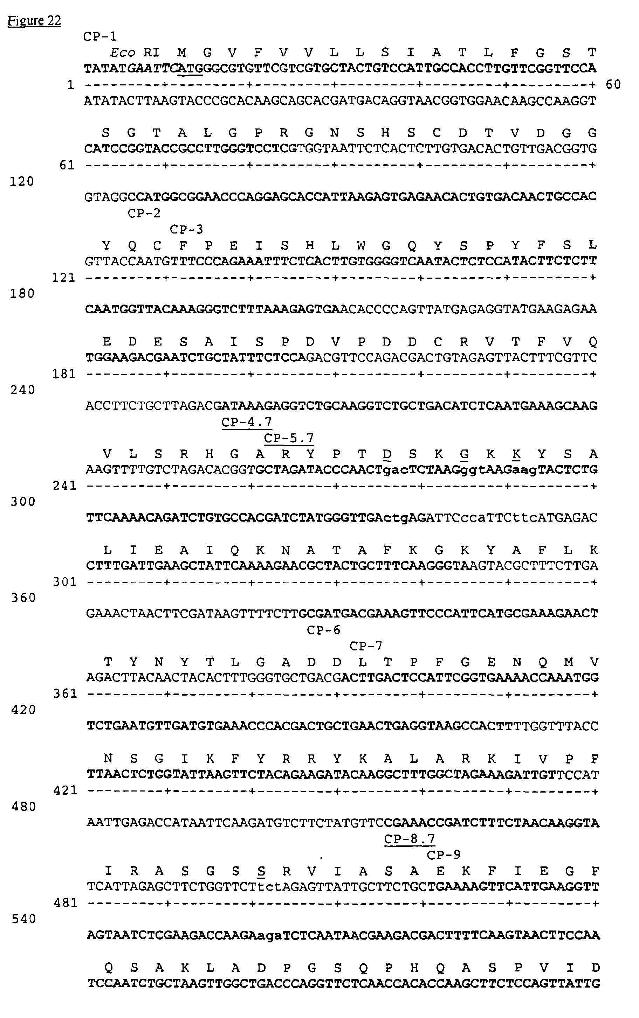

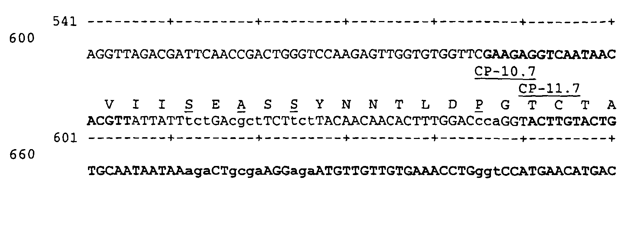

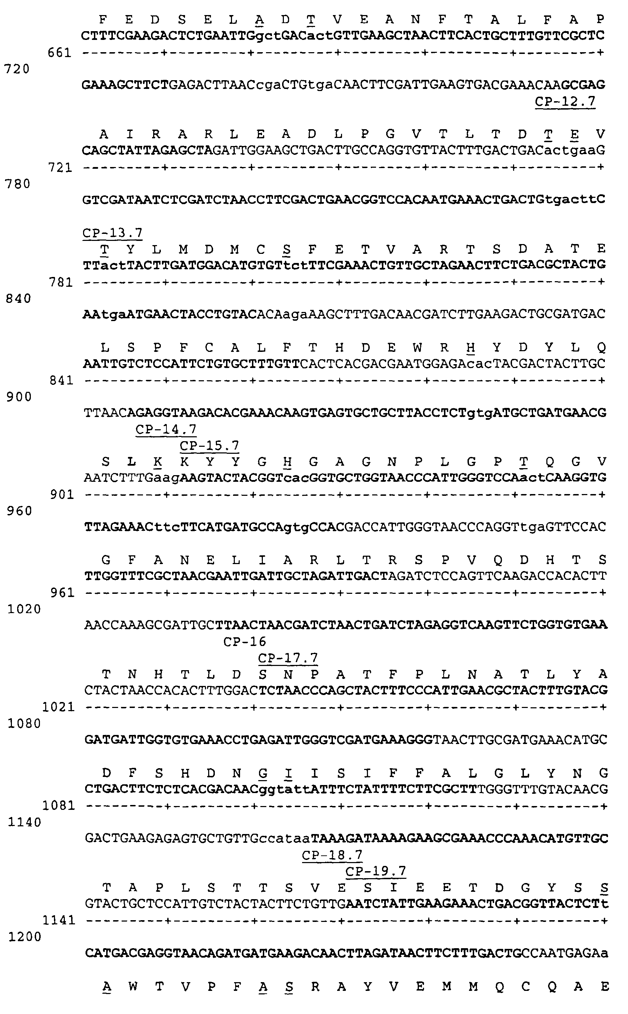

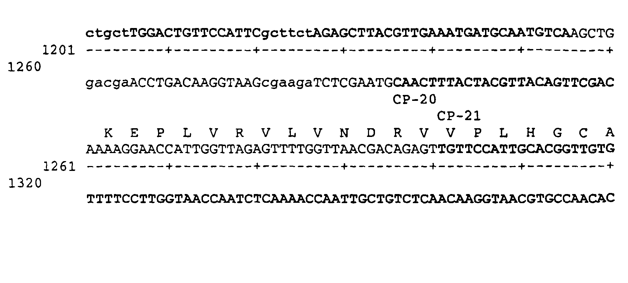

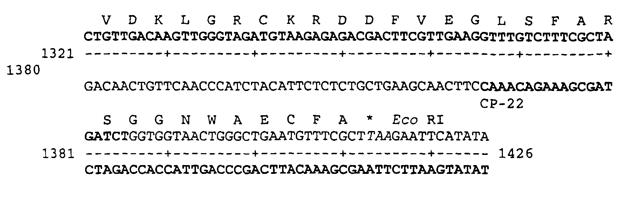

**Figure 22** DNA and amino acid sequence of consensus phytase-7. The amino acids are written above the corresponding DNA sequence using the one-letter code. The sequence of the oligonucleotides used to assemble the gene are in bold letters. Oligonucleotides and amino acids that were exchanged are underlined and their corresponding triplets are highlighted in small cases. The *fcp*7 gene was assembled from the following oligonucleotides: CP-1, CP-2, CP-3, CP-4.7, CP-5.7, CP-6, CP-7, CP-8.7, CP-9, CP-10.7, CP-11.7, CP-12.7, CP-13.7, CP-14.7, CP-15.7, CP-16, CP-17.7, CP-18.7, CP-19.7, CP-20, CP-21, CP-22. The newly synthesized oligonucleotides are additionally marked by number 7. The phytase contains the following 24 exchanges in comparison to the original consensus phytase: S89D, S92G, A94K, D164S, P201S, G203A, G205S, H212P, G224A, D226T, E255T, D256E, V258T, P265S, Q292H, G300K, Y305H, A314T, S364G, M365I, A397S, S398A, G404A, and A405S.

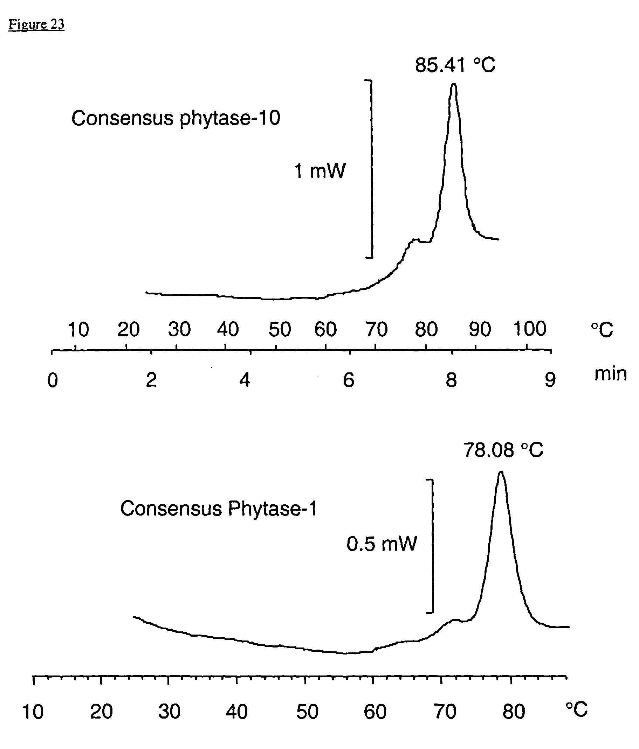

**Figure 23** Differential scanning calorimetry (DSC) of consensus phytase-1 and consensus phytase-10. The protein samples were concentrated to ca. 50-60 mg/ml and extensively dialyzed against 10 mM sodium acetate, pH 5.0. A constant heating rate of 10 °C/min was applied up to 95 °C. DSC of consensus phytase-10 (upper graph) yielded a melting temperature of 85.4 °C, which is 7.3 °C higher than the melting point of consensus phytase-1 (78.1 °C, lower graph).

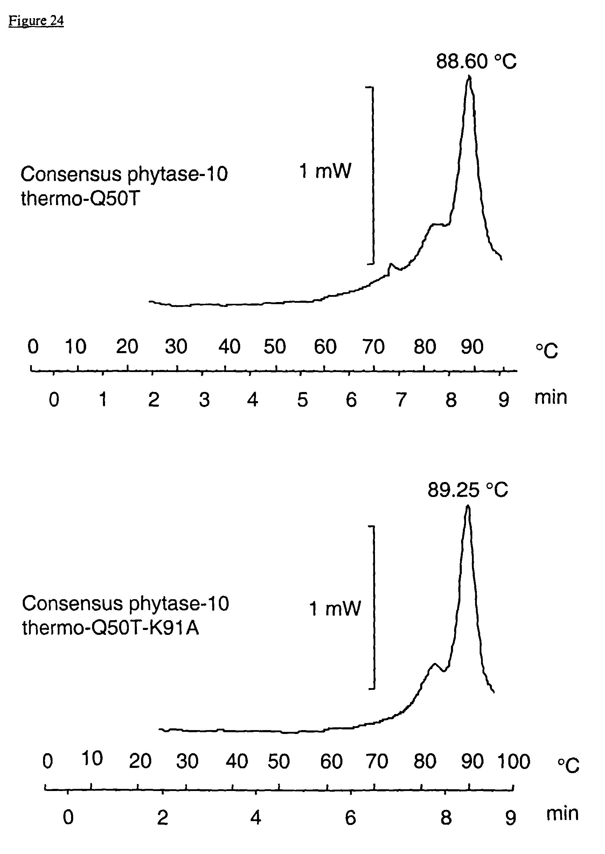

**Figure 24** Differential scanning calorimetry (DSC) of consensus phytase-10-thermo-Q50T and consensus phytase-10-thermo-Q50T-K91A. The protein samples were concentrated to ca. 50-60 mg/ml and extensively dialyzed against 10 mM sodium acetate, pH 5.0. A constant heating rate of 10 °C/min was applied up to 95 °C. DSC of consensus phytase-10-thermo-Q50T (upper graph) yielded a melting temperature of 88.6 °C, while the melting point of consensus phytase-10-thermo-Q50T-K91A was found at 89.3 °C.

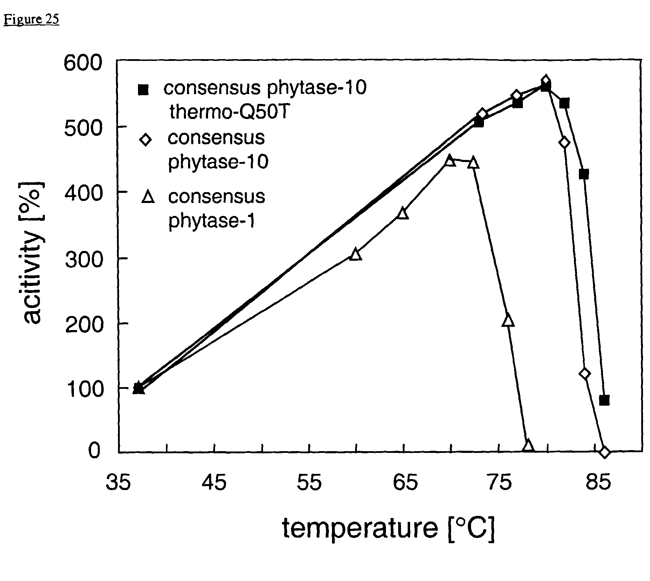

**Figure 25** Comparison of the temperature optimum between consensus phytase-1, consensus phytase-10 and consensus phytase-10-thermo-Q50T. For the determination of the temperature optimum, the phytase standard assay was performed at a series of temperatures between 37 and 86 °C. The diluted supernatant of transformed *S. cerevisiae* strains was used for the determination. The other components of the supernatant showed no influence on the determination of the temperature optimum: ∧, consensus phytase-1; 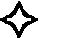 , consensus phytase-10; ■, consensus phytase 10-thermo-Q50T.

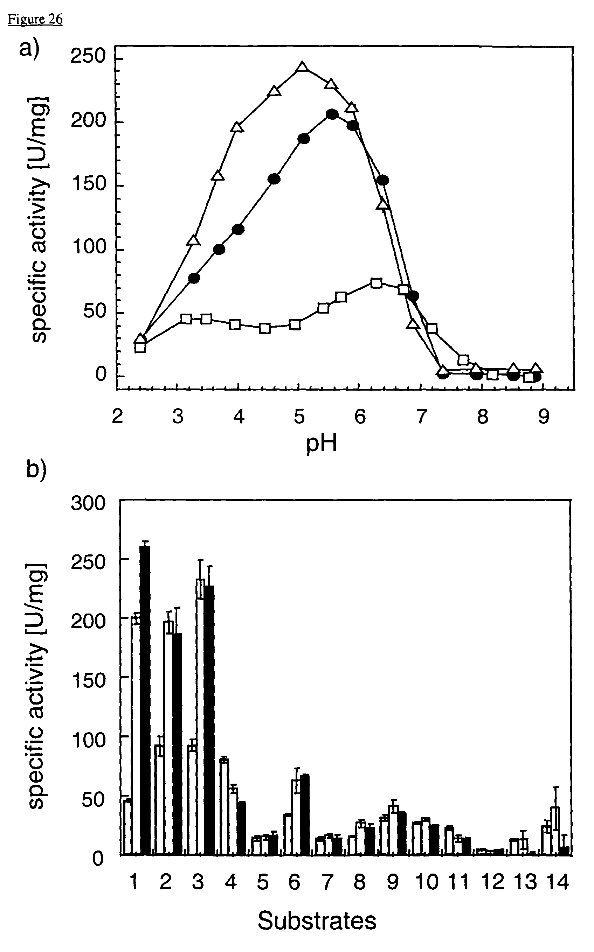

**Figure 26** pH-dependent activity profile and substrate specificity of consensus phytase-10 and its variants thermo-Q50T and thermo-Q50T-K91A. The phytase activity was determined using the standard assay in appropriate buffers (see Example 11) at different pH-values. Graph a) shows the pH-dependent activity profile of consensus phytase-10 (□), consensus phytase-10-thermo-Q50T (·), and consensus phytase-10-thermo-Q50T-K91A (∧). Graph b) shows the corresponding substrate specificity tested by replacement of phytate by the indicated compounds in the standard assay; open bars, consensus phytase-10 (grey bars, consensus phytase-10-thermo-Q50T; dark bars, consensus phytase-10-thermo-Q50T-K91A). The numbers correspond to the following compounds: 1, phytate; 2, *p*-nitrophenyl phosphate; 3, phenyl phosphate; 4, fructose-1,6-bisphosphate; 5, fructose-6-phosphate; 6, glucose-6-phosphate; 7, ribose-5-phosphate; 8, DL-glycerol-3-phosphate; 9, glycerol-2-phosphate; 10, 3-phosphoglycerate; 11, phosphoenolpyruvate; 12, AMP; 13, ADP; 14, ATP.

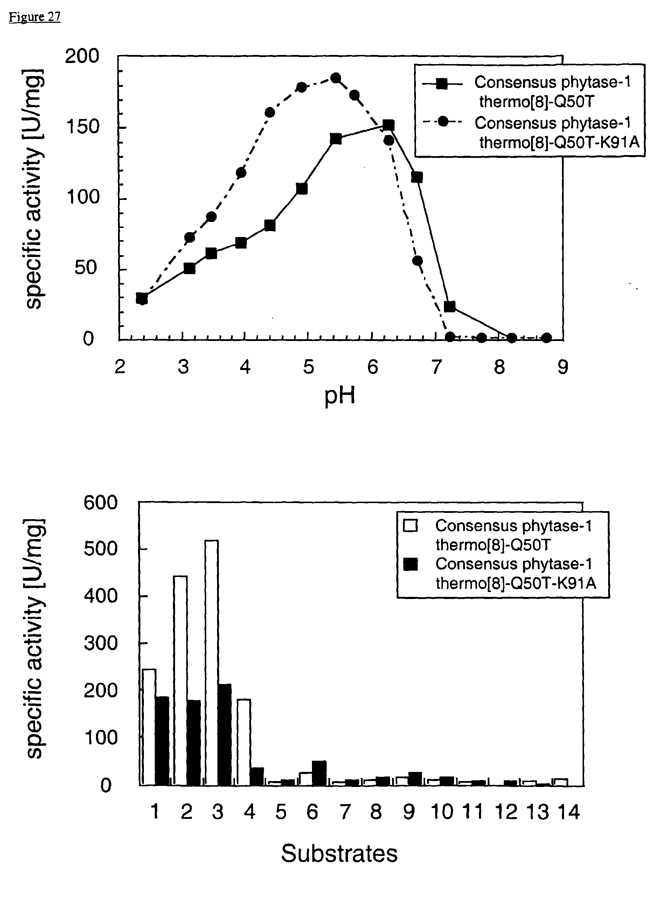

**Figure 27** pH-dependent activity profile and substrate specificity of consensus phytase-1-thermo[8]-Q50T and of consensus phytase-1-thermo[8]-Q50T-K91A. The phytase activity was determined using the standard assay in appropriate buffers (see Example 11) at different pH-values. Graph a) shows the pH-dependent activity profile of the Q50T- (■) and the Q50T-K91A-variant (·). Graph b) shows the corresponding substrate specificities tested by replacement of phytate by the indicated compounds in the standard assay (open bars, consensus phytase-1-thermo[8]-Q50T; filled bars, consensus phytase-1-thermo[8]-Q50T-K91A.). The substrates are listed in the legend of Figure 26.

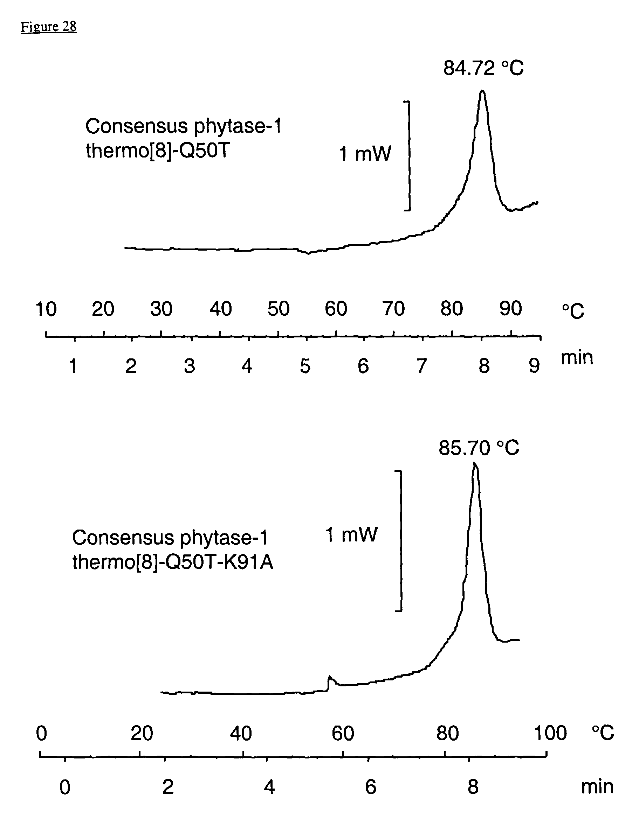

**Figure 28** Differential scanning calorimetry (DSC) of consensus phytase-1-thermo[8]-Q50T and consensus phytase-1-thermo[8]-Q50T-K91A. The protein samples were concentrated to ca. 50-60 mg/ml and extensively dialyzed against 10 mM sodium acetate, pH 5.0. A constant heating rate of 10 °C/min was applied up to 95 °C. DSC of consensus phytase-1-thermo[8]-Q50T (upper graph) showed a melting temperature of 84.7 °C, while the melting point of consensus phytase-1-thermo[8]-Q50T-K91A was found at 85.7 °C.

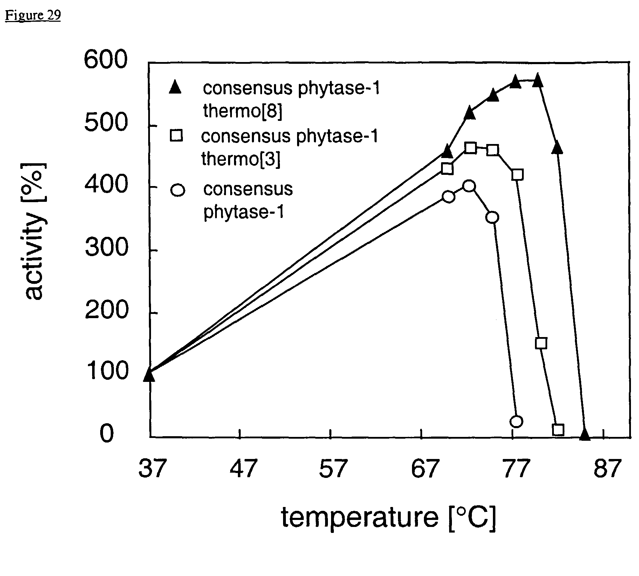

**Figure 29** Comparison of the temperature optimum between consensus phytase-1, consensus phytase-1-thermo[3] and consensus phytase-1-thermo[8]. For the determination of the temperature optimum, the phytase standard assay was performed at a series of temperatures between 37 and 86 °C. Purified protein from the supernatant of transformed *S. cerevisiae* strains was used for the determination. ○ , consensus phytase-1; □, consensus phytase-1-thermo[3]; ▲, consensus phytase 1-thermo[8].

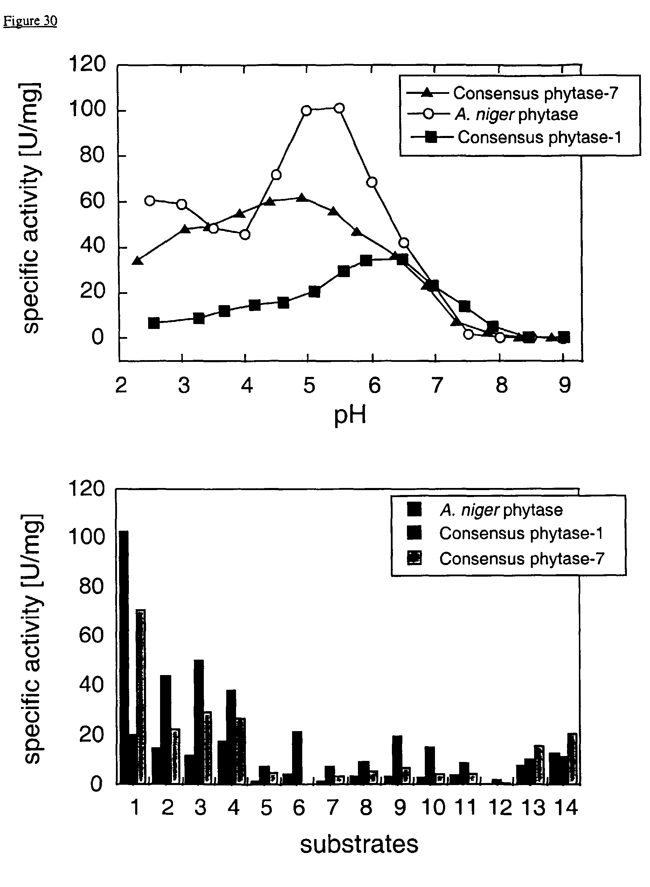

**Figure 30** Comparison of the pH-dependent activity profile and substrate specificity of consensus phytase-1, consensus phytase-7, and of the phytase from *A. niger* NRRL 3135. The phytase activity was determined using the standard assay in appropriate buffers (see Example 11) at different pH-values. Graph a) shows the pH-dependent activity profile of consensus phytase-1 (■), the phytase from *A. niger* NRRL 3135 (○ ), and of consensus phytase-7 (▲). Graph b) shows the corresponding substrate specificity tested by replacement of phytate by the indicated compounds in the standard assay (black bars, *A. niger* NRRL 3135 phytase; grey bars, consensus phytase-1, dashed bars, consensus phytase-7). The substrates are listed in the legend of Figure 26.

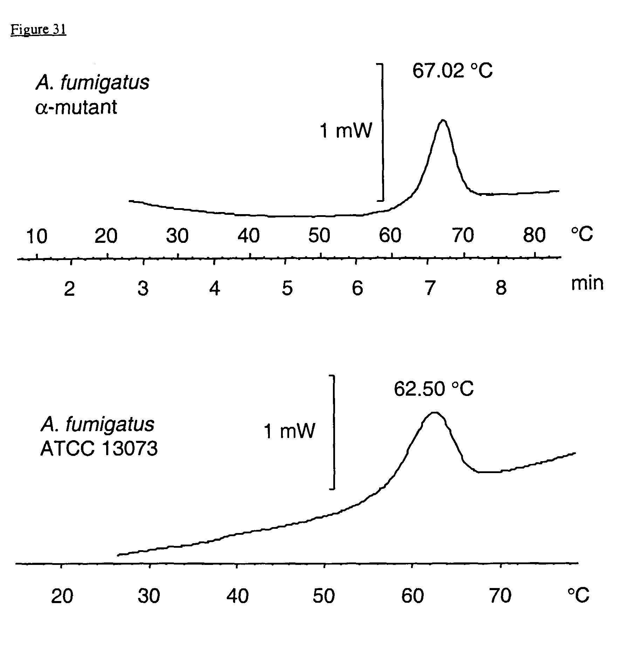

**Figure 31** Differential scanning calorimetry (DSC) of the phytase from A. fumigatus ATCC 13073 and of its stabilized α-mutant, which contains the following amino acid exchanges F55Y, V100I, F114Y, A243L, S265P, N294D. The protein samples were concentrated to ca. 50-60 mg/ml and extensively dialyzed against 10 mM sodium acetate, pH 5.0. A constant heating rate of 10 °C/min was applied up to 95 °C. DSC of consensus *A. fumigatus* 13073 phytase (upper graph) revealed a melting temperature of 62.5 °C, while the melting point of the α-mutant was found at 67.0 °C

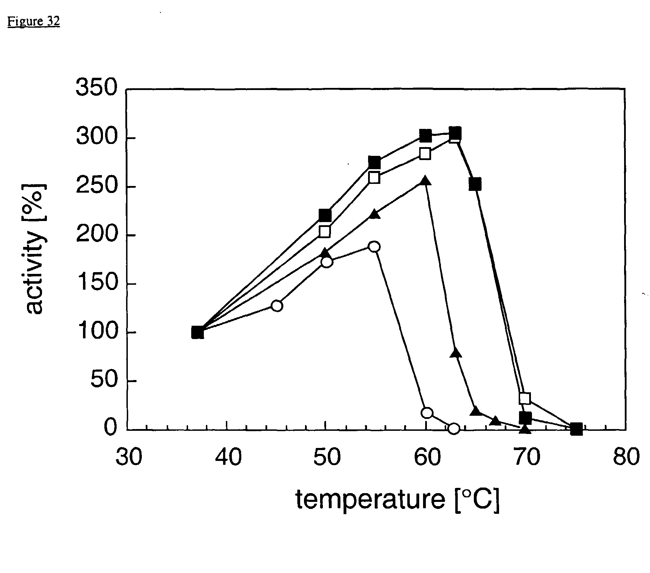

**Figure 32** Comparison of the temperature optimum of *A. fumigatus* 13073 wild-type, its *A. fumigatus* α-mutant, and a further stabilized α-mutant (E59A-S126N-R329H-S364T-G404A). For the determination of the temperature optimum, the phytase standard assay was performed at a series of temperatures between 37 and 75 °C. The diluted supernatant of transformed *S. cerevisiae* strains was used for the determination. The other components of the supernatant showed no influence on the determination of the temperature optimum. ○ , *A. fumigatus* ATCC 13073 phytase; ▲, *A. fumigatus* ATCC 13073 α-mutant; □, *A. fumigatus* ATCC 13073 alpha-mutant-(E59A-S126N-R329H-S364T-G404A)-Q27T; ■ , *A. fumigatus* ATCC 13073 α-mutant-(E59A-S126N-R329H-S364T-G404A)-Q27T-K68A. Q27T and K68A corresponds to consensus phytase-1 Q50T and K91A, respectively.

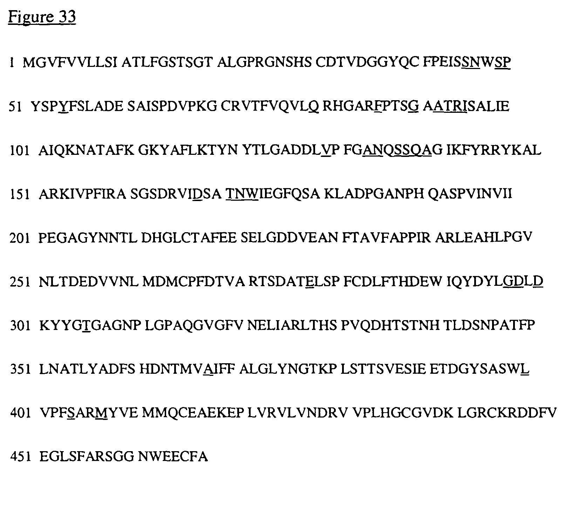

**Figure 33** Amino acid sequence of consensus phytase 12 (consphy12) which contains a number of active site residues transferred from the "basidio" consensus sequence to consensus phytase-10-thermo-Q50T-K91A.

### Example 1

### a) Materials

Phytic acid (dodecasodium salt) and polyethylene glycols, polyols, sodium dicarboxylates, sodium periodate, adipic acid dihydrazide and other additives were purchased from commercial suppliers. All other chemicals were at least of analytical grade. Five-ml HiTrap desalting columns were obtained from Pharmacia. SDS-PAGE gels (4-12% NuPAGE Bis-Tris Pre-Cast) and buffers were delivered by NOVEX.

### b) Expression and purification of phytases

*A. fumigatus*, *A. terreus* CBS phytase and consensus phytase were overexpressed in *Hansenula polymorpha*. *A. niger* and *A. nidulans* phytase were overexpressed in *A. niger* Cloning, purification and characterization of these phytases was previously described by Pasamontes *et al* [Appl. Environ. Microbiol. (1997), 63, p. 1696-1700]. Construction, cloning and purification of consensus phytase were performed according to European Patent Application Publication No. 897 985. Non-formulated consensus phytase had an increased thermal stability of up to 70 °C and, due to an amino acid exchange (L at position 50 for Q), a three-fold higher specific activity compared to *A. fumigatus* phytase.

### c) Phytase activity assay

For the determination of thermostability the enzymatic activity measurements with phytic acid were done at different temperatures by diluting the purified enzymes to 0.05 U/ml (activities measured at 37 °C) in 0.2 M sodium acetate, pH 5.0 (+/- additives in % w/w). An aliquot of the protein solution (250 µl) was preincubated for 5 mm at the desired temperature, followed by addition of an equal volume of a solution containing 1% phytic acid in 0.2 M sodium acetate, pH 5.0 (preincubated as a 10 ml aliquot for 10 mm at the same temperature). After incubation of the sample for 15 mm at the desired temperature (e.g. at 60 or 65 °C for the screening of additive effects), the reaction was stopped by addition of
0.5 ml 15% trichloroacetic acid. Determination of liberated inorganic phosphate was performed by standard methods.

### d) Evaluation of thermostabilizing additives

In general, the polyols have been dissolved at a concentration of 25 or 50% (w/w) in 0.2 M sodium acetate, pH 5.0. PEGs have been dissolved at a concentration of 50% with the exception of PEGs with a molecular weight of 4000, 8000 and 10000 which were used at a concentration of 25%. For the screening of PEGs and other polyols, the preincubation and reaction temperature was chosen as 60 °C for *A. nidulans* and *A. terreus* CBS phytase, 65 °C for *A. fumigatus a*nd *A. niger* phytase and 75 °C for consensus phytase.

Disodium malonate, succinate and glutarate were dissolved at concentrations of 5, 10 and 25% and phytase activity was measured after preincubation of enzyme plus additive and substrate (see above) at the following temperatures: 37, 45, 50, 55, 60, 65, 70, 75, 80, and 85 °C. In the same way, the temperature dependence of the activity of different phytases in the presence of 25% xylitol and ribitol was tested. It should be noted that the concentration of the additives was reduced by half after substrate addition.

### e) Crosslinking of carbohydrate chains

Crosslinking of phytase carbohydrate chains was performed as described for invertase by Cesi et al. [Studies in Organic Chemistry 47: Stability and Stabilization of Enzymes, Proceedings of an International Symposium held in Maastricht, The Netherlands, 1992, Elsevier Science Publications B.V., Amsterdam, The Netherlands]. Phytase samples (5 mg protein/ml) were incubated for 2 h at 30 °C in the presence of different concentrations (0, 5, 10, 20, 30, 40 and 50 mM) of sodium periodate in 0.2 M sodium acetate, pH 5.0, and stored at 4 °C overnight. Each sample was desalted on a 5-ml HiTrap desalting column (Pharmacia) connected to an ÄktaExplorer system (Pharmacia), using 0.2 M sodium acetate, pH 5.0, as elution buffer. Crosslinking was achieved by adding 100 µl of 0.5 M adipic acid dihydrazide dissolved in 0.2 M sodium acetate, pH 5.0, to 900 µl of the desalted oxidation products. Phytase activity measurements and gel electrophoresis of the samples were performed after both the oxidation and crosslinking steps.

### f) High-shear granulation of thermostabilized phytases

100-250 ml of a phytase solution (in total 2500 - 5000 units of crosslinked or non-crosslinked phytase) were added to 1 kg of a dry mixture of 5-10% calcium lignosulfonate (Borregard, Norway), 5-20% silica (Sipernat 50S, Degussa, Germany), 0-20% thermostabilizing agent and gipsum. During the high-shear granulation process water was added until granulates with desired properties were formed. The granulates were dried in a fluid bed dryer for 15 mm at 45 °C and subsequently fat coated with natural palm fat (Palm 46, Florin, Basel, Switzerland).

### g) Pelleting stability of thermostabilized dry and liquid phytase formulations

Thermostabilized dry or liquid formulations of phytases (as mentioned above) were mixed with feed and subsequently pelleted under steam conditioning at 85 °C. The pelleting stability of phytase was determined by measurement of the phytase activity both in the mash before pelleting and in the delivered pellets.

### Example 2

Investigations of the temperature dependence of activity of different fungal phytases as described in Example 1 revealed activity maxima at the following temperatures: 55 °C for *A. fumigatus phytase* and *A. niger* phytase, 45 °C for *A. terrreus* CBS phytase and *A. nidulans* phytase, and 65 °C for consensus phytase. A temperature 10-15 °C above the determined temperature maximum was chosen as screening point for studying the effects of polyols, polyethylene glycols, dicarboxylates, carboxymethylcellulose and sodium alginate on the thermostability of phytases.

### a) Addition of polyethylene glycols of different molecular weights

The addition of 50% or 25% (25% and 12.5% final concentration during the reaction period) polyethylene glycol enhanced the specific activity of *A. fumigatus* phytase measured at 65 °C in a molecular weight-dependent fashion, with a maximum being observed with PEG 1450 (specific activity 80 U*(mg protein)⁻¹) and considerable activities also with PEG 1000 (50 U*(mg protein)⁻¹) and PEG 3350 (42 U*(mg protein)⁻¹). The results of this experiment are summarized in Figure 2.

PEGs with molecular weights of 600, 1000, 1450, 3350 and 4000 Da showed similar effects on the other phytases tested. The results of this experiment are shown in Figure 3.

### b) Addition of polyols

The polyols ribitol, xylitol (C₅ sugars) and sorbitol (C₆ sugar) in concentrations of 25 and 50% significantly improved the thermostability of *A. fumigatus* phytase. This is shown in Figure 4.

Erythritol, mannitol, mannoheptulose and mannoheptose were not soluble in 0.2 M sodium acetate, pH 5.0, at a concentration of 50% (w/w) and, therefore, only the 25% values are shown. The specific activities measured at 65 °C were 11, 21 and 11 U*(mg protein)⁻¹ in the presence of 25% ribitol, xylitol and sorbitol, and 51, 90 and 74 U*(mg protein)⁻¹ in the presence of 50% solutions of ribitol, xylitol and sorbitol, respectively.

Polyols containing more than 6 or less than 5 carbon atoms such as glycerol (C₃ sugar), erythritol (C₄ sugar), mannoheptose and mannoheptulose (C₇ sugars) showed an inferior effect on the thermostabilization of *A. fumigatus* phytase.

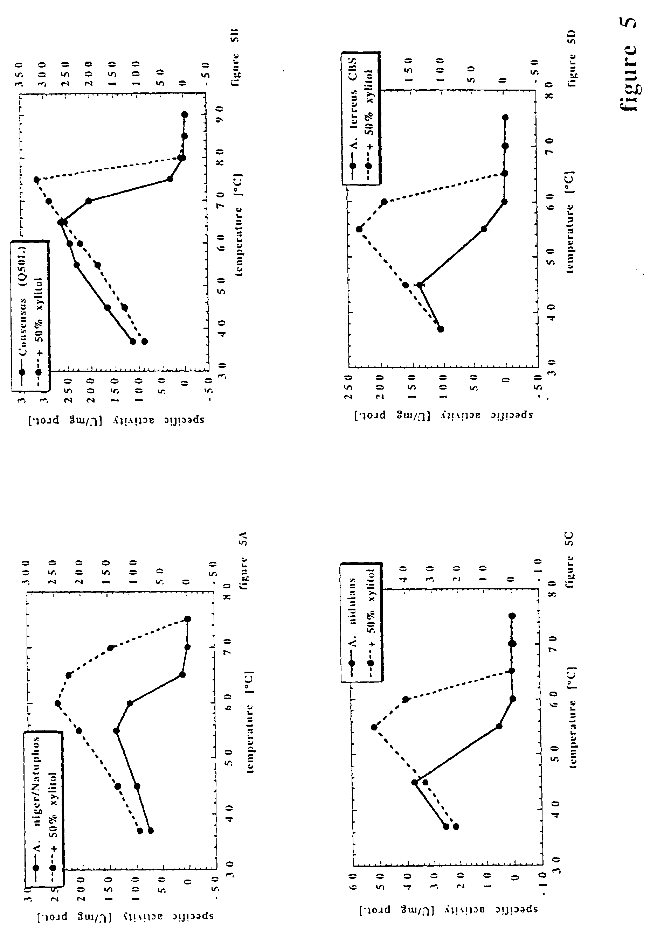

Xylitol at a concentration of 50% also increased the temperature optimum of *A. nidulans*, *A. terreus* CBS, *A. niger* and consensus phytase by 10-15 °C. The results are shown in Figure 5.

### c) Addition of dicarboxylic acids

Malonate, succinate and glutarate at a concentration of 25% (12.5% final concentration in the activity assay) resulted in a significant increase in *A. fumigatus* phytase thermostability with considerable activity still being detected at 70 °C for malonate and at 65 °C for succinate and glutarate. The results are shown in Figure 6.

In addition, dicarboxylates stimulated *A. fumigatus* phytase activity measured at 37 °C, with an approximately 4-fold increase in phytase activity in the case of malonate, a 2-fold increase for succinate and minor effects for glutarate. Investigation of different concentrations (5, 10 and 25%) of malonate showed that thermostabilization of *A. fumigatus* phytase is concentration-dependent whereas stimulation of enzymatic activity, at least in this concentration range, is not. This is shown in Figure 7.

In contrast to these findings, different concentrations of sodium acetate (5, 10 and 25%), a monocarboxylic acid, caused a 2-fold increase in specific activity of *A. fumigatus* phrase at 37 °C, but had only minor effects on the thermal stability of the protein. This can be seen in Figure 8.

Disodium malonate and succinate generally increased the thermostability of *A. nidulans*, *A. terreus* CBS, *A. niger* and consensus phytase by 5-15 °C. On the other hand, stimulation of phytase activity was only observed for *A. nidulans* and *A. fumigatus* phytase, both having a rather low specific activity, but not for *A. terreus* CBS, *A. niger* and consensus phytase. This is demonstrated in Figures 9 and 10.

### d) Effect of crosslinking

In a preliminary experiment, *A. fumigatus* phytase monomers were crosslinked by incubation with glutaraldehyde. The resulting thermostabilization measured at 60 °C reached a maximum after 1 hr reaction time but led to activity loss (measured at 37 °C). In a further set of experiments, *A. fumigatus* phytase monomers were crosslinked via their carbohydrate chains. This type of crosslinking was achieved with only minor loss of specific activity (< 10%) and resulted in the formation of oligomeric forms at sodium periodate concentrations above 15 mM. This can be seen from Figure 11.

The extent of thermostabilization was dependent on periodate concentration and reached a maximum at 50 mM where high specific activities were observed up to 75 °C (see Figure 12). A pronounced effect of phytase oligomerization on thermostability was also observed for consensus phytase crosslinked via its carbohydrate chains. This can be seen from Figure 12.

In the present work, we focused our efforts on the thermostabilization effects of low-Mᵣ additives - which are highly recommended for stabilization of industrial enzymes - and of chemical modification - even though this latter approach is commonly regarded as less attractive for technical and economical reasons.

We have found thermostabilization by C₅ sugars for a range of different phytases expressed in filamentous fungi (*A. niger*) or yeasts (*Hansenula polymorpha*). The increase in thermostability varied to some extent between the different phytases, but was around 10 °C. The effect of PEGs was molecular weight-dependent. The optimal thermostabilization of all phytases was obtained with PEGs having a molecular weight between 1000 and 3350 Da.

Sodium acetate, a monocarboxylic acid and main component of the standard phytase activity assay, caused a concentration-dependent increase in *A. fumigatus* phytase activity, but had no effect on phytase thermostability. Therefore, carboxylate groups might be responsible for the activity modulation whereas bifunctional dicarboxylates possibly stabilize phytases by ionic interactions.

### Example 3

### Design of the amino acid sequence of consensus phytase-1

### Alignment of the amino acid sequences

The alignment was calculated using the program PILEUP from the Sequence Analysis Package Release 9.0 (Devereux *et al.*, 1984) with the standard parameter (gap creation penalty 12, gap extension penalty 4). The location of the gaps was refined using a text editor. Table 1 shows the sequences (see Figure 13) without the signal sequence that were used for the performance of the alignment starting with the amino acid (aa) as mentioned in Table 1.

**Table 1**

| Origin and vote weight of the phytase amino acid sequences used for the design of consensus phytase-1 |
|---|
| -*phyA* from *Aspergillus terreus* 9A-1, aa 27, vote weight 0.5 (Mitchell *et al*., 1997) |
| -*phyA* from *Aspergillus terreus* cbs116.46, aa 27, vote weight 0.5 (van Loon *et al*., 1998) |
| - *phyA* from *Aspergillus niger* var. *awamori*, aa 27, vote weight 0.33 (Piddington *et al.*, 1993) |
| -*phyA* from *Aspergillus niger* T213, aa 27, vote weight 0.33 |
| - *phyA* from *Aspergillus nige*r strain NRRL3135, aa 27, vote weight 0.33 (van Hartingsveldt *et al*., 1993) |
| - *phyA* from *Aspergillus fumigatus* ATCC 13073, aa 26, vote weight 0.2 (Pasamontes *et al.,* 1997) |
| -*phyA* from *Aspergillus fumigatus* ATCC 32722, aa 26, vote weight 0.2 (van Loon *et al.*, 1998) |
| - *phyA* from *Aspergillus fumigatus* ATCC 58128, aa 26, vote weight 0.2 (van Loon *et al*., 1998) |
| - *phyA* from *Aspergillus fumigatus* ATCC 26906, aa 26, vote weight 0.2 (van Loon *et al.*, 1998) |
| - *phyA* from *Aspergillus fumigatus* ATCC 32239, aa 30, vote weight 0.2 (van Loon *et al.*, 1998) |
| -*phyA* from *Emericella nidulans* , aa 25, vote weight 1.0, Pasamontes *et al.*, 1997a) |
| - *phyA* from *Talaromyces rhermophilus* ATCC 20186, aa 24, vote weight 1.0 (Pasamontes *et al*., 1997a) |
| -*phyA* from *Myceliophthora thermophila,* aa 19, vote weight 1.0 (Mitchell *et al*., 1997) |

### Calculation of the amino acid sequence of consensus phytase-1

Using the refined alignment as input, the consensus sequence was calculated by the program PRETTY from the Sequence Analysis Package Release 9.0 (Devereux *et al*., 1984). PRETTY prints sequences with their columns aligned and can display a consensus sequence for an alignment. A vote weight that pays regard to the similarity between the amino acid sequences of the phytases aligned was assigned to all sequences. The vote weight was set such as the combined impact of all phytases from one sequence subgroup (same species, but from different strains), e. g. the amino acid sequences of all phytases from *A. fumigatus*, on the election was set one, that means that each sequence contributes with a value of 1 divided by the number of strain sequences (see Table 1). By this means, it was possible to prevent that very similar amino acid sequences, e. g. of the phytases from different *A. fumigatus* strains, dominate the calculated consensus sequence.

The program PRETTY was started with the following parameters: The plurality defining the number of votes below which there is no consensus was set on 2.0. The threshold, which determines the scoring matrix value below which an amino acid residue may not vote for a coalition of residues, was set on 2. PRETTY used the PrettyPep.Cmp consensus scoring matrix for peptides.

Ten positions of the alignment (position 46, 66, 82, 138, 162, 236, 276, 279, 280, 308; Figure 13), for which the program was not able to determine a consensus residue, were filled by hand according to the following rules: if a most frequent residue existed, this residue was chosen (138, 236, 280); if a prevalent group of similar or phylogenetically equivalent residues occurred, the most frequent or, if not available, one residues of this group was selected (46, 66, 82, 162, 276, 308). If there was either a prevalent residue nor a prevalent group, one of the occurring residues was chosen according to common assumption on their influence on the protein stability (279). Eight other positions (132, 170, 204, 211, 275, 317, 384, 447; Figure 13) were not filled with the amino acid residue selected by the program but normally with amino acids that occur with the same frequency as the residues that were chosen by the program. In most cases, the slight underrating of the three *A. niger* sequences (sum of the vote weights: 0.99) was eliminated by this corrections.

### Conversion of the consensus phytase-1 amino acid sequence to a DNA sequence

The first 26 amino acid residues of *A. terreus* cbs116.46 phrase were used as signal peptide and, therefore, fused to the N-terminus of all consensus phrases. For this stretch, we used a special method to calculate the corresponding DNA sequence. Purvis et al (1987) proposed that the incorporation of rare codons in a gene has an influence on the folding efficiency of the protein. Therefore, at least the distribution of rare codons in the signal sequence of *A. terreus* cbs116.46, which was used for the consensus phrase and which is very important for secretion of the protein, but converted into the *S. cerevisiae* codon usage, was transferred into the new signal sequence generated for expression in *S. cerevisiae*. For the remaining parts of the protein, we used the codon frequency table of highly expressed *S. cerevisiae* genes, obtained from the GCG program package, to translate the calculated amino acid sequence into a DNA sequence.

The resulting sequence of the *fcp* gene is shown in Figure 14.

### Construction and cloning of the consensus phytase-1 gene

The calculated DNA sequence of consensus phytase-1 (*fcp*) was divided into oligonucleotides of 85 bp, alternately using the sequence of the sense and the anti-sense strand. Every oligonucleotide overlaps 20 bp with its previous and its following oligonucleotide of the opposite strand. The location of all primers, purchased by Microsynth, Balgach (Switzerland) and obtained in a PAGE-purified form, is indicated in Figure 14.

### PCR-Reactions

In three PCR reactions, the synthesized oligonucleotides were composed to the entire gene. For the PCR, the High Fidelity Kit from Boehringer Mannheim (Boehringer Mannheim, Mannheim, Germany) and the thermo cycler The Protokol™ from AMS Biotechnology (Europe) Ltd. (Lugano, Switzerland) was used.

Oligonucleotide CP-1 to CP-10 (Mix 1, Figure 14) were mixed to a concentration of 0.2 pMol/µl of each oligonucleotide. A second oligonucleotide mixture (Mix 2) was prepared with CP-9 to CP-22 (0.2 pMol/µl of each oligonucleotide). Additionally, four short primers were used in the PCR reactions: 
CP-a: Eco RI
5'-TATATGAATTCATGGGCGTGTTCGTC-3'
CP-b:
5'-TGAAAAGTTCATTGAAGGTTTC-3'
CP-c:
5'-TCTTCGAAAGCAGTACAAGTAC-3'
CP-e: Eco RI
5'-TATATGAATTCTTAAGCGAAAC-3'
PCR reaction α: 10 µl Mix 1 (2.0 pmol of each oligonucleotide)
2 µl nucleotides (10 mM each nucleotide)
2 µl primer CP-a (10 pmol/µl)
2 µl primer CP-c (10 pmol/µl)
10,0 µl PCR buffer
0.75 µl polymerase mixture
73.25 µl H₂O

PCR reaction *b*: 10 µl Mix 2 (2.0 pmol of each oligonucleotide)
2 µl nucleotides (10 mM each nucleotide)
2 µl primer CP-b (10 pmol/µl)
2 µl primer CP-e (10 pmol/µl)
10,0 µl PCR buffer
0.75 µl polymerase mixture (2.6 U)
73.25 µl H₂O

Reaction conditions for PCR reaction *a* and *b*:

| | |
|---|---|
| step 1 | 2 min - 45°C |
| step 2 | 30 sec - 72°C |
| step 3 | 30 sec - 94°C |
| step 4 | 30 sec - 52°C |
| step 5 | 1 min - 72°C |

Step 3 to 5 were repeated 40-times.

The PCR products (670 and 905 bp) were purified by an agarose gel electrophoresis (0.9% agarose) and a following gel extraction (QIAEX II Gel Extraction Kit, Qiagen, Hilden, Germany). The purified DNA fragments were used for the PCR reaction *c*.
PCR reaction *c*: 6 µl PCR product of reaction a (≈ 50 ng)
6 µl PCR product of reaction b (≈ 50 ng)
2 µl primer CP-a (10 pmol/µl)
2 µl primer CP-e (10 pmol/µl)
10,0 µl PCR buffer
0.75 µl polymerase mixture (2.6 U)
73.25 µl H₂O

Reaction conditions for PCR reaction *c*:

| | |
|---|---|
| step 1 | 2 mm - 94°C |
| step 2 | 30 sec - 94°C |
| step 3 | 30 sec - 55°C |
| step 4 | 1 mm - 72°C |

Step 2 to 4 were repeated 31-times.

The resulting PCR product (1.4 kb) was purified as mentioned above, digested with *Eco* RI, and ligated in an *Eco* RI-digested and dephosphorylated pBsk(-)-vector (Stratagene, La Jolla, CA, USA). 1 µl of the ligation mixture was used to transform *E. coli* XL-1 competent cells (Stratagene, La Jolla, CA, USA). All standard procedures were carried out as described by Sambrook *et al*. (1987). The DNA sequence of the constructed consensus phytase gene (*fcp*, Figure 14) was controlled by sequencing as known in the art.

### Example 4

### Design of an improved consensus phytase (consensus phytase-10) amino acid sequence

The alignments used for the design of consensus phytase-10 were calculated using the program PILEUP from the Sequence Analysis Package Release 9.0 (Devereux *et al.*, 1984) with the standard parameter (gap creation penalty 12, gap extension penalty 4). The location of the gaps was refined using a text editor.

The following sequences were used for the alignment of the *Basiodiomycetes* phytases starting with the amino acid (aa) mentioned in Table 2:

**Table 2**

| Origin and vote weight of five *Basidiomycetes* phytases used for the calculation of the corresponding amino acid consensus sequence (basidio) |
|---|
| - *phyA1* from *Paxillus involutus* NN005693, aa 21, vote weight 0.5 (WO 98/28409) |
| - *phyA2* from *Paxillus involutus* NN005693, aa 21, vote weight 0.5 (WO 98/28409) |
| - *phyA* from *Trametes pubescens* NN9343, aa 24, vote weight 1.0 (WO 98/28409) |
| - *phyA* from *Agrocybe pediades* NN009289, aa 19, vote weight 1.0 (WO 98/28409) |
| - *phyA* from *Peniophora lycii* NN006113, aa 21, vote weight 1.0 (WO 98/28409) |

The alignment is shown in Figure 3.

In Table 3 the genes, which were used for the performance of the final alignment, are arranged. The first amino acid (aa) of the sequence which is used in the alignment is mentioned behind the organism designation.

**Table 3**

| Origin and vote weight of the phytase sequences used for the design of consensus phytase 10 |
|---|
| - *phyA* from *Aspergillus terreus* 9A-1, aa 27, vote weight 0.5 (Mitchell *et al*., 1997) |
| - *phyA* from *Aspergillus terreus* cbs116.46, aa 27, vote weight 0.5 (van Loon *et al*., 1998) |
| - *phyA* from *Aspergillus niger* var. *awamori*, aa 27, vote weight 0.5 (Piddington *et al.,* 1993) |
| - *phyA* from *Aspergillus niger* strain NRRL3135, aa 27, vote weight 0.5 (van Hartingsveldt *et al*., 1993) |
| - *phyA* from *Aspergillus fumigatus* ATCC 13073, aa 26, vote weight 0.2 (Pasamontes *et al*., 1997) |
| - *phyA* from *Aspergillus fumigatus* ATCC 32722, aa 26, vote weight 0.2 (van Loon *et al*., 1998) |
| - *phyA* from *Aspergillus fumigatus* ATCC 58128, aa 26, vote weight 0.2 (van Loon *et al*., 1998) |
| - *phyA* from *Aspergillus fumigatus* ATCC 26906, aa 26, vote weight 0.2 (van Loon *et al*., 1998) |
| - *phyA* from *Aspergillus fumigatus* ATCC 32239, aa 30, vote weight 0.2 (van Loon *et al*., 1998) |
| - *phyA* from *Emericella nidulans* , aa 25, vote weight 1.0, Pasamontes *et al.*, 1997a) |
| - *phyA* from *Talaromyces thermophilus* ATCC 20186, aa 24, vote weight 1.0 (Pasamontes *et al.*, 1997a) |
| - *phyA* from *Myceliophthora thermophila*, aa 19, vote weight 1.0 (Mitchell *et al*., 1997) |
| - *phyA* from *Thermomyces lanuginosa*, aa 36, vote weight 1.0 (Berka *et al.*, 1998) |
| - Consensus sequence of five *Basidiomycetes* phytases, vote weight 1.0 (Basidio, Figure 15) |

The corresponding alignment is shown in Figure 16.

### Calculation of the amino acid sequence of consensus-10

To improve the alignment, we added the original consensus sequence of five phytases from four different *Basidiomycetes*, called Basidio, still containing the undefined sequence positions (see Figure 15), nearly all phytase sequences used for calculation of the original consensus phytase and one new phytase sequence from the *Ascomycete Thermomyces lanuginosa* to a larger alignment. Using the consensus sequence of the basidiomycetal phytase sequences, does not pay regard to the diversity among the five amino acid sequences, but pays regard to the common and different amino acid residues between the phytases from the *Ascomycetes* and the *Basidiomycetes*.

We set plurality on 2.0 and threshold on 3. The used vote weight are listed in Table 3. The alignment and the corresponding consensus sequence is presented in Figure 16. The new consensus phytase sequence has 32 different amino acids in comparison to the original consensus phytase. Positions for which the program PRETTY was not able to calculate a consensus amino acid residue were filled according to rules mentioned in Example 3. None of the residues suggested by the program was replaced.

Furthermore, we included all *Basidiomycetes* phytases as single amino acid sequences but assigning a vote weight of 0.2 in the alignment. The corresponding alignment is shown in Figure 18. The calculated consensus amino acid sequence (consensus phytase-11) has the following differences to the sequence of consensus phytase-10. Letter X means that the program was not able to calculate a consensus amino acid; the amino acid in parenthesis corresponds to the amino acid finally included into the consensus phytase-10.
D35X, X(K)69K, X(E)100E, A101R, Q134N, X(K)153N, X(H)190H, X(A)204S, X(E)220D, E222T, V227A, X(R)271R, H287A, X(D)288D, X(K)379K, X(I)389I, E390X, X(E)415E, X(A)416A, X(R)446L, E463A, whereas the numbering is as in Fig. 17.

We also checked single amino acid replacements suggested by the improved consensus sequences 10 and 11 on their influence on the stability of the original consensus phytase. The approach is described in example 5.

### Conversion of consensus phytase-10 amino acid sequence to a DNA sequence

The first 26 amino acid residues of *A. terreus* cbs116.46 phytase were used as signal peptide and, therefore, fused to the *N*-terminus of consensus phytase-10. The used procedure is further described in Example 3.

The resulting sequence of the *fcp*10 gene is shown in Figure 17.

### Construction and cloning of the consensus phytase-10 gene (fcp10)

The calculated DNA sequence of *fcp*10 was divided into oligonucleotides of 85 bp, alternately using the sequence of the sense and the anti-sense strand. Every oligonucleotide overlaps 20 bp with its previous and its following oligonucleotide of the opposite strand. The location of all primers, purchased by Microsynth, Balgach (Switzerland) and obtained in a PAGE-purified form, is indicated in Figure 17.

### PCR-Reactions

In three PCR reactions, the synthesized oligonucleotides were composed to the entire gene. For the PCR, the High Fidelity Kit from Boehringer Mannheim (Boehringer Mannheim, Mannheim, Germany) and the thermo cycler The Protokol™ from AMS Biotechnology (Europe) Ltd. (Lugano, Switzerland) was used. The following oligonucleotides were used in a concentration of 0.2 pMol/µl.
Mix 1.10: CP-1, CP-2, CP-3.10, CP-4.10, CP-5.10, CP-6, CP-7.10, CP-8.10, CP-9.10, CP-10.10
Mix 2.10: CP-9.10, CP-11.10, CP-12.10, CP-13.10, CP-14.10, CP-15.10, CP-16.10, CP-17.10, CP18.10, CP-19.10, CP-20.10, CP-21.10, CP-22.10

The newly synthesized oligonucleotides are marked by number 10. The phytase contains the following 32 exchanges, which are underlined in Figure 17, in comparison to the original consensus phytase: Y54F, E58A, D69K, D70G, A94K, N134Q, I158V, S187A, Q188N, D197N, S204A, T214L, D220E, L234V, A238P, D246H, T251N, Y259N, E267D, E277Q, A283D, R291I, A320V, R329H, S364T, I366V, A379K, S396A, G404A, Q415E, A437G, A463E.

Four short PCR primer were used for the assembling of the oligonucleotides: 
CP-a:                    Eco RI
5'-TATATGAATTCATGGGCGTGTTCGTC-3'

CP-b:
5'-TGAAAAGTTCATTGAAGGTTTC-3'

CP-c.10:
5'-TCTTCGAAAGCAGTACACAAAC-3'

CP-e:          Eco RI
5'-TATATGAATTCTTAAGCGAAAC-3'
PCR reaction *a*: 10 µl Mix 1.10 (2.0 pmol of each oligonucleotide)
2 µl nucleotides (10 mM each nucleotide)
2 µl primer CP-a (10 pmol/ml)
2 µl primer CP-c.10 (10 pmol/ml)
10,0 µl PCR buffer
0.75 µl polymerase mixture
73.25 µl H₂O

PCR reaction *b*: 10 µl Mix 2.10 (2.0 pmol of each oligonucleotide)
2 µl nucleotides (10 mM each nucleotide)
2 µl primer CP-b (10 pmol/ml)
2 µl primer CP-e (10 pmol/ml)
10,0 µl PCR buffer
0.75 µl polymerase mixture (2.6 U)
73.25 µl H₂O

Reaction conditions for PCR reaction *a* and *b*:

| | |
|---|---|
| step 1 | 2 min - 45°C |
| step 2 | 30 sec - 72 °C |
| step 3 | 30 sec - 94 °C |
| step 4 | 30 sec - 52 °C |
| step 5 | 1 min - 72°C |

Step 3 to 5 were repeated 40-times.

The PCR products (670 and 905 bp) were purified by an agarose gel electrophoresis (0.9% agarose) and a following gel extraction (QIAEX II Gel Extraction Kit, Qiagen, Hilden, Germany). The purified DNA fragments were used for the PCR reaction *c*.
PCR reaction *c*: 6 µl PCR product of reaction a ≈50 ng)
6 µl PCR product of reaction b ≈50 ng)
2 µl primer CP-a (10 pmol/ml)
2 µl primer CP-e (10 pmol/ml)
10,0 µl PCR buffer
0.75 µl polymerase mixture (2.6 U) 73.25 µl H₂O

Reaction conditions for PCR reaction *c*:

| | |
|---|---|
| step 1 | 2 min - 94°C |
| step 2 | 30 sec - 94 °C |
| step 3 | 30 sec - 55 °C |
| step 4 | 1 min - 72 °C |

Step 2 to 4 were repeated 31-times.

The resulting PCR product (1.4 kb) was purified as mentioned above, digested with *Eco* RI, and ligated in an *Eco* RI-digested and dephosphorylated pBsk(-)-vector (Stratagene, La Jolla, CA, USA). 1 µl of the ligation mixture was used to transform *E. coli* XL-1 competent cells (Stratagene, La Jolla, CA, USA). All standard procedures were carried out as described by Sambrook *et al*. (1987). The DNA sequence of the constructed gene (*fcp10*) was checked by sequencing as known in the art.

### Example 5

### Increasing the thermostability of consensus phytase-1 by introduction of single mutations suggested by the amino acid sequence of consensus phytase-10 and consensus phytase-11

In order to increase the thermostability of homologous genes, it is also possible to test the stability effect of each differing amino acid residue between the protein of interest and the calculated consensus sequence and to combine all stabilizing mutations into the protein of interest. We used the consensus phytase as protein of interest and tested the effect on the protein stability of 34 amino acid residues, differing to consensus phytase 10 and/or 11 as single mutations.

To construct muteins for expression in *A. niger, S. cerevisiae*, or *H. polymorpha*, the corresponding expression plasmid containing the consensus phytase gene was used as template for site-directed mutagenesis (see Example 8 - 10). Mutations were introduced using the "quick exchange™ site-directed mutagenesis kit" from Stratagene (La Jolla, CA, USA) following the manufacturer's protocol and using the corresponding primers. All mutations made and their corresponding primers are summarized in Table 4. Plasmids harboring the desired mutation were identified by DNA sequence analysis as known in the art.

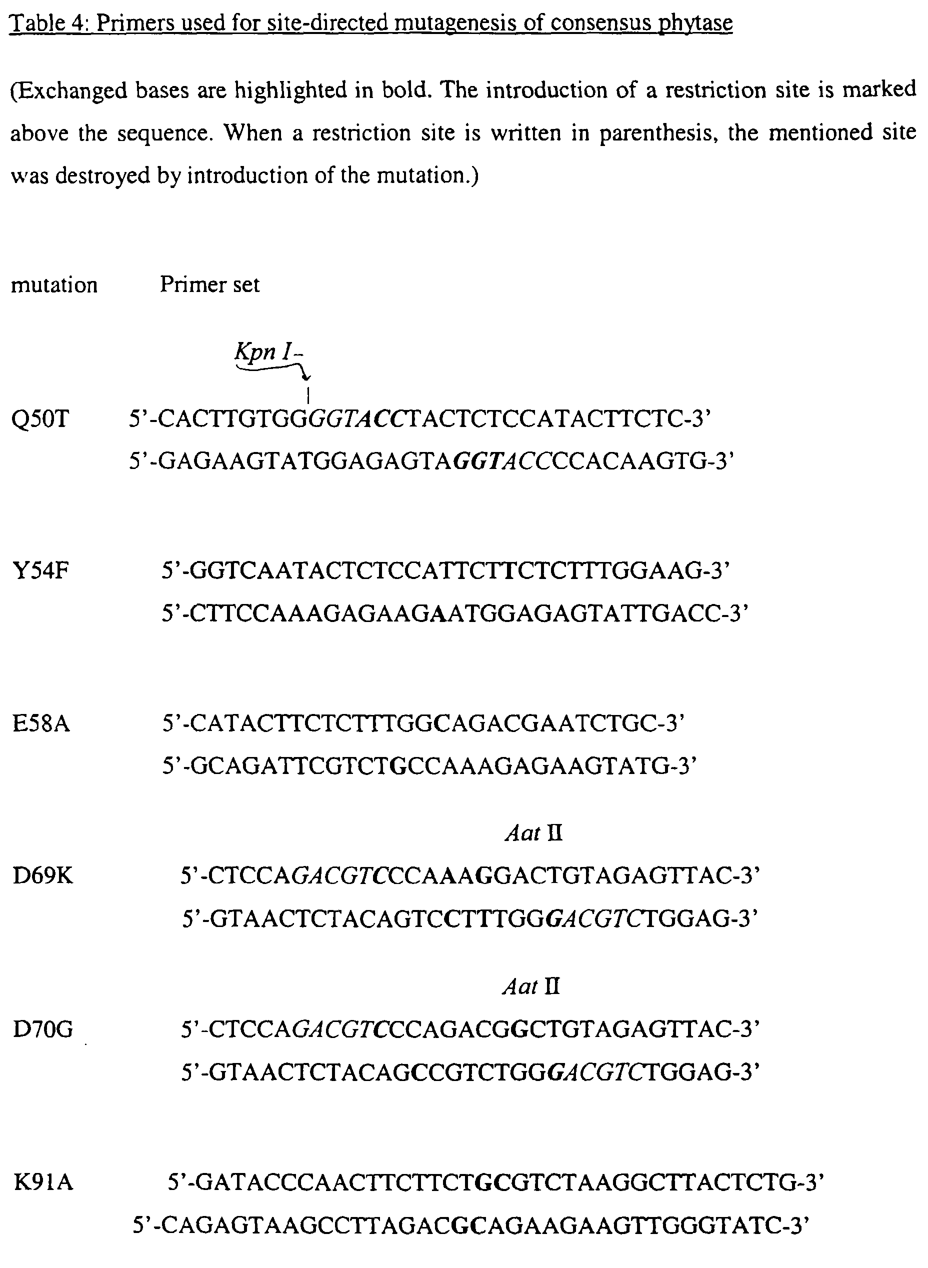

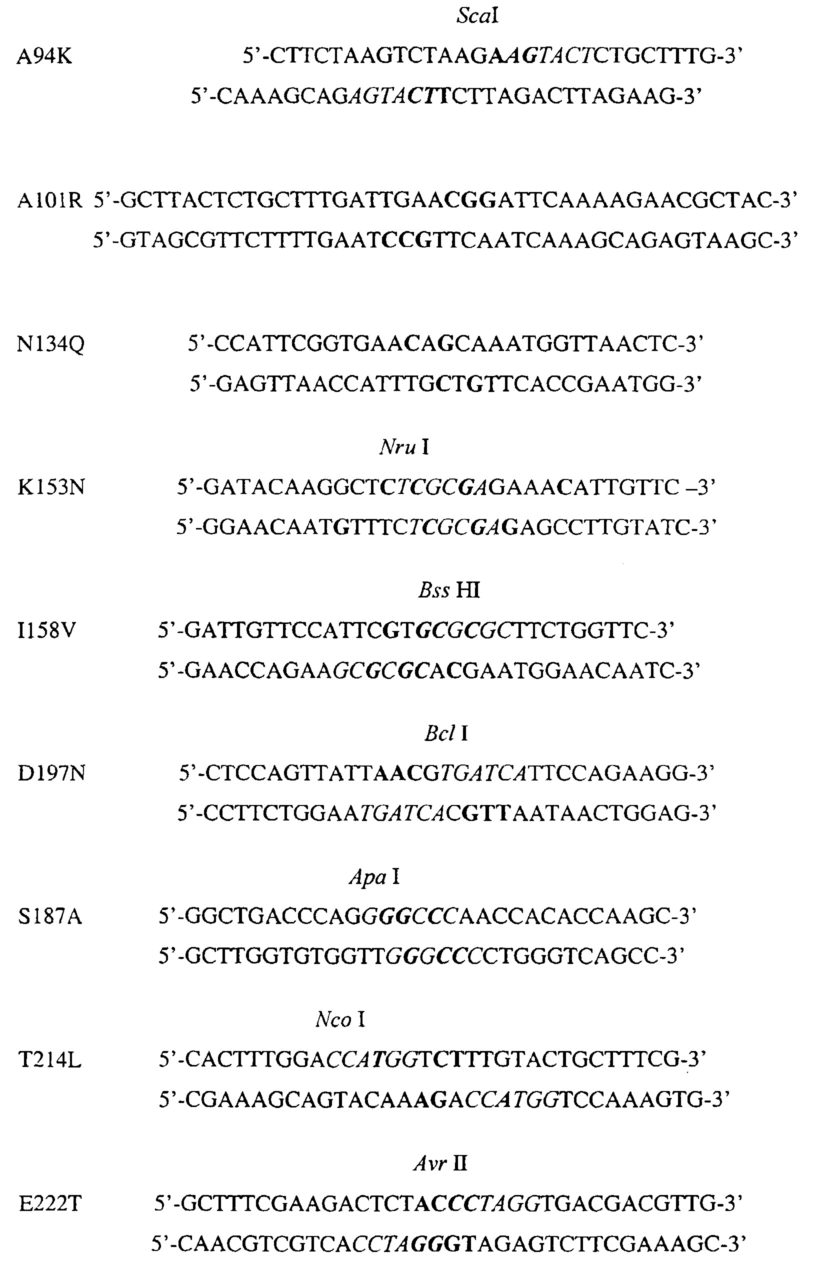

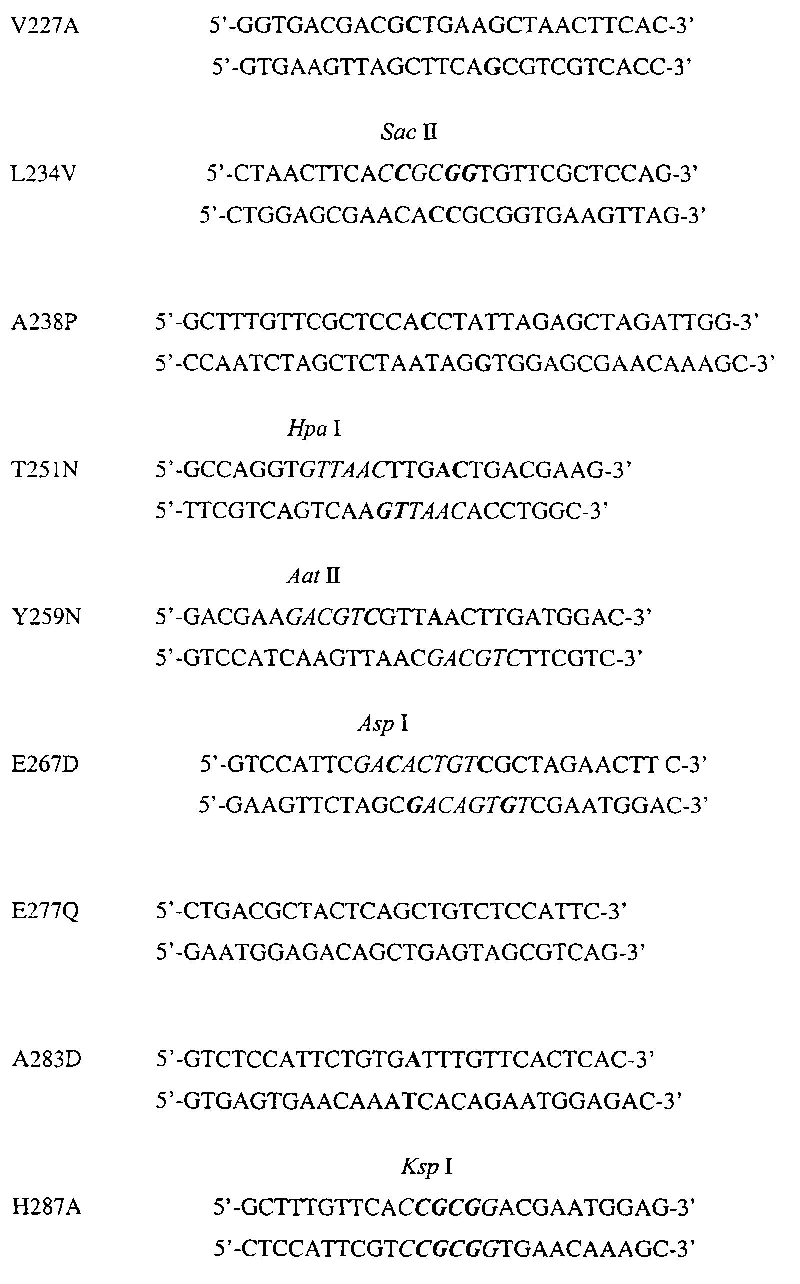

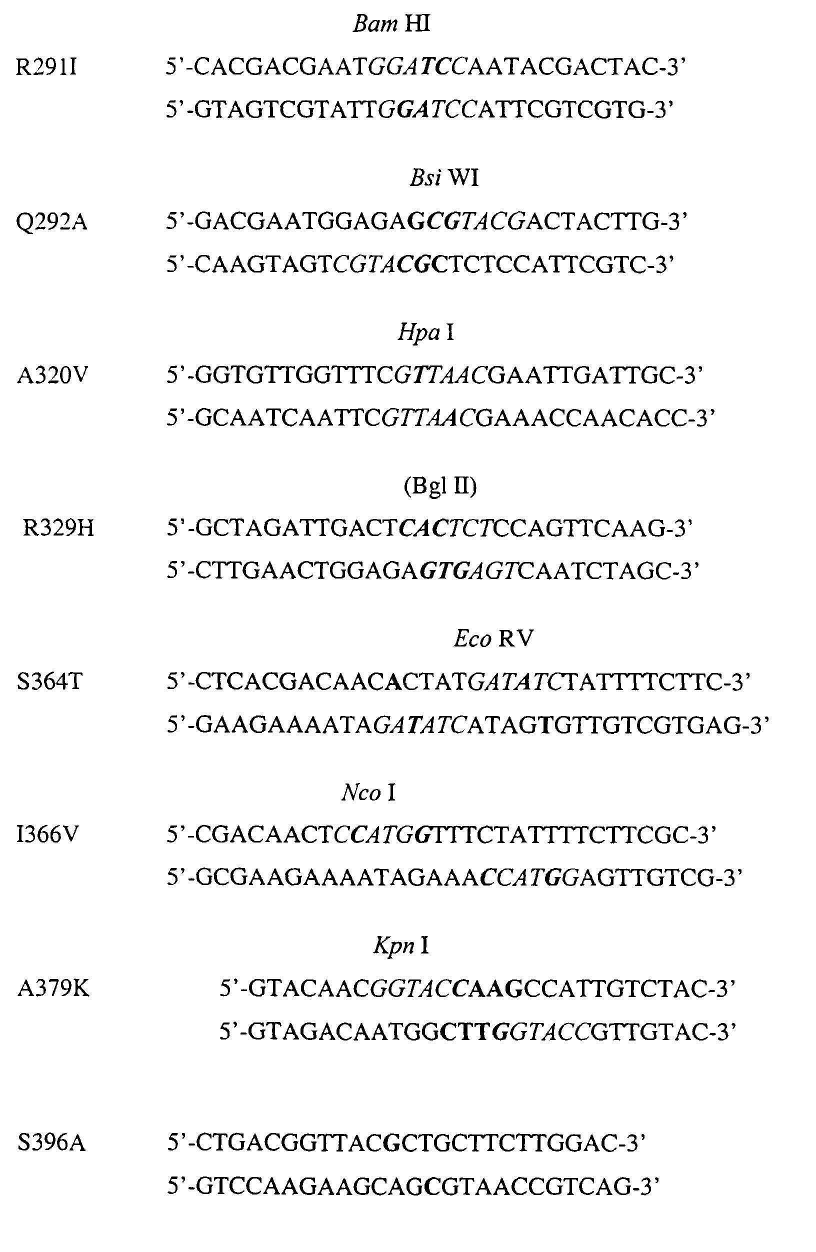

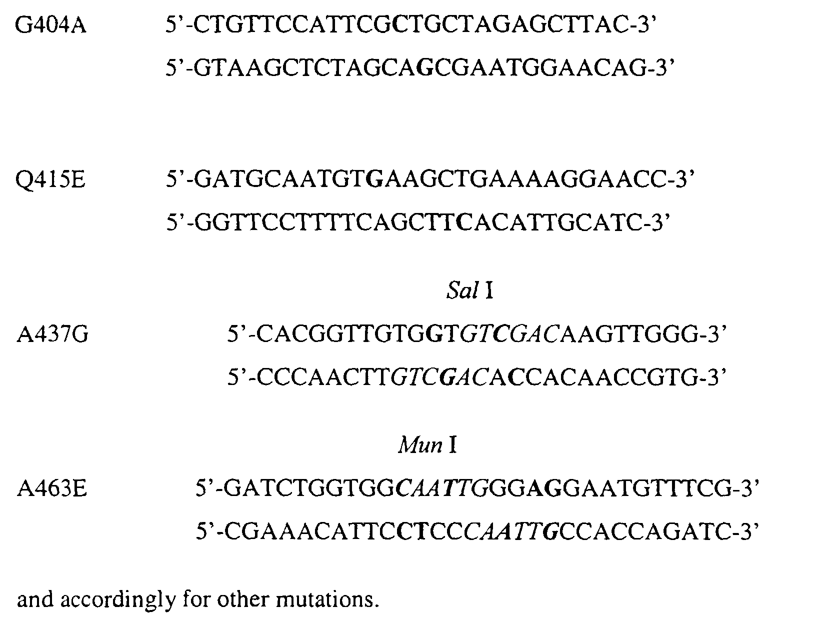

The temperature optimum of the purified phytases, expressed in *Saccharomyces cerevisiae* (Example 9), was determined as outlined in Example 11. Table 5 shows the effect on the stability of consensus phytase for each mutation introduced.

**Table 5**

| Stability effect of the individual amino acid replacements in concensus phytase-1 | | | | | |
|---|---|---|---|---|---|
| (+ or - means a positive, respectively, negative effect on the protein stability up to 1 °C, ++ and -- means a positive, respectively, negative effect on the protein stability between 1 and 3 °C; the number 10 or 11 corresponds to the consensus phytase sequence that suggests the amino acid replacement.) | | | | | |

| stabilizing | | neutral | | destabilizing | |
|---|---|---|---|---|---|
| mutation | effect | mutation | effect | mutation | effect |
| E58A (10) | + | D69A | ± | Y54F (10) | - |
| D69K (11) | + | D70G (10) | ± | V73I | - |
| D197N (10) | + | N134Q (10) | ± | A94K (10) | - |
| T214L (10) | ++ | G186H | + | A101R (11) | - |
| E222T (11) | ++ | S187A (10) | ± | K153N (11) | - |
| E267D (10) | + | T214V | ± | I158V (10) | -- |
| R291I* | + | T251N (10) | ± | G203A | -- |
| R329H (10) | + | Y259N (10) | ± | G205S | - |
| S364T (10) | ++ | A283D (10) | ± | A217V | - |
| A379K (11) | + | A320V (10) | ± | V227A (11) | -- |
| G404A (10) | ++ | K445T | ± | L234V (10) | - |
| | | A463E (10) | ± | A238P (10) | -- |
| | | | | E277Q (10) | - |
| | | | | H287A (11) | - |
| | | | | Q292A (10) | - |
| | | | | I366V (10) | - |
| | | | | S396A (10) | -- |
| | | | | Q415E (11) | - |
| | | | | A437G (10) | -- |
| | | | | E451R | -- |

| | | | | | |
|---|---|---|---|---|---|
| *: This amino acid replacement was found in another round of mutations. | | | | | |

We combined eight positive mutations (E58A, D197N, E267D, R291I, R329H, S364T, A379K, G404A) in the consensus phytase using the primers and the technique mentioned above in this example. Furthermore, the mutations Q50T and K91A were introduced which mainly influence the catalytical characteristics of the phytase (see European Patent Application Publication No. 897 985 as well as Example 11). The DNA and amino acid sequence of the resulting phytase gene (consensus phytase-thermo[8]-Q50T-K91A) is shown in Figure 19. In this way, the temperature optimum and the melting point of the consensus phytase was increased by 7 °C (Figure 27, 28, 29).

Using the results of Table 5, we further improved the thermostability of consensus phytase 10 by the following back mutations K94A, V158I, and A396S that revealed a strong negative influence on the stability of consensus phytase. The resulting protein is phytase-10-thermo [3]. Furthermore, we introduced the mutations Q50T and K91A which mainly influence the catalytical characteristics of consensus phytase (see patent application EP Publication No. 897 985 as well as Example 11 and Figure 26 and 27). The resulting DNA and amino acid sequence is shown in Figure 20. The optimized phytase showed a 4 °C higher temperature optimum and melting point than consensus phytase 10 (Figure 24 and 25). Furthermore, the phytase has also a strongly increased specific activity with phytate as substrate of 250 U/mg at pH 5.5 (Figure 26).

### Example 6

### Stabilization of the phytase of A. fumigatus ATCC 13073 by replacement of amino acid residues with the corresponding consensus phytase-1 and consensus phytase-10 residues

At six typical positions where the *A. fumigatus* 13073 is the only or nearly the only phytase in the alignment of Figure 13 that does not contain the corresponding consensus phytase amino acid residue, the non-consensus amino acid residue was replaced by the consensus one. In a first round, the following amino acids were substituted in *A. fumigatu*s 13073 phytase, containing the Q27T substitution and the signal sequence of *A. terreus* cbs.116.46 phytase (see Figure 21):
F55(28)Y, V100(73)I, F114(87)Y, A243(220)L, S265(242)P, N294(282)D.

The numbers in parentheses confer to the numbering of Figure 13.

In a second round, four of the seven stabilizing amino acid exchanges (E59A, R329H, S364T, G404A) found in the consensus phytase-10 sequence and, tested as single mutation in consensus phytase-1 (Table 5), were additionally introduced into the *A. fumigatus* a-mutant. Furthermore, the amino acid replacement S126N, shown to reduce the protease susceptibility of the phytase, was introduced.

The mutations were introduced as described in example 5 (see Table 6) and expressed as described in example 8 to 10. The resulting *A. fumigatus* 13073 phytase variants were called a-mutant and α-mutant-E59A-S126N-R329H-S364T-G404A.

The temperature optimum (60 °C, Figure 32) and the melting point (67.0 °C, Figure 31) of the *A. fumigatus* 13073 phytase α-mutant was increased by 5 °C in comparison to the values of the wild-type (temperature optimum: 55 °C, *T*_{*m*}: 60 °C). The five additional amino acid replacements further increased the temperature optimum by 3 °C (Figure 32).

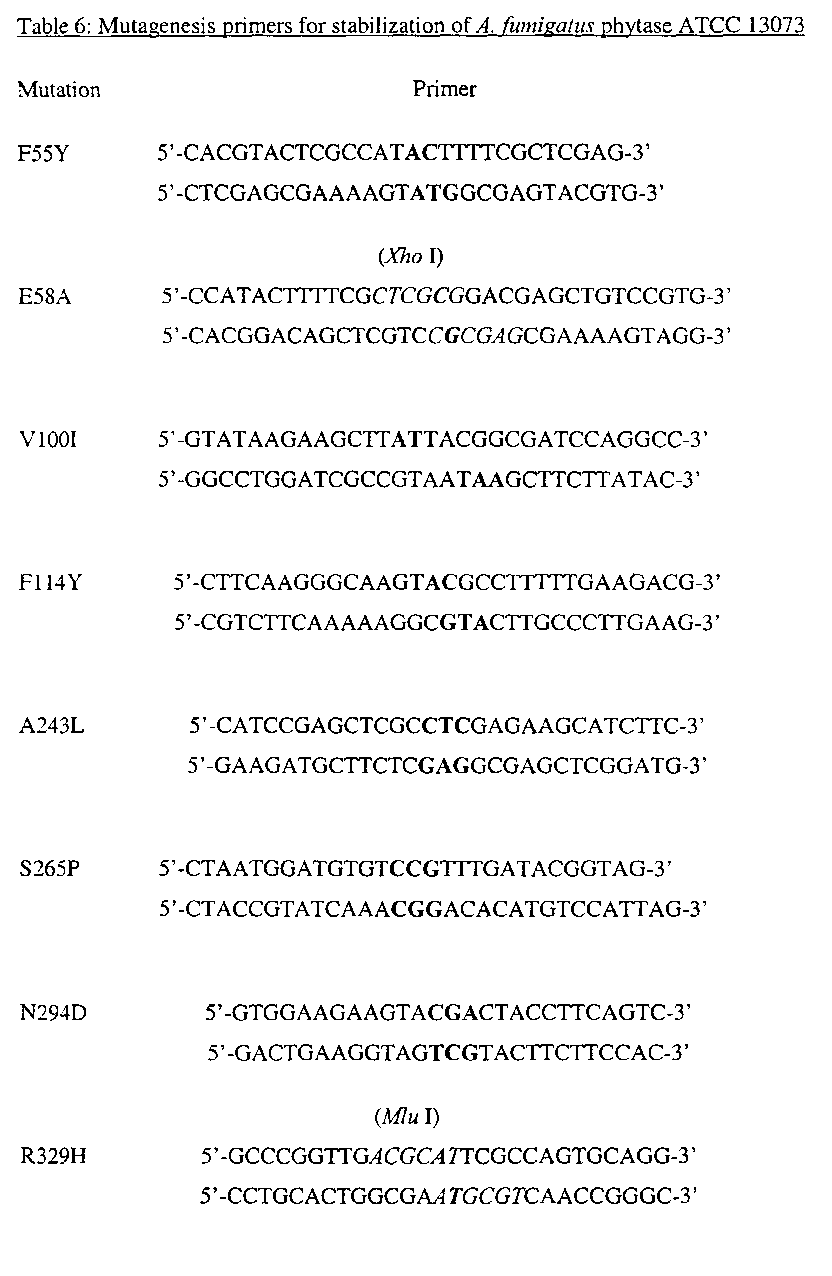

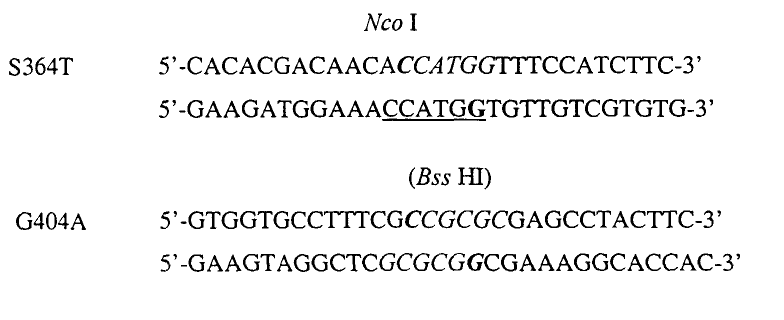

### Example 7

### Introduction of the active site amino acid residues of the A. niger NRRL 3135 phytase into the consensus phytase-1

We used the crystal structure of the *Aspergillus niger* NRRL 3135 phytase to define all active site amino acid residues (see Reference Example and EP 897 010). Using the alignment of Figure 13, we replaced the following active site residues and additionally the not identical adjacent ones of the consensus phytase by that of the *A. niger* phytase:
S89D, S92G, A94K, D164S, P201S, G203A, G205S, H212P, G224A, D226T, E255T, D256E, V258T, P265S, Q292H, G300K, Y305H, A314T, S364G, M365I, A397S, S398A, G404A, and A405S

The new protein sequence consensus phytase -7 was backtranslated into a DNA sequence (Figure 22) as described in Example 3. The corresponding gene (*fcp7*) was generated as described in Example 3 using the following oligonucleotide mixes:
Mix 1.7: CP-1, CP-2, CP-3, CP-4.7, CP-5.7, CP-6, CP-7, CP-8.7, CP-9, CP-10.7
Mix 2.7: CP-9, CP-10.7, CP-11.7, CP-12.7, CP-13.7, CP-14.7, CP-15.7, CP-16, CP-17.7, CP-18.7, CP-19.7, CP-20, CR-21, CP-22.

The DNA sequences of the oligonucleotides are indicated in Figure 15 The newly synthesized oligonucleotides are additionally marked by number 7. After assembling of the oligonucleotides using the same PCR primers as mentioned in Example 3, the gene was cloned into an expression vector as described in Examples 8 - 10.

The pH-profile determined after expression in *H. polymorpha* and purification was shifted into the acidic range of the pH-spectrum showing an optimum at pH 4.5-5.0 (see Figure 30). The enzyme had a broad pH-optimum reaching at least 60% of its maximum activity from pH 2.5 to pH 6.0. Up to pH 5.0, the profile resembled the profile of the *A. niger* NRRL 3135 phytase. However, below pH 5.0 it lacked the typical low at pH 4.0 of the profile of *A. niger* phytase.

### Example 8

### Expression of the consensus phytase genes in Hansenula polymorpha

The phytase expression vectors, used to transform *H. polymorpha* RB11 (Gellissen *et al*., 1994), was constructed by inserting the *Eco* RI fragment of pBsk^{*-*}*fcp* or variants thereof into the multiple cloning site of the *H. polymorpha* expression vector pFPMT121, which is based on an *ura3* selection marker from *S. cerevisiae*, a formate dehydrogenase (*FMD*) promoter element and a methanol oxidase (*MO*) termimator element from *H. polymorpha*. The 5' end of the *fcp* gene is fused to the *FMD* promoter, the 3' end to the *MOX* terminator (Gellissen *et al*., 1996; EP 0299 108 B). The resulting expression vector are designated pFPMT*fcp*, pFPMT*fcp*10, pFPMT*fcp*7.

The constructed plasmids were propagated in *E. coli*. Plasmid DNA was purified using standard state of the art procedures. The expression plasmids were transformed into the *H. polymorpha* strain RP11 deficient in orotidine-5'-phosphate decarboxylase (*ura3*) using the procedure for preparation of competent cells and for transformation of yeast as described in Gelissen *et al.* (1996). Each transformation mixture was plated on YNB (0.14% w/v Difco YNB and 0.5% ammonium sulfate) containing 2% glucose and 1.8% agar and incubated at 37 °C. After 4 to 5 days individual transformant colonies were picked and grown in the liquid medium described above for 2 days at 37 °C. Subsequently, an aliquot of this culture was used to inoculate fresh vials with YNB-medium containing 2% glucose. After seven further passages in selective medium, the expression vector integrates into the yeast genome in multimeric form. Subsequently, mitotically stable transformants were obtained by two additional cultivation steps in 3 ml non-selective liquid medium (YPD, 2% glucose, 10 g yeast extract, and 20 g peptone). In order to obtain genetically homogeneous recombinant strains an aliquot from the last stabilization culture was plated on a selective plate. Single colonies were isolated for analysis of phytase expression in YNB containing 2% glycerol instead of glucose to derepress the *fmd* promoter. Purification of the consensus phytases was done as described in Example 9.

### Example 9

### Expression of the consensus phytase genes in Saccharomyces cerevisiae and purification of the phytases from culture supernatant

The consensus phytase genes were isolated from the corresponding Bluescript-plasmid (pBsk⁻*fcp*, pBSK⁻*fcp10*, pBsk⁻*fcp7*) and ligated into the *Eco* RI sites of the expression cassette of the *Saccharomyces cerevisiae* expression vector pYES2 (Invitrogen, San Diego, CA, USA) or subcloned between the shortened GAPFL (glyceraldhyde-3-phosphate dehydrogenase) promoter and the *pho5* terminator as described by Janes *et al*. (1990). The correct orientation of the gene was checked by PCR. Transformation of *S*. *cerevisiae* strains. e. g. INVSc1 (Invitrogen, San Diego, CA, USA) was done according to Hinnen *et al.* (1978). Single colonies harboring the phytase gene under the control of the GAPFL promoter were picked and cultivated in 5 ml selection medium (SD-uracil, Sherman *et al*., 1986) at 30°C under vigorous shaking (250 rpm) for one day. The preculture was then added to 500 ml YPD medium (Sherman *et al.,* 1986) and grown under the same conditions. Induction of the *gal1* promoter was done according to manufacturer's instruction. After four days of incubation cell broth was centrifuged (7000 rpm, GS3 rotor, 15 mm, 5°C) to remove the cells and the supernatant was concentrated by way of ultrafiltration in Amicon 8400 cells (PM30 membranes) and ultrafree-15 centrifugal filter devices (Biomax-30K, Millipore, Bedford, MA, USA). The concentrate (10 ml) was desalted on a 40 ml Sephadex G25 Superfine column (Pharmacia Biotech, Freiburg, Germany), with 10 mM sodium acetate, pH 5.0, serving as elution buffer. The desalted sample was brought to 2 M (NH₄)₂SO₄ and directly loaded onto a 1 ml Butyl Sepharose 4 Fast Flow hydrophobic interaction chromatography column (Pharmacia Biotech, Feiburg, Germany) which was eluted with a linear gradient from 2 M to 0 M (NH4)₂SO₄ in 10 mM sodium acetate, pH 5.0. Phytase was eluted in the break-through, concentrated and loaded on a 120 ml Sephacryl S-300 gel permeation chromatography column (Pharmacia Biotech, Freiburg, Germany). Consensus phytase and consensus phytase -7 eluted as a homogeneous symmetrical peak and was shown by SDS-PAGE to be approx. 95% pure.

### Example 10

### Expression of the consensus phytase genes in Aspergillus niger

The Bluescript-plasmids pBsk⁻*fcp*, pBSK⁻*fcp10*, and pBsk⁻*fcp7* were used as template for the introduction of a *Bsp* HI-site upstream of the start codon of the genes and an *Eco* RV-site downstream of the stop codon. The Expand™ High Fidelity PCR Kit (Boehringer Mannheim, Mannheim, Germany) was used with the following primers:

Primer Asp-1:

Bsp HI

5'-TATATCATGAGCGTGTTCGTCGTGCTACTGTTC-3'

Primer Asp-2 used for cloning of fcp and fcp7:

Eco RV

3'-ACCCGACTTACAAAGCGAATTCTATAGATATAT-5'

Primer Asp-3 used for cloning of fcp10:

Eco RV

3'-ACCCTTCTTACAAAGCGAATTCTATAGATATAT-5'

The reaction was performed as described by the supplier. The PCR-amplified *fcp*-genes had a new *Bsp* HI site at the start codon, introduced by primer Asp-1, which resulted in a replacement of the second amino acid residue glycine by seine. Subsequently, the DNA-fragment was digested with *Bsp* HI and *Eco* RV and ligated into the *Nco* I site downstream of the glucoamylase promoter of *Aspergillus niger* (*glaA*) and the *Eco* RV site upstream of the *Aspergillus nidulans* tryptophan C terminator (*trpC*) (Mullaney *et al*., 1985). After this cloning step, the genes were sequenced to detect possible failures introduced by PCR. The resulting expression plasmids which basically corresponds to the pGLAC vector as described in Example 9 of EP 684 313, contained the orotidine-5'-phosphate decarboxylase gene (*pyr4*) of *Neurospora crassa* as a selection marker. Transformation of *Aspergillus niger* and expression of the consensus phytase genes was done as described in EP 684 313. The consensus phytases were purified as described in Example 9.

### Example 11

### Determination of phytase activity and of temperature optimum

Phytase activity was determined basically as described by Mitchell et al (1997). The activity was measured in an assay mixture containing 0.5% phytic acid (≈5 mM) in 200 mM sodium acetate, pH 5.0. After 15 mm of incubation at 37 °C, the reaction was stopped by addition of an equal volume of 15% trichloroacetic acid. The liberated phosphate was quantified by mixing 100 µl of the assay mixture with 900 µl H₂O and 1 ml of 0.6 M H₂SO₄, 2% ascorbic acid and 0.5% ammonium molybdate. Standard solutions of potassium phosphate were used as reference. One unit of enzyme activity was defined as the amount of enzyme that releases 1 µmol phosphate per minute at 37 °C. The protein concentration was determined using the enzyme extinction coefficient at 280 nm calculated according to Pace et al (1995): consensus phytase, 1.101; consensus phytase 7, 1.068; consensus phytase 10, 1.039.

In case of pH-optimum curves, purified enzymes were diluted in 10 mM sodium acetate, pH 5.0. Incubations were started by mixing aliquots of the diluted protein with an equal volume of 1% phytic acid (≈10 mM) in a series of different buffers: 0.4 M glycine/HCl, pH 2.5; 0.4 M acetate/NaOH, pH 3.0, 3.5, 4.0, 4.5, 5.0, 5.5; 0.4 M imidazole/HCl, pH 6.0, 6.5; 0.4 M Tris/HCl pH 7.0, 7.5, 8.0, 8.5, 9.0. Control experiments showed that pH was only slightly affected by the mixing step. Incubations were performed for 15 min at 37 °C as described above.

For determinations of the substrate specificities of the phytases, phytic acid in the assay mixture was replaced by 5 mM concentrations of the respective phosphate compounds. The activity tests were performed as described above.

For determination of the temperature optimum, enzyme (100 µl) and substrate solution (100 µl) were pre-incubated for 5 mm at the given temperature. The reaction was started by addition of the substrate solution to the enzyme. After 15 min incubation, the reaction was stopped with trichloroacetic acid and the amount of phosphate released was determined.

The pH-optimum of the original consensus phytase was around pH 6.0-6.5 (70 U/mg). By introduction of the Q50T mutation, the pH-optimum shifted to pH 6.0 (130 U/mg). After introduction of K91A, the pH optimum shifted one pH-unit into the acidic pH-range showing a higher specific activity between pH 2.5 and pH 6.0. That was shown for the stabilized mutants and for consensus phytase-10, too (Figure 26 and 27).

Consensus phytase-7, which was constructed to transfer the catalytic characteristics of the *A. niger* phytase NRRL 3135 into the consensus phytase, had a pH-profile which is shifted into the acidic range of the pH-spectrum showing an optimum between pH 4.5 and 5.0 (see Figure 31). The enzyme had a broad pH-optimum reaching at least 60% of its increased maximum activity from pH 2.5 to pH 6.0. The substrate spectrum, too, resemble more to that of the A. niger NRRL 3135 phytase than to the consensus phytase-1.

The temperature optimum of consensus phytase-1 (71 °C) was 16-26 °C higher than the temperature optimum of the wild-type phytases (45-55 °C, Table 7) which were used to calculate the consensus sequence. The improved consensus phytase-10 showed a further increase of its temperature optimum to 80 °C (Figure 33). The temperature optimum of the consensus phytase-1-thermo[8] was found in the same range (78 °C) using the supernatant of an overproducing *S. cerevisiae* strain. The highest temperature optimum reached of 82 °C was determined for consensus phytase-10-thermo-Q50T-K91A.

**Table 7**

| | | |
|---|---|---|
| Temperature optimum and *T*ₘ-value of consensus phytase and of the phytases from *A. fumigatus*, *A. niger*, *E. nidulans*, and *M. thermophila*. The determination of the temperature optimum was performed as described in Example 11 The *T*ₘ-values were determined by differential scanning calorimetry as described in Example 12. | | |

| phytase | temperature optimum [°C] | *T*m [°C] |
|---|---|---|
| Consensus phytase-10-thermo-Q50T-K91A | 82 | 89.3 |
| Consensus phytase-10-thermo-Q50T | 82 | 88.6 |
| Consensus phytase-10 | 80 | 85.4 |
| Consensus phytase-1-thermo[8]-Q50T | 78 | 84.7 |
| Consensus phytase-1-thermo[8]-Q50T-K91A | 78 | 85.7 |
| Consensus phytase-1 | 71 | 78.1 |
| *A. niger* NRRL3135 | 55 | 63.3 |
| *A. fumigatus* 13073 | 55 | 62.5 |
| *A. fumigatus* 13073 α-mutant | 60 | 67.0 |
| *A. fumigatus* 13073 α-mutant (optimized) | 63 | - |
| *A. terreus* 9A-1 | 49 | 57.5 |
| *A. terreus* cbs.116.46 | 45 | 58.5 |
| *E. nidulans* | 45 | 55.7 |
| *M. thermophila* | 55 | n. d. |
| *T. thermophilus* | 45 | n. d. |

### Example 12

### Determination of the melting point by differential scanning calorimetry (DSC)

In order to determine the unfolding temperature of the phytases, differential scanning calorimetry was applied as previously published by Brugger et al (1997). Solutions of 50-60 mg/ml homogeneous phytase were used for the tests. A constant heating rate of 10 °C/min was applied up to 90-95 °C.

The determined melting points reflect the results obtained for the temperature optimums (Table 7). The most stable consensus phytase designed is consensus phytase-10-thermo-Q50T-K91A showing a melting temperature under the choosen condition of 89.3 C. This is 26 to 33.6 °C higher than the melting point of the wild-type phytases used.

### Example 13

### Transfer of basidiomycete phytase active site into consensus phytase-10-thermo-Q50T-K91A

As described previously (Example 5), mutations derived from the basidiomycete phytase active site were introduced into the consensus phytase 10. The following five constructs a) to e) were prepared:

This construct is called consensus phytase 12, and it comprises a selected number of active site residues of the basidio consensus sequence, its amino acid sequence (consphy12) is shown in Fig. 33 (the first 26 amino acids forms the signal peptide, amended positions are underlined);
a cluster of mutations (Cluster II) was transferred to the consensus 10 sequence, viz.: S80Q, Y86F, S90G, K91A, S92A, K93T, A94R, Y95I;
analogously, another cluster of mutations (Cluster III) was transferred, viz.: T129V, E133A, Q143N, M136S, V137S, N138Q, S139A;
analogously, a further cluster of mutations (Cluster IV) was transferred, viz.: A168D, E171T, K172N, F173W;
and finally, a further cluster of mutations (Cluster V) was transferred, viz.: Q297G, S298D, G300D, Y305T.

These constructs were expressed as described in Examples 8 to 10.

### References:

Akanuma, S., Yamagishi, A., Tanaka, N. & Oshima, T. (1998). Serial increase in the thermal stability of 3-isopropylmalate dehydrogenase from *Bacillus subtilis* by experimental evolution. *Prot. Sci.* **7**, 698-705.
Arase, A., Yomo, T., Urabe, I., Hata, Y., Katsube, Y. & Okada, H. (1993). Stabilization of xylanase by random mutagenesis. *FEBS Lett*. **316**, 123-127.
Berka, R. M., Rey, M. W., Brown, K. M., Byun, T. & Klotz, A. V. (1998). Molecular characterization and expression of a phytase gene from the thermophilic fungus Thermomyces lanuginosus. *Appl. Environ. Microbiol*. **64**, 4423-4427.
Blaber, M., Lindstrom, J. D., Gassner, N., Xu, J., Heinz, D. W. & Matthews, B. W. (1993). Energetic cost and structural consequences of burying a hydroxyl group within the core of a protein determined from Ala'Ser and Val'Thr substitutions in T4 lysozyme. *Biochemistry* **32**, 11363-11373.
Brugger, R., Mascarello, F., Augem, S., van Loon, A. P. G. M. & Wyss, M. (1997). Thermal denaturation of phytases and pH 2.5 acid phosphatase studied by differential scanning calorimetry. In *The Biochemistry of phytate and phytase* (eds. *Rasmussen*, *S.K*; *Raboy*, *V*.; *Dalbøge*, *H.* and *Loewus, F*.; Kluwer Academic Publishers.
Cosgrove, D.J. (1980) Inositol phosphates - their chemistry, biochemistry and physiology: studies in organic chemistry, chapter 4. Elsevier Scientific Publishing Company, Amsterdam, Oxford, New York.
Devereux, J., Haeberli, P.& Smithies, O. (1984) A comprehensive set of sequence analysis programs for the VAX. *Nucleic Acids Res*. **12**, 387-395.
Gellissen, G., Hollenberg, C. P., Janowicz, Z. A. (1994) Gene expression in methylotrophic yeasts . In: Smith, A. (ed.) Gene expression in recombinant microorganisms. Dekker, New York, 395-439.
Gellissen, G., Piontek, M., Dahlems, U., Jenzelewski, V., Gavagan, J. E., DiCosimo, R., Anton, D. I. & Janowicz, Z. A. (1996) Recombinant *Hansenula polymorpha* as a biocatalyst: coexpression of the spinach glycolate oxidase (*GO*) and the *S. cerevisiae* catalase T (*CTT1*) gene. *Appl. Microbiol. Biotechnol*. **46**, 46-54.
Gerber, P. and Müller, K. (1995) Moloc molecular modeling software. *J. Comput. Aided Mol*. *Des.* **9**, 251-268
Hinnen, A., Hicks, J. B. & Fink, G, R. (1978) Transformation of yeast*. Proc. Natl. Acad. Sci.* USA **75**, 1929-1933.
Imanaka, T., Shibazaki, M. & Takagi, M. (1986). A new way of enhancing the thermostability of proteases. *Nature* **324**, 695-697.
Janes, M., Meyhack, B., Zimmermann, W. & Hinnen, A. (1990) The influence of GAP promoter variants on hirudine production, average plasmid copy number and cell growth in *Saccharomyces cerevisiae. Curr Genet*. **18**, 97-103.
Karpusas, M., Baase, W. A., Matsumura, M. & Matthews, B. W. (1989). Hydrophobic packing in T4 lysozyme probed by cavity-filling mutants. *Proc. Natl. Acad Sci*.*(USA)* **86**, 8237-8241.
Margarit, I., Campagnoli, S., Frigerio, F., Grandi, G., Fillipis, V. D. & Fontana, A. (1992). Cumulative stabilizing effects of glycine to alanine substitutions in *Bacillus subtilis* neutral protease. *Prot. Eng*. **5**, 543-550.
Matthews, B. W. (1987a). Genetic and structural analysis of the protein stability problem. *Biochemistry* **26**, 6885-6888.
Matthews, B. W. (1993). Structural and genetic analysis of protein stability. *Annu. Rev. Biochem.* **62**, 139-160.
Matthews, B. W., Nicholson, H. & Becktel, W. (1987). Enhanced protein thermostability from site-directed mutations that decrease the entropy of unfolding. *Proc. Natl. Acad. Sci*. *(USA)* **84**, 6663-6667.
Mitchell, D. B., Vogel, K., Weimann, B. I., Pasamontes, L. & van Loon, A. P. G. M. (1997) The phytase subfamily of histidine acid phosphatases: isolation of genes for two novel phytases from the fungi *Aspergillus terreus* and *Myceliophthora thermophila*, *Microbiology* **143**, 245-252.
Mullaney, E. J., Hamer, J. E., Roberti, K. A., Yelton, M. M. & Timberlake, W. E. (1985) Primary structure of the *trpC* gene from *Aspergillus nidulans*. *Mol. Gen*. *Genet.* **199,** 37-46.
Munoz, V. & Serrano, L. (1995). Helix design, prediction and stability. *Curr. Opin. Biotechnol.* **6**, 382-386.
Pace, N. C., Vajdos, F., Fee, L., Grimsley, C. & Gray, T. (1995). How to measure and predict the molar absorption coefficient of a protein. *Prot. Sci*. **4**, 2411-2423.
Pantoliano, M. W., Landner, R. C., Brian, P. N., Rollence, M. L., Wood, J. F. & Poulos, T. L. (1987). Protein engineering of subtilisin BPN': enhanced stabilization through the introduction of two cysteines to form a disulfide bond. *Biochemistry* **26**, 2077-2082.
Pasamontes, L., Haiker, M., Henriquez-Huecas, M., Mitchell, D. B. & van Loon, A. P. G. M. (1997a). Cloning of the phytases from *Emericella nidulans* and the thermophilic fungus *Talaromyces thermophilus*. *Biochim. Biophys. Acta* **1353**, 217-223.
Pasamontes, L., Haiker, M., Wyss, M., Tessier, M. & van Loon, A. P. G. M. (1997) Cloning, purification and characterization of a heat stable phytase from the fungus *Aspergillus fumigatus*, *Appl. Environ. Microbiol.* **63**, 1696-1700.
Piddington, C. S., Houston, C. S., Paloheimo, M., Cantrell, M., Miettinen-Oinonen, A. Nevalainen, H., & Rambosek, J. (1993) The cloning and sequencing of the genes encoding phytase (*phy*) and pH 2.5-optimum acid phosphatase (*aph*) from *Aspergillus niger* var. *awamori. Gene* **133**, 55-62.
Purvis, I. J., Bettany, A. J. E., Santiago, T. C., Coggins, J. R., Duncan, K., Eason, R. & Brown, A. J. P. (1987). The efficiency of folding of some proteins is increased by controlled rates of translation in vivo. *J. Mol. Biol*. **193**, 413-417.
Risse, B., Stempfer, G., Rudolph, R., Schumacher, G. & Jaenicke, R. (1992). Characterization of the stability effect of point mutations of pyruvate oxidase from *Lactobacillus plantarum*: protection of the native state by modulating coenzyme binding and subunit interaction. *Prot. Sci.* **1**, 1710-1718.
Sambrook, J., Fritsch, E. F. & Maniatis, T. (1989) *Molecular Cloning: A Laboratory Manual*, 2nd Ed., Cold Spring Harbor Laboratory, Cold Spring Harbor, NY.
Sauer, R., Hehir, K., Stearman, R., Weiss, M., Jeitler-Nilsson, A., Suchanek, E. & Pabo, C. (1986). An engineered intersubunit disulfide enhances the stability and DNA binding of the N-terminal domain of 1-repressor. *Biochemistry* **25**, 5992-5999.
Serrano, L., Day, A. G. & Fersht, A. R. (1993). Step-wise mutation of barnase to binase. A procedure for engineering increased stability of proteins and an experimental analysis of the evolution of protein stability. *J. Mol. Biol.* **233**, 305-312.
Sheman, J. P., Finck, G. R. & Hicks, J. B. (1986) Laboratory course manual for methods in yeast genetics. Cold Spring Harbor University.
Steipe, B., Schiller, B., Plueckthun, A. & Steinbach, S. (1994). Sequence statistics reliably predict stabilizing mutations in a protein domain. *J. Mol. Biol.* **240**, 188-192.
van den Burg, B., Vriend, G., Veltman, O. R., Venema & G., Eijsink, V. G. H. (1998). Engineering an enzyme to resist boiling. *Proc. Natl. Acad. Sci.* (USA) **95**, 2056-2060.
Van Etten, R.L. (1982) Human prostatic acid phosphatase: a histidine phosphatase. *Ann. NY Acad. Sci.* **390**,27-50
van Hartingsveldt, W., van Zeijl, C. M. F., Harteveld, G. M., Gouka, R. J., Suykerbuyk, M. E. G., Luiten, R. G. M., van Paridon, P. A., Selten, G. C. M., Veenstra, A. E., van Gorcom, R. F. M., & van den Hondel, C. A. M. J. J. (1993) Cloning, characterization and overexpression of the phytase-encoding gene (*phyA*) of *Aspergillus niger*. *Gene* **127**, 87-94.
van Loon, A. P. G. M., Simoes-Nunes, C., Wyss, M., Tomschy, A., Hug, D., Vogel, K. & Pasamontes, L. (1998). A heat resistant phytase of *Aspergillus fumigatus* with superior performance in animal experiments. Phytase optimization and natural variability. In *the Biochemistry of phytate and phytases*. Kluwer Academic Press, s.a.

## Claims

1. A stabilized dry or liquid enzyme formulation comprising phytase and one or more stabilizing agents selected from the group consisting of:
a) C₅ sugars, preferably xylitol or ribitol,
b) polyethylene glycols having a molecular weight of 600 to 4000 Da, preferably 1000 to 3350 Da.
c) the disodium salts of malonic, glutaric and succinic acid,
d) carboxymethylcellulose, and
e) alginate, preferably sodium alginate.

2. A stabilized dry or liquid enzyme formulation comprising phytase which has been crosslinked:
a) with glutaraldehyde, or by
b) oxidation with sodium periodate and reaction with adipic acid dihydrazide.

3. Enzyme formulation according to claims 1 or 2, characterized in that the phytase is a fungal or a consensus phytase.

4. Enzyme formulation according to claim 3, characterized in that the fungal phytase is selected from the group consisting of *Aspergillus fumigatus, Aspergillus nidulans*, *Aspergillus terreus* and *Aspergillus niger* phytase.

5. Enzyme formulation according to anyone of claims 1 to 4 characterized in that the formulation is liquid.

6. Enzyme formulation according to claim 5, characterized in that the stabilizing agent is polyethylene glycol whereby the polyethylene glycol is present in a concentration of 10-50% (w/w) in the final formulation.

7. Enzyme formulation according to claim 5 or 6, characterized in that the stabilizing agent is xylitol and/or ribitol which is present in the final formulation in a concentration of 20-60% (w/w).

8. Enzyme formulation according to any of claims 5 to 7, characterized in that the stabilizing agent is the disodium salt of glutaric, succinic or malonic acid whereby the concentration of the salt in the final formulation ranges between 10 and 30% (w/w).

9. Enzyme formulation according to any of claims 5 to 8, characterized in that the stabilizing agent is carboxymethylcellulose whereby the concentration of the polymer in the final formulation ranges between 1 and 10% (w/w).

10. Enzyme formulation according to any of claims 5 to 9, characterized in that the stabilizing agent is sodium alginate whereby the concentration of the polymer in the final formulation ranges between 1 and 10% (w/w).

11. Enzyme formulation according to any of claims 1-4, characterized in that the formulation is dry/solid.

12. Enzyme formulation according to claim 11, characterized in that the stabilizing agent is polyethylene glycol whereby the polyethylene glycol is present in a concentration of 1-20% (w/w) in the final formulation.

13. Enzyme formulation according to claim 11 or 12, characterized in that the stabilizing agent is xylitol and/or ribitol which is present in the final formulation in a concentration of 1-20% (w/w).

14. Enzyme formulation according to any of claims 11 to 13, characterized in that the stabilizing agent is the disodium salt of glutaric, succinic or malonic acid whereby the concentration of the salt in the final formulation ranges between 1 and 20% (w/w).

15. Enzyme formulation according to any of claims 11 to 14, characterized in that the stabilizing agent is carboxymethylcellulose whereby the concentration of the polymer in the final formulation ranges between 1 and 10% (w/w).

16. Enzyme formulation according to any of claims 11 to 15, characterized in that the stabilizing agent is sodium alginate whereby the concentration of the polymer in the final formulation ranges between 1 and 10% (w/w).

17. Enzyme formulation according to any of claims 2-5 or 11 characterized in that the phytase monomers are crosslinked by addition of glutaraldehyde.

18. Enzyme formulation according to any of claims 2-5 or 11 characterized in that the phytase monomers are crosslinked by oxidation of carbohydrate residues with sodium periodate and subsequent addition of adipic acid dihydrazide.

19. A method of preparing a feed composition for monogastric animals, characterized in that the feed is treated with a stabilized dry or liquid enzyme formulation according to any of claims 1-18.

20. A feed composition for monogastric animals, characterized in that the feed comprises a stabilized dry or liquid enzyme formulation according to any one of claims 1-18.

21. A method of providing a monogastric animal with its dietary requirement of phosphorous, characterized in that the animal is feeded with a feed according to claim 20 and that no additional phosphorous is added to the feed.

22. A method of preparing a dry or liquid phytase formulation, characterized in that a stabilized phytase according to claims 1-18 is used.
